# EUROPEAN PATENT SPECIFICATION

(11) **EP 3 027 738 B1**
(45) Date of publication and mention of the grant of the patent: **02.03.2022**
(21) Application number: 14781963.5
(22) Date of filing: 31.07.2014
(51) Int. Cl.: C12N 5/0775, A61K 35/28, A61K 38/17, A61K 35/16, A61P 11/10, A61P 11/00

(54) **MSCS IN THE TREATMENT OF INFLAMMATORY PULMONARY DISEASES**
MSCS BEI DER BEHANDLUNG ENTZÜNDLICHER LUNGENKRANKHEITEN
CELLULES SOUCHES MÉSENCHYMATEUSES DANS LE TRAITEMENT DE MALADIES INFLAMMATOIRES PULMONAIRES

(30) Priority: 01.08.2013 SE 1300521
(43) Date of publication of application: 08.06.2016
(73) Proprietor: Swedish StromaBio AB, 181 33 Lidingö (SE)
(72) Inventor: SIMONSON, Oscar, SE-11526 Stockholm (SE); LE BLANC, Katarina, SE-12862 Sköndal (SE); JOHANSSON, Henrik, SE-17165 Solna (SE); GRINNEMO, Karl-Henrik, SE-12942 Hägersten (SE)
(74) Representative: Potter Clarkson
(86) International application number: PCT/SE2014/000103
(87) International publication number: WO 2015/016761

(56) References cited:
- WO-A1-2012/125471
- KATARINA LE BLANC ET AL: "Multipotent mesenchymal stromal cells and the innate immune system", NATURE REVIEWS IMMUNOLOGY, vol. 12, no. 5, 25 April 2012 (2012-04-25) , pages 383-396, XP055162760, ISSN: 1474-1733, DOI: 10.1038/nri3209
- CONESE MASSIMO ET AL: "Paracrine Effects and Heterogeneity of Marrow-Derived Stem/Progenitor Cells: Relevance for the Treatment of Respiratory Diseases", CELLS TISSUES ORGANS, vol. 197, no. 6, 4 May 2013 (2013-05-04), pages 445-473, XP009182013,
- GOTTS J E ET AL: "Mesenchymal Stem Cells and Acute Lung Injury", CRITICAL CARE CLINICS, W.B. SAUNDERS, US, vol. 27, no. 3, 1 July 2011 (2011-07-01), pages 719-733, XP009159539, ISSN: 0749-0704, DOI: 10.1016/J.CCC.2011.04.004
- ANDREIA MADEIRA ET AL: "Human Mesenchymal Stem Cell Expression Program upon Extended Ex-Vivo Cultivation, as Revealed by 2-DE-Based Quantitative Proteomics", PLOS ONE, vol. 7, no. 8, 20 August 2012 (2012-08-20) , page e43523, XP055163281, DOI: 10.1371/journal.pone.0043523

## Description

### Technical Field

The present invention relates to, *inter alia,* methods for culturing mesenchymal stem cells (MSCs) (without the use of non-human serum components), MSCs obtained from such culturing methods, MSCs and/or extracellular vesicles derived from said MSCs having proteomic profiles which ensure immuno-modulatory capacity, pharmaceutical compositions comprising such MSCs, and medical treatment and prophylactic methods and medical uses of MSCs and/or extracellular vesicles in a variety of diseases.

### Background Art

Mesenchymal stem cells (MSCs) can be found in *inter alia* the bone marrow, blood, dermis, periosteum and various other tissues and MSCs can differentiate into a variety of cell types, including adipose, areolar, osseous, cartilaginous, elastic, marrow stroma, muscle, fibrous connective tissue, and cardiac tissue, depending upon external milieu and stimulants. Due to their cellular origin and their phenotype, MSC do normally not stimulate adverse immune responses, which enables using unrelated human donors in clinical settings.

MSCs are normally isolated from the tissue, purified, and then expanded in an appropriate culture medium. Suitable culture medium for MSCs may comprise various components to promote MSC expansion, for instance growth factors, cytokines, and serum. After the isolation, purification, and culture expansion steps, the MSCs are washed and centrifuged, followed by freezing in a suitable cryopreservation medium. At the time of treatment, the MSCs are thawed just prior to administration to a patient. The prior art normally discloses expansion processes involving cell culturing in the presence of non-human components (commonly fetal bovine serum (FBS)).

Xenogeneic proteins deriving from the non-human serum may elicit cell-mediated or humoral immune responses (e.g., the generation of anti-bovine serum protein antibodies), which may result in less efficient engraftment of the MSCs, particularly if such xenogeneic proteins become associated with MSC cell-surface membranes. Media comprising autologous human serum have been suggested but this approach has been considered unfeasible when the quantities of cells required in the ultimate MSC product exceed that which can be grown in a fixed amount of autologous serum. Additionally, the use of autologous human serum presupposes that the patient will have sufficient time and be in sufficient health to donate serum in advance of the initiation of MSC therapy. The current conventional MSC culturing process typically requires 2 to 10 weeks to isolate, expand, harvest and purify a suitable number of cells to constitute a pharmaceutical treatment. In some cases a pharmaceutical treatment consists of 1 dose. In other cases a pharmaceutical treatment consists of 2 or more doses. Unfortunately, in some cases, MSC therapy is needed less than about 2 weeks from diagnosis or presentation of clinical symptoms, or in less than about 1 week from diagnosis or presentation of clinical symptoms, or in less than about 48 hours of diagnosis or presentation of clinical symptoms. When MSC therapy is needed within a short time period from diagnosis or presentation of clinical symptoms, MSCs that have already been manufactured, purified and cryopreserved exhibit the significant benefit of being available upon diagnosis or presentation of an acute illness. However, determining clinical therapeutic potency of MSCs remains a challenge.

*In vitro* findings indicate that MSC are immunosuppressive, and rodent, baboon or human MSCs suppress lymphocyte proliferation in mixed lymphocyte cultures, as well as inhibiting the formation of cytotoxic T-cells and NK-cells. The unique immunoregulatory and regenerative properties of MSCs make them an attractive tool for cellular treatment of autoimmunity and inflammation and MSCs have been applied in a variety of clinical contexts, for instance in the treatment of severe graft-versus-host disease (Le Blanc, 2004, Lancet), but also for treating multiple sclerosis and Crohn's disease. However, there are numerous other severe indications for which there is currently a lack of efficient medical treatments, and the present invention thus aims to expand the usage of MSCs, obtained from cell cultures devoid of non-human animal products, to additional indications where there is an unmet medical need. Furthermore, although MSCs are being investigated in several ongoing clinical trials very little is known about what makes MSCs clinically effective, i.e. which characteristics that are necessary for MSCs to be capable of exerting the desired therapeutic effects in a given disease.

Gotts et al. (2011, Critical Care Clinics, W.B. Saunders, US; Volume 27, pages 719-733) is a review article outlining the therapeutic effect of MSCs in the treatment of acute lung diseases.

WO 2012/125471 relates to compositions comprising MSCs-derived exosomes and their use for the treatment of inflammatory lung diseases.

### Summary of the Invention

The present invention is defined in the accompanying claims.

Disclosed herein are methods for culturing mesenchymal stem cells (MSCs) (without the use of non-human serum components), methods for selecting clinically potent MSCs and/or extracellular components (primarily extracellular vesicles such as exosomes) for the treatment of respiratory inflammation and associated conditions, and MSCs and/or extracellular vesicles having clinically efficacious proteomics profiles and immuno-modulatory capacity obtained from such culturing and/or selection methods. The invention relates to pharmaceutical compositions comprising such MSCs and/or vesicles, and medical treatment and prophylactic methods and medical uses of MSCs in a variety of diseases, notably acute respiratory distress syndrome (ARDS) and associated conditions, as defined in the claims.

Disclosed herein is a method for culturing mesenchymal stem cells (MSCs), comprising culturing MSCs (of a suitable origin) in a culture medium comprising lyzed human trombocytes, as well as, in additional aspects, selection methods and immuno-modulation criteria, and cell culture compositions for culturing MSCs and MSCs obtainable by the methods. Further, in yet other aspects, the instant invention pertains to MSCs and extracellular vesicles thereof as such, pharmaceutical compositions, methods for preparing said pharmaceutical compositions, and medical uses involving MSCs and extracellular vesicles, as well as treatment methods involving MSCs. Disclosed herein are methods for selecting clinically effective immuno-modulatory MSCs and related paracrine factors (e.g. extracellular vesicles such as exosomes), by utilizing proteomics profiling and by assessing the functional characteristics of the MSCs. More specifically, the MSCs display certain characteristics in terms of polypeptide expression as well as in terms of polypeptide expression in extracellular fractions (which may comprise extracellular vesicles such as exosomes and, additionally, extracellular polypeptides).

Also disclosed herein is MSCs and/or extracellular vesicles obtainable by the methods disclosed herein.

Also disclosed herein is a cell culture composition for culturing MSCs, wherein the composition comprises a cell culture medium (for instance Dulbecco's Modified Eagles Medium (DMEM)) and lyzed human trombocytes.

Also disclosed herein is a method of preparing a pharmaceutical composition comprising MSCs, comprising the steps of culturing MSCs (preferably of crista iliaca origin and/or sternum origin) in a cell culture composition comprising lyzed human trombocytes, passaging the MSCs in the culture not more than 5 times, and, re-suspending the MSCs in saline solution to a final concentration of between approximately 5×10⁵ and 4×10⁶ MSCs per ml.

In a another aspect, the instant invention pertains to pharmaceutical compositions in accordance with the present invention for use in medicine, specifically, in a seventh aspect, for use in the treatment and/or prophylaxis of acute respiratory distress syndrome (ARDS), infant respiratory distress syndrome (IRDS), pulmonary hypertension (PH), and/or acute organ failure (for instance kidney, liver, and/or heart failure) in connection with ARDS or IRDS.

Also disclosed herein is a mesenchymal stem cell (MSC), wherein the MSCs display a spindle-shape morphology and expressing CD73, CD90, and CD105, and wherein the MSCs are devoid of CD34, CD45, CD14, and CD3. The MSCs may be either positive or negative for HLA-ABC (MHC class I) and/or HLA-DR (MHC class II), for instance depending on the degree of priming/activation of the MSCs. Further, the MSCs may be negative for CD11 b, CD19, and/or CD31.

In a further aspect, the instant invention pertains to a method of treating and/or preventing diseases and/or disorders selected from the group comprising acute respiratory distress syndrome (ARDS), infant respiratory distress syndrome (IRDS), pulmonary hypertension (PH), and acute organ (for instance kidney, liver, and/or heart) failure (optionally in connection with ARDS or IRDS), comprising the steps of providing a therapeutic dose of MSCs, and administering the therapeutic dose to a patient suffering from any one of the abovementioned diseases.

Thus the present invention provides a novel therapeutic modality, based on MSCs displaying a specific proteomics profile, potency in *in vitro* immuno-modulatory assays, cultured via surprisingly advantageous methods, for the treatment of various illnesses where there is currently a significant unmet medical need, notably ARDS, IRDS, PH, and related disorders and illnesses. The polypeptide profiling and the proven immuno-modulatory activity result in MSCs that are clinically effective, in stark contrast to many MSC preparations described in the art. Furthermore, the absence of non-human components during the culturing processes and the advantageous components comprised in the cell culture compositions and in the pharmaceutical compositions enable highly efficacious clinical treatment of these severe illnesses.

### Brief Description of the Drawings

Figure 1. CRP decreased strongly after administration of the MSCs.
Figure 2. Bronchoalveolar lavage (BAL) interleukin-6 (IL-6) decreased post-treatment, evidencing decreased pulmonary inflammation.
Figure 3. Partial oxygen pressure (pO₂) increased significantly from pre-injection to day 3 post-injection and partial carbon dioxide pressure (pCO₂) displayed a concomitant decrease.
Figure 4. Expiratory tidal volume increased in response to MSC administration.
Figure 5. Pulmonary compliance increased significantly post MSC treatment.
Figure 6. Serum levels of RANTES were clearly elevated approximately four weeks after treatment, indicating stem cell mobilization in response to the MSC injection.
Figure 7. Serum creatinine levels decreased considerably upon MSC injection in a patient with acute kidney failure induced by ARDS
Figure 8 and 9. Lung X-rays evidencing significant pulmonary recovery.
Figure 10. Plasma surfactant B levels increased substantially post-treatment, showing recovery of pneumocytes type 2.
Figure 11. (A) In line with the consensus statement of the International Society for Cellular Therapy (ISCT) infused bone marrow-derived MSCs expressed CD73, CD90, and CD105 and lacked expression of CD14, CD34, CD45, and HLA-DR as assessed by flow cytometry (FACS) and shown as histograms. Furthermore, MSCs were negative for the endothelial marker CD31.
Figure 12. Inflammatory cytokines can activate ("prime") MSCs, which is considered a key step (also known as "licensing") for MSCs to efficiently control inflammation. The characteristic up-regulation of ICAM-1 (CD54), VCAM-1 (CD106), HLA-ABC, and HLA-DR upon 48 hours of cell exposure towards the pro-inflammatory IFN-γ and TNF-α was confirmed by FACS on the MSCs.

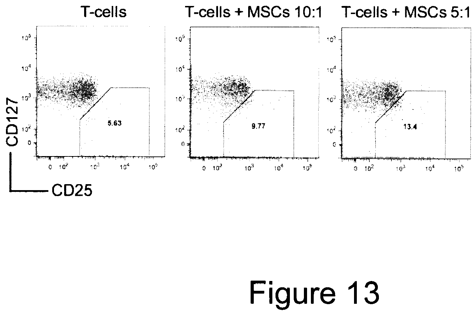

Figure 13. MSCs promote *in vitro* generation of regulatory T-cells. MSCs are capable of promoting the induction and expansion of immune suppressive regulatory T-cells (T_{Regs}). Co-culturing MSCs in different ratios with purified healthy control T-cells led to an increase of the CD25⁺CD127^{dim} T_{Reg}-fraction among the CD4⁺ T-cells. Control T-cells were stimulated using activating anti-CD3 and anti-CD28 microbeads. The left panel shows a representative flow cytometry (FACS) dot plot analysis and the right panel the mean values (columns) of two independent experiments performed in duplicates.

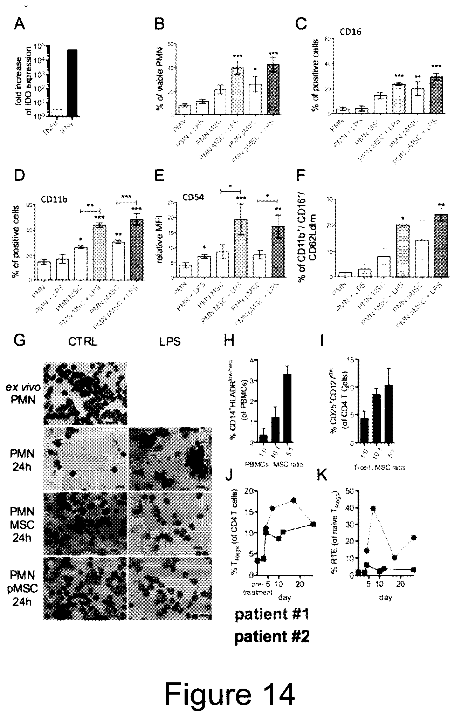

Figure 14. In selected experiments MSCs were exposed to IFN-γ and TNF-α for 48 hours (primed MSCs, pMSCs). After priming, IDO expression was substantially up-regulated as assessed by quantitative PCR **(A).** Unstimulated MSC and pMSC affected viability of control or LPS-stimulated PMN as demonstrated in panel **B. (C)** CD16 (FcγR-III) expression was demonstrated to be used as a marker of PMN viability and matched with the percentage of viable PMNs in all culture conditions. Similarly, CD11b and CD54 expressions were associated with PMN activation status. The percentage of CD11b-positive PMNs was higher in the presence of MSCs and further enhanced by LPS treatment **(D),** while CD54 relative mean fluorescence intensity (rMFI) was up-regulated by LPS treatment and this effect was enhanced by co-culture with MSCs **(E).** Next, the capacity of MSC to promote the generation of myeloid-derived suppressor cells (MDSC) was tested within our co-culture model with PMNs. Co-culture with MSCs led to a marked increase of mature CD11^{bright}/CD16^{bright}/CD62L^{dim} (N2-type) PMNs resembling granulocytic MDSCs **(F). (G)** MSC and PMN co-cultures were stained with May Grunwald-Giemsa dye to observe cell morphology following reciprocal interaction for 24 hours. According to the marked increase of mature CD11^{bright}/CD16^{bright}/CD62L^{dim} (N2-type) PMNs shown at this time by flow-cytometry, PMNs displayed a hypersegmented nuclear morphology after co-culture with both resting (PMN MSC 24 h) and primed MSCs (PMN pMSC 24h). Similarly, co-culturing MSCs with healthy control peripheral blood mononuclear cells (PBMCs) at different ratios promoted CD14⁺HLA-DR^{low} monocytes resembling monocytic MDSCs **(H).** MSCs were also capable of promoting the induction and expansion of immune suppressive CD4⁺CD25^{high}CD127^{low} regulatory T-cells (T_{Regs}), when co-cultured in different ratios with purified healthy control T-cells (I). **(J)** Increased levels of circulating CD4⁺CD25^{high}CD127^{low} T_{Regs}, were also observed up to 20 days following MSC administration. **(K)** This could in part result from increased thymic output as indicated by increased proportion of CD31⁺ recent thymic emigrants (RTE) among CD45RA⁺ naive T_{regs} in patients. Bars indicate the standard error mean. Abbreviations: *p-value≤0.05, **p-value≤0.01, ***p-value≤0.01.

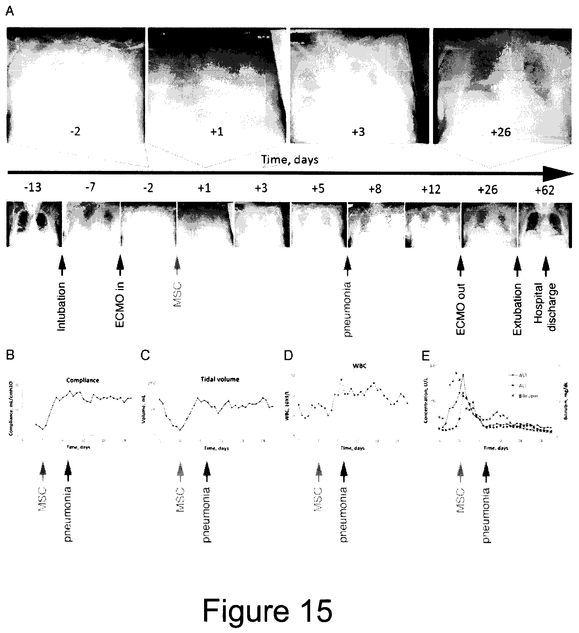

Figure 15. Panel **A** demonstrates the disease progression and concomitant chest-X-rays (CXRs) from the onset of influenza A H1N1, until hospital discharge. As demonstrated, there was a progression of the bilateral opacifications of the lungs after the onset of the influenza infection, and the patient required both mechanical ventilation and ECMO support. By 24 hours after MSC infusion, there was a decrease in pulmonary infiltrates on CXR, followed by progressive improvement until the time of weaning of the ECMO and extubation 4 weeks after MSC administration. In parallel with the improvement in CXRs, both the pulmonary compliance **(B)** and tidal volumes **(C)** improved back to normal levels within the first 2 days. However, 5 days after MSC administration, the patient developed a nosocomial pneumonia with increased white blood cell (WBC) count (D), and new infiltrates on CXR (A), which resolved within 3 days. Liver function also improved during the first week with normalization of serum ALT, AST and bilirubin levels (E).
Figure 16. Panel A demonstrates the progression of ARDS both on CXRs and serial computed tomographic scanning. As demonstrated, there was progressive decrease in pulmonary infiltrates starting the first day after MSC infusion. By day 7, the CXR improved back to normal and the patient was weaned off ECMO the following day and extubation at day 12. (B) Pulmonary compliance and (C) tidal volumes improved back to normal levels during the first week. After MSC infusion, the bone-marrow function continued to be reconstituted with resolution of (D) leukopenia and (E) thrombocytopenia.

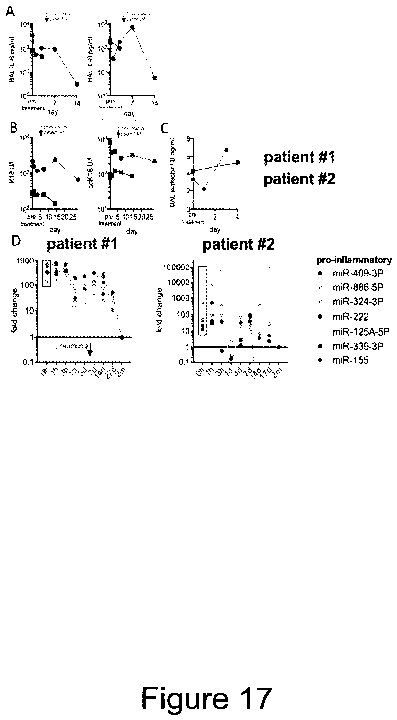

Figure 17. The cytokine response in BAL (A) Levels of caspase-cleaved cytokeratin (ccK)-18, indicative of epithelial apoptosis, and of uncleaved cytokeratin (K)-18, indicative of total epithelial death in BAL fluid, decreased in both patients within a few hours after MSC administration. (B) In parallel, surfactant protein B (SP-B) increased during the first 3 days after MSC infusion, suggesting recovery of the alveolar epithelial function (C). Several miRNAs related to inflammation demonstrated a significant decline within the first 24 hours after MSC administration. A transient increase in these miRNA levels was observed by day 7 in patient one due to pneumonia and returned to normal by day 28. As compared to healthy donors.

### Detailed Description of the Invention

The present invention is disclosed in the accompanying claims. Also disclosed are methods for culturing mesenchymal stem cells (MSCs) (without the use of non-human serum components), methods for selecting clinically potent immuno-modulatory MSCs and/or extracellular components (primarily extracellular vesicles such as exosomes) for the treatment of respiratory inflammation and associated conditions, MSCs and/or extracellular vesicles having clinically efficacious proteomics profiles obtained from such culturing and/or selection methods. The invention relates to pharmaceutical compositions comprising such MSCs and/or vesicles, and medical treatment and prophylactic methods and medical uses of MSCs in a variety of diseases, notably acute respiratory distress syndrome (ARDS) and associated conditions.

Where features, embodiments, or aspects of the present invention are described in terms of Markush groups, a person skilled in the art will recognize that the invention is also thereby described in terms of any individual member or subgroup of members of the Markush group. The person skilled in the art will further recognize that the invention is also thereby described in terms of any combination of individual members or subgroups of members of Markush groups. Additionally, it should be noted that embodiments and features described in connection with one of the aspects and/or embodiments of the present invention also apply mutatis mutandis to all the other aspects, alternatives, and/or embodiments of the invention. For instance, aspects, alternatives, and/or embodiments described in connection with the methods for obtaining clinically effective immuno-modulatory MSCs (e.g. the immuno-modulation criteria) may also be applicable, relevant, and/or combined with teachings pertaining to, for instance, embodiments relating to the MSCs and/or extracellular vesicles obtainable by said methods. By way of another non-limiting example, aspects, alternatives, and/or embodiments described in connection with the method for culturing MSCs in the absence of non-human serum components may also be applicable to other aspects and/or embodiments as per the present invention, for instance the immuno-modulation selection criteria, meaning that the present invention although not explicitly mentioned herein also encompasses combing the teachings relating to, for instance, the culturing methods with the teachings of the immuno-modulation selection methods. Similarly, aspects, alternatives, and/or embodiments described in connection with the MSCs and/or the extracellular vesicles may naturally also be applicable, relevant, and/or combined with teachings pertaining to the pharmaceutical compositions *per se,* which comprises said MSCs and/or extracellular vesicles.

Generally, all polypeptides and/or nucleotides disclosed in the present application naturally encompass polypeptide and/or nucleotide sequences that have at least 50% sequence identity to the polypeptide and/or nucleotide in question, preferably 70% sequence identity to the polypeptide and/or nucleotide in question, more preferably a sequence identity of at least 80%, and even more preferably a sequence identify of at least 90% to the polypeptide and/or nucleotide in question.

For convenience and clarity, certain terms employed herein are collected below. Unless otherwise defined, all technical and scientific terms used herein have the same meaning as commonly understood by one of ordinary skill in the art to which this invention belongs.

The terms "positive for" and "negative for" in the context of the present invention (e.g. an MSC which is "positive for" a polypeptide and/or polynucleotide in question) shall be understood in accordance with the meaning normally given to the term within the biological and medical sciences, in essence a cell that is positive for a certain polypeptide and/or polynucleotide expresses said polypeptide and/or polynucleotide. The polypeptide and/or polynucleotide in question may be identified via various means, for instance using fluorescence-activated cell sorting (FACS) and/or immunohistochemical techniques and/or proteomics techniques such as LC-MS and/or 2D-PAGE. The term "positive for" may in certain instances be understood to comprise cell populations where at least 50% of the cells express the polypeptide (or polynucleotide or any other marker) in question, but preferably at least 70% or even more preferably at least 90% of the population expresses the polypeptide in question. The term "negative for" may, in the same vein, naturally be understood to be the opposite of the term "positive for", i.e. at least 50% - but preferably at least 70% or even more preferably at least 90% - of the cells of the population shall not express the polypeptide (or other suitable marker) in question.

The term "population", which may relate to MSCs or to extracellular vesicles such as exosomes, shall be understood to encompass a plurality of entities constituting a given population, for instance the individual MSCs which when present in a plurality constitute an MSC population. Thus, naturally, the present invention pertains also to the individual cells and vesicles of e.g. an MSC population or a population of extracellular vesicles, respectively.

The terms "subject" and/or "individual" and/or "patient" may be used interchangeably herein and are to be understood to refer broadly to an animal, for instance a human being, from whom cells can be obtained and/or to whom treatment, including prophylaxis or preventative treatment (for instance using the cells as per the present invention) is provided. Advantageously, the subject of the treatments as described in the context of the present invention is a mammal, preferably a human, or other mammals, preferably domesticated or production mammals.

The term "therapeutically effective amount" is to be understood to refer to an amount which results in an improvement, allevation, or remediation of the disease, disorder, or symptoms of the disease or condition.

The terms "administering," "introducing" and "transplanting" are used interchangeably for the purposes of the present invention, for instance in the context of the administration of the MSCs to a patient suffering from any of the diseases and/or disorders mentioned in the context of the present invention. A suitable method or route is one which leads to at least partial localization of the MSCs at a desired site. The cells may be administered (delivered) by any appropriate route which results in delivery of the cells and/or paracrine factors excreted from the cells to a desired location/tissue/site in the subject. The modes of administration suitable for the purposes of the present invention comprise for instance (without limitation) intravenous, intramuscular, intraarterial, intrathecal, intraventricular, intracapsular, intraorbital, intracardiac, intradermal, intraperitoneal, transtracheal, subcutaneous, subcuticular, intraarticular, sub capsular, subarachnoid, intraspinal, intracerebro spinal, and intrasternal injection and infusion.

The phrase "pharmaceutically acceptable excipient" as used herein is to be understood to relate to a pharmaceutically acceptable material, composition or vehicle, for instance a solid or liquid filler, a diluent, an excipient, a carrier, a solvent or an encapsulating material, involved in suspending, maintaining the activity of or carrying or transporting the subject agents from one organ, or portion of the body, to another organ, or portion of the body.

Disclosed herein are methods for obtaining immune-modulatory MSCs. Ensuring that MSCs have clinically effective immuno-modulatory capacity is a crucial but in the prior art frequently overlooked aspect. Disclosed are methods for validating the immuno-modulatory properties of MSCs by methods comprising the steps of (i) culturing MSCs (ii) establishing that the MSC population fulfils at least one of the following immuno-modulation criteria:
(a) the MSC population is positive for at least one of the following polypeptides: vimentin (SEQ ID No 1 ), caldesmon (SEQ ID No 2), annexin A1 (SEQ ID No 3), 14-3-3 protein epsilon (SEQ ID No 4), ADP ribosylation factor 1 (SEQ ID No 5), calnexin (SEQ ID No 6), ADP ribosylation factor 5 (SEQ ID No 7), transforming protein RhoA (SEQ ID No 8), CD44 (SEQ ID No 9), coactosin-like protein (SEQ ID No 10), mitogen-activated protein kinase 3 (SEQ ID No 11 ), insulin-like growth factor-binding protein 7 (SEQ ID No 12), N-acetyl-glucosamine-6-sulfatase (SEQ ID No 13), cellular retinoic acid-binding protein 2 (SEQ ID No 14), transcription elongation factor B polypeptide 1 (SEQ ID No 15), NEDD8 (SEQ ID No 16), fatty acid-binding protein, heart (SEQ ID No 17);
(b) a population of extracellular vesicles derived from the MSC population is positive for at least one of the following polypeptides: serotransferrin (SEQ ID No 18), versican core protein (SEQ ID No 19), annexin A2 (SEQ ID No 20), serine protease HTRA1 (SEQ ID No 21 ), insulin-like growth factor-binding protein 3 (SEQ ID No 22), connective tissue growth factor (SEQ ID No 23), vinculin (SEQ ID No 24), neuroblast differentiation associated protein AHNAK (SEQ ID No 25), microtubule-associated protein 1B (SEQ ID No 26), fatty acid-synthase (SEQ ID No 27), triosephosphate isomerase (SEQ ID No 28), ATP-citrate synthase (SEQ ID No 29), calreticulin (SEQ ID No 30), vigilin (SEQ ID No 31 ), DNA-dependent protein kinase catalytic subunit (SEQ ID No 32), Rab GDP dissociation inhibitor beta (SEQ ID No 33), ATP synthase subunit beta, mitochondrial (SEQ ID No 34);
(c) the MSC population in (a) displays the following order of polypeptide abundance: vimentin > Annexin A1. Preferably, the MSC population exhibits the following order of polypeptide abundance: vimentin > Annexin A1 > CD44 > insulin-like growth factor binding protein 7 > fatty-acid binding protein 3;
(d) the extracellular vesicle population in (b) displays the following order of polypeptide abundance: serotransferrin > annexin A2. Preferably, the extracellular vesicle population displays the following polypeptide abundance: serotransferrin > annexin A2 > connective tissue growth factor
(e) the fold-increase expression of indoleamine 2,3-dioxygenase (IDO) in the MSC population is <10 when the MSCs are primed with 15 ng/mL TNF-alpha;
(f) the fold-increase expression of indoleamine 2,3-dioxygenase (IDO) in the MSC population is >100 when the MSCs are primed with 10 ng/mL IFN-gamma;
(g) the viability of polymorphonuclear neutrophils (PMNs) is increased by at least 20% (preferably at least 30% or even more preferably at least 50%) when co-cultured with MSCs from the MSC population primed with IFN-gamma or TNF-alpha in accordance with (e) or (f);
(h) the number of CD14⁺HLA-DR^{low} monocytes is increased at least 1.5-fold (more preferably at least 2-fold, or even more preferably at least 3-fold) when healthy control human peripheral blood mononuclear cells (PBMCs) are co-cultured with the MSC population primed with IFN-gamma or TNF-alpha in accordance with (e) or (f);
(i) the number of CD4⁺CD25^{high}CD127^{low} regulatory T-cells (T_{Regs}) is increased at least 1.5-fold (more preferably at least 2-fold, or even more preferably at least 3-fold) when healthy control human peripheral blood mononuclear cells (PBMCs) are co-cultured with the MSC population primed with IFN-gamma or TNF-alpha in accordance with (e) or (f).

Furthermore, the extracellular vesicle population obtainable from the MSC population may preferably be negative for (i.e. devoid of) at least one of the following polypeptides: LIM domain only protein7 (SEQ ID No 35), LIM domain and actin-binding protein 1 (SEQ ID No 36), coatomer protein complex, subunit beta 2 (Beta prime), isoform CRA_b (SEQ ID No 37), ribonuclease inhibitor (SEQ ID No 38), PDZ and LIM domain protein 5 (SEQ ID No 39), reticulocalbin-1 (SEQ ID No 40), early endosome antigen 1 (SEQ ID No 41), septin-2 (SEQ ID No 42), actin-related protein 2/3 complex subunit 2 (SEQ ID No 43), septin 11 (SEQ ID No 44).

Naturally, the MSC culture (i.e. the MSC population, the population of extracellular vesicles derived from the MSCs, and both the MSC population and the population of extracellular vesicles derived from the MSCs) may fulfil only one (1) of the above criteria, but preferably several criteria are met. For instance, the culture may meet criteria (a) and (b), (a) and (c), (b) and (c), (a) and (d), (a) and (e), (b) and (d), (a) and (e), (a), (b), and (c), (a), (b), and (d), (b), (c), and (d), (c), (d), and (e), (d) and (e), (a), (f), and (g), (e), (f), and (g), (a), (b), and (g), (a), (c), and (f), etc., etc. Thus, the MSC culture may meet all possible combinations and permutations of the above criteria (a)-(i) without departing from the scope of the present invention.

In essence, based on screening of a large number of MSC cultures the inventors have realized that certain polypeptide profiles of both cells and extracellular components contribute strongly to the therapeutic immuno-modulatory efficacy. Some of the key polypeptide expression features are summarized in the tables below but other polypeptide expression patterns in addition to the ones explicitly mentioned have also been linked to immuno-modulatory properties.

| **Polypeptide Expression** - **Active MSC-derived Extracellular Vesicles** | | | |
|---|---|---|---|
| **Preferably positive for at least one of:** | **SEQ ID No** | **Preferably negative for at least one of:** | **SEQ ID No** |
| Serotransferrin | 18 | LIM domain only protein 7 | 35 |
| Versican core protein | 19 | LIM domain and actin-binding protein 1 | 36 |
| Annexin A2 | 20 | Coatomer protein complex, subunit beta 2 (Beta prime), isoform CRA_b | 37 |
| Serine protease HTRA1 | 21 | Ribonuclease inhibitor | 38 |
| IGFBP3 | 22 | PDZ and LIM domain protein 5 | 39 |
| Connective tissue GF | 23 | Reticulocalbin-1 | 40 |
| Vinculin | 24 | Early endosome antigen 1 | 41 |
| Neuroblast differentiation-associated protein AHNAK | 25 | Septin-2 | 42 |
| Microtubule-associated protein | 26 | Actin-related protein 2/3 complex subunit 2 | 43 |
| Fatty acid-synthase | 27 | Septin 11 | 44 |
| Triosephosphate isomerise | 28 | | |
| ATP-citrate synthase | 29 | | |
| Calreticulin | 30 | | |
| Vigilin | 31 | | |
| DNA-dependent protein kinase catalytic subunit | 32 | | |
| Rab GDP dissociation inhibitor beta | 33 | | |
| ATP synthase subunit beta, mitochondrial | 34 | | |

| **Polypeptide Abundance Patterns (Higher-to-Lower) of Active MSCs & Extracellular Vesicles** | |
|---|---|
| **MSCs** | **Extracellular Vesicles** |
| Vimentin < Annexin A1 | Serotransferrin < Annexin A2 |
| Vimentin < Caldesmon | Serotransferrin < Versican core protein |
| Vimentin < Transforming protein RhoA | Serotransferrin < Annexin A2 < Connective tissue growth factor |
| Vimentin < CD44 | Serotransferrin < Vinculin |
| Vimentin < Annexin A1 < CD44 | Serotransferrin < Annexin A2 < Vinculin |

The sources of the MSCs as per the present invention may be selected from the group comprising bone marrow, cord blood, amniotic tissue, Wharton's jelly, tooth bud, adipose tissue, embryonic or fetal material, but various other sources of MSCs could also be applicable. MSCs of bone marrow origin are normally of crista iliaca origin and/or of sternum origin.

Importantly, in order to retain the immuno-modulatory potential of the MSCs (for instance the order of abundance between vimentin and annexin A1, or any of the other abundance patterns of the MSCs and/or the extracellular vesicles referred to above), the MSCs shall preferably not be passaged more than 5 times before clinical use.

Also disclosed herein is a population of immune-modulatory MSCs obtainable by the methods of the present invention.

Also disclosed herein is a population of immune-modulatory MSCs having the following antigen profile: CD73+, CD90+, CD105+, CD34-, CD45-, CD14-, and CD3-. In a further embodiment, the MSC population may be positive for vimentin and/or Annexin A1, and further positive for insulin-like growth factor binding protein 7 and/or fatty-acid binding protein 3 and/or Annexin A1.

MSCs are normally defined as functional and biologically active through a colony unit forming (CFU) test and differentiation into adipocytes (fat cells), osteoblasts (bone cells), and chondrocytes (cartilage cells). Madeira and co-authors (PLOS One, 2012) have compared biologically active and inactive MSCs and found that in inactive cells, expression of annexin A1 is upregulated 1.5 fold. Annexin A1 is a known apoptosis-related protein, which impacts adaptive and innate immunity. In contrast, expression of vimentin, which is a cellular cytoskeleton component, is downregulated 2.5 fold in biologically inactive MSCs in comparison with that in the active ones. This probably reflects downregulation in proliferation capacity of the inactive MCSs. However, what the inventors of the present inventions have unexpectedly found is that it is clearly preferential with a larger abundance of vimentin (and related polypeptides) than of annexin A1 (and related polypeptides). Similarly, other abundance patterns have been shown in accordance with the present invention to result in enhanced immuno-modulatory potency, for instance in the context of the whole cells lysates: Vimentin < Caldesmon, Vimentin < CD44, and Vimentin < Annexin A1 < CD44. And, in the context of the lysate of the fraction containing extracellular vesicles: Serotransferrin < Versican core protein, Serotransferrin < Annexin A2 < Connective tissue growth factor, and Serotransferrin < Annexin A2 < Vinculin. Importantly, the different polypeptide profiles of the MSCs and the extracellular vesicles may co-exist, for instance in that the whole cell lysate (i.e. the MSC polypeptide pattern) may display a greater abundance of vimentin than of Annexin A1 , whereas the extracellular vesicle fraction derived from the MSC population exhibits a greater abundance of serotransferrin than of Annexin A2.

Naturally, the abovementioned profiles may be detected/assessed either at the point of obtaining the material from a donor, at various time points during the expansion/culturing of the MSCs prior to clinical application, at various time points after the cells have been cultured *in vitro,* and/or at the point when it is time to administer the MSCs and/or extracellular vesicles to a patient to be treated.

In a further embodiment, the MSC population may be positive for the CD44 antigen, and CD44 may advantageously be present in a lower abundance than Annexin A1.

As abovementioned, the MSC population in accordance with the present invention meet at least one of the following immuno-modulation critera:
(a) the MSC population is positive for at least one of the following polypeptides: vimentin (SEQ ID No 1), caldesmon (SEQ ID No 2), annexin A1 (SEQ ID No 3), 14-3-3 protein epsilon (SEQ ID No 4), ADP ribosylation factor 1 (SEQ ID No 5), calnexin (SEQ ID No 6), ADP ribosylation factor 5 (SEQ ID No 7), transforming protein RhoA (SEQ ID No 8), CD44 (SEQ ID No 9), coactosin-like protein (SEQ ID No 10), mitogen-activated protein kinase 3 (SEQ ID No 11), insulin-like growth factor-binding protein 7 (SEQ ID No 12), N-acetyl-glucosamine-6-sulfatase (SEQ ID No 13), cellular retinoic acid-binding protein 2 (SEQ ID No 14), transcription elongation factor B polypeptide 1 (SEQ ID No 15), NEDD8 (SEQ ID No 16), fatty acid-binding protein, heart (SEQ ID No 17);
(b) a population of extracellular vesicles derived from the MSC population is positive for at least one of the following polypeptides: serotransferrin (SEQ ID No 18), versican core protein (SEQ ID No 19), annexin A2 (SEQ ID No 20), serine protease HTRA1 (SEQ ID No 21), insulin-like growth factor-binding protein 3 (SEQ ID No 22), connective tissue growth factor (SEQ ID No 23), vinculin (SEQ ID No 24), neuroblast differentiation associated protein AHNAK (SEQ ID No 25), microtubule-associated protein 1B (SEQ ID No 26), fatty acid-synthase (SEQ ID No 27), triosephosphate isomerise (SEQ ID No 28), ATP-citrate synthase (SEQ ID No 29), calreticulin (SEQ ID No 30), vigilin (SEQ ID No 31), DNA-dependent protein kinase catalytic subunit (SEQ ID No 32), Rab GDP dissociation inhibitor beta (SEQ ID No 33), ATP synthase subunit beta, mitochondrial (SEQ ID No 34);
(c) the MSC population in (a) displays the following order of polypeptide abundance: vimentin > annexin A1;
(d) the extracellular vesicle population in (b) displays the following order of polypeptide abundance: serotransferrin > annexin A2.
(e) the fold-increase expression of indoleamine 2,3-dioxygenase (IDO) in the MSC population is <10 when the MSCs are primed with 15 ng/mL TNF-alpha;
(f) the fold-increase expression of indoleamine 2,3-dioxygenase (IDO) in the MSC population is >100 when the MSCs are primed with 10 ng/mL IFN-gamma;
(g) the viability of polymorphonuclear neutrophils (PMNs) is increased by at least 20% (preferably by at least 30%) when co-cultured with MSCs from the MSC population primed with IFN-gamma or TNF-alpha in accordance with (e) or (f);
(h) the number of CD14⁺HLA-DR^{low} monocytes is increased at least 1.5-fold (preferably 2-fold or 3-fold) when healthy control human peripheral blood mononuclear cells (PBMCs) are co-cultured with the MSC population primed with IFN-gamma or TNF-alpha in accordance with (e) or (f);
(i) the number of CD4⁺CD25^{high}CD127^{low} regulatory T-cells (T_{Regs}) is increased at least 1.5-fold (preferably 2-fold or 3-fold) when healthy control human peripheral blood mononuclear cells (PBMCs) are co-cultured with the MSC population primed with IFN-gamma or TNF-alpha in accordance with (e) or (f).

Similarly, as mentioned in connection with the immuno-modulation criteria above and also as exemplified by e.g. the above tables, the population of the extracellular vesicles obtainable from the MSCs as per the present invention is preferably negative for (i.e. devoid of) the following polypeptides: LIM domain only protein7 (SEQ ID No 35), LIM domain and actin-binding protein 1 (SEQ ID No 36), coatomer protein complex, subunit beta 2 (Beta prime), isoform CRA_b (SEQ ID No 37), ribonuclease inhibitor (SEQ ID No 38), PDZ and LIM domain protein 5 (SEQ ID No 39), reticulocalbin-1 (SEQ ID No 40), early endosome antigen 1 (SEQ ID No 41 ), septin-2 (SEQ ID No 42), actin-related protein 2/3 complex subunit 2 (SEQ ID No 43), septin 11 (SEQ ID No 44).

Also disclosed is a vesicle population of extracellular vesicles derived from the MSC population in accordance with the present invention. The vesicle population preferably comprises extracellular vesicles in the form of exosomes.

As above-mentioned, the MSCs as per the present invention (i.e. the MSC population, the population of extracellular vesicles derived from the MSCs, and both the MSC population and the population of extracellular vesicles derived from the MSCs) may fulfil only one (1) of the above criteria, but preferably several criteria are met. For instance, the culture may meet criteria (a) and (b), (a) and (c), (b) and (c), (a) and (d), (a) and (e), (b) and (d), (a) and (e), (a), (b), and (c), (a), (b), and (d), (b), (c), and (d), (c), (d), and (e), (d) and (e), (a), (f), and (g), (e), (f), and (g), (a), (b), and (g), (a), (c), and (f), etc., etc. Thus, the MSC culture may meet all possible combinations and permutations of the above criteria (a)-(i) without departing from the scope of the present invention.

Naturally, all the expression features and patterns mentioned in connection with the immuno-modulation criteria above also apply to the MSCs and/or the extracellular vesicles as such.

Extracellular vesicles and other paracrine factors are of great importance for the therapeutic efficacy of MSCs and the vesicle population thus meets the above-mentioned immuno-modulation criteria, namely that the vesicle population may be positive for at least one of the following polypeptides: serotransferrin (SEQ ID No 18), versican core protein (SEQ ID No 19), annexin A2 (SEQ ID No 20), serine protease HTRA1 (SEQ ID No 21 ), insulin-like growth factor-binding protein 3 (SEQ ID No 22), connective tissue growth factor (SEQ ID No 23), vinculin (SEQ ID No 24), neuroblast differentiation associated protein AHNAK (SEQ ID No 25), microtubule-associated protein 1B (SEQ ID No 26), fatty acid-synthase (SEQ ID No 27), triosephosphate isomerise (SEQ ID No 28), ATP-citrate synthase (SEQ ID No 29), calreticulin (SEQ ID No 30), vigilin (SEQ ID No 31 ), DNA-dependent protein kinase catalytic subunit (SEQ ID No 32), Rab GDP dissociation inhibitor beta (SEQ ID No 33), ATP synthase subunit beta, mitochondrial (SEQ ID No 34). And, as above-mentioned, the population of extracellular vesicles is preferably negative for (i.e. devoid of) the following polypeptides: LIM domain only protein7 (SEQ ID No 35), LIM domain and actin-binding protein 1 (SEQ ID No 36), coatomer protein complex, subunit beta 2 (Beta prime), isoform CRA_b (SEQ ID No 37), ribonuclease inhibitor (SEQ ID No 38), PDZ and LIM domain protein 5 (SEQ ID No 39), reticulocalbin-1 (SEQ ID No 40), early endosome antigen 1 (SEQ ID No 41 ), septin-2 (SEQ ID No 42), actin-related protein 2/3 complex subunit 2 (SEQ ID No 43), septin 11 (SEQ ID No 44).

The inventors have unexpectedly realized that in order to optimize immuno-modulatory capacity the vesicle population may preferably have a greater abundance of annexin A2 than of serotransferrin, and preferably greater abundance of annexin A2 than of connective tissue growth factor. Additional abundance patterns which are important for the MSCs and the extracellular vesicles immuno-modulatory capacity are also mentioned above.

In another aspect, the present invention pertains to a pharmaceutical composition comprising the MSC population and/or the extracellular vesicle population as defined in the claims. In a further embodiment, the pharmaceutical composition may further comprise plasma of blood type AB, preferably at a concentration of at least around 1 %, preferably around 10%. Furthermore, the MSCs are preferably present at a final concentration of between approximately 5×10⁵ and 4×10⁶ MSCs per ml of the pharmaceutical composition.

In yet another aspect, the present invention pertains to the MSC population and/or the vesicle population and/or the pharmaceutical composition for use in medicine.

In yet another aspect, the present invention pertains to the MSC population and/or the vesicle population and/or the pharmaceutical composition for use in the treatment of acute respiratory distress syndrome (ARDS), infant respiratory distress syndrome (IRDS), pulmonary hypertension (PH), or acute organ failure (for instance kidney, liver and/or heart failure) in connection with ARDS or IRDS.

In one embodiment, the MSC population and/or the vesicle population and/or the pharmaceutical composition may be used in the treatment of ARDS and/or acute organ failure in connection with ARDS through administration via a peripheral intravenous injection, central venous injection into the right atrium, injection into the right ventricle of the heart, and/or injection into the pulmonary trunk/artery

Patients that suffer from the above diseases/disorders and that are eligible for and/or are undergoing extra-corporal membranous oxygenation (ECMO) treatment are particularly suitable for treatment using the MSCs and/or the extracellular vesicles and/or the pharmaceutical compositions as per the present invention. Furthermore, one subgroup of patients where the therapeutic efficacy is optimal is the group of patients has having elevated levels of caspase-cleaved cytokeratin-18 (ccK18), as an indicator of epithelial apoptosis. Additionally, another subpopulation of patients is the patient group having elevated levels of at least one of the following microRNAs (miRs): miR-409-3P, miR-886-5P, miR-324-3P, miR-222, miR-125A-5P, miR-339-3P, and/or miR-155 (Figure 17). In these patients the treatment has been shown to be particularly effective and the clinical outcomes have been excellent.

Also disclosed is a method for culturing mesenchymal stem cells (MSCs). The culturing method does not utilize any non-human components and therefore greatly reduces the risk of eliciting immune responses, thereby possibly improving engraftment and therapeutic outcomes. In a first step, mesenchymal stem cells are obtained from a suitable source, followed by culturing the cells in a culture medium comprising lyzed human trombocytes (platelets), preferably at least 10⁷ (or even higher numbers, such as 10⁸ or 10⁹) lyzed human trombocytes per ml of culture medium.

MSCs may be obtained from various tissues, for instance bone marrow, blood, dermis, and/or the periosteum, using either allogeneic or autologous sources. When using an allogeneic source of MSCs it is of utmost importance to obtain cells from a young, healthy donor, who is preferably 35 years of age, preferably under 30 years of age, even more preferably under 25 years of age, and most preferably under 20 years of age.

In a further embodiment, when the MSCs are to be utilized for medical treatment of a patient suffering from any of the diseases and disorders that may be prevented, treated, cured or alleviated by MSCs, the cells and/or the extracellular vesicles may be harvested (and subsequently used for therapeutic treatment) when the population of MSCs have reached preferably at most 750×10⁶ cells, preferably at most 500×10⁶ cells, starting from at most 60 ml (cm³) of bone marrow aspirate. Thus, the entire procedure from obtaining the cells to the preparing a pharmaceutical composition may entail aspirating not more than 60 ml (preferably not more than 40 ml and even more preferably not more than 30 ml) from the bone marrow of a healthy donor (preferably from crista iliaca and/or sternum), expanding the MSCs obtained from the aspirate to preferably not more than 750^{∗}10⁶ cells, and, finally, harvesting the cells to prepare a pharmaceutical composition to be administered to a patient. The MSCs and/or the extracellular vesicles derived from the MSCs obtained by the methods disclosed herein display a highly surprising level of therapeutic potency and a significantly smaller number of cells may hence be administered to a patient without reducing the therapeutic efficacy, thereby simplifying and speeding up the entire procedure. The key behind the therapeutic potency is the fact that the MSCs and/or the extracellular vesicles may be selected based on the immuno-modulatory criteria of the present invention, meaning that the MSCs and/or the extracellular components have a specific, advantageous proteomics profile and immuno-modulatory capacity. Another key aspect behind the enhanced therapeutic potency lies in the lower number of cell divisions the MSCs in accordance with the present invention have undergone, meaning that their beneficial immuno-modulating characteristics are conserved. When MSCs are obtained from the donor they may be frozen immediately, followed by thawing and starting of the cell culture upon the need for therapeutic intervention (alternatively, cells may be obtained (and cultivated directly) from a donor when a clinical need for MSCs arises). Surprisingly, it is crucial to not cultivate the MSCs for any longer period of time, preferably less than 5 passages (after obtaining the cells from the donor or after thawing), and even more preferably not more than 3 or 2 passages, in order to conserve the potency of the MSCs. The limited expansion of the bone marrow aspirate to not more than 750×10⁶ cells from not more than 60 ml (preferably not more than 50 ml, preferably not more than 40 ml, or even more preferably not more than 40 ml), and the limited number of passages, represent a completely novel approach in comparison to the existing art, with clearly beneficial therapeutic effects.

Also disclosed is a cell culture composition for culturing MSCs. The cell culture composition comprises a cell culture medium (for instance Dulbecco's Modified Eagles Medium (DMEM), having a glucose concentration of between 0.5 and 2 g/l (preferably 1 g/l) (e.g. Gibco, InVitrogen Corp, # 31885-023)), and lyzed human trombocytes (platelets), preferably at least 10⁷ lyzed platelets per ml of cell culture composition. The inclusion of lyzed human trombocytes is highly advantageous as it reduces the risk of immune responses to foreign antigens introduced when using conventional non-human serum (e.g. fetal calf serum). The platelet-containing culture medium/composition may be obtained using the following method: (1) isolating platelets from human blood (using conventional isolation techniques), (2) lyzing the platelets (preferably using radiation, e.g. ionizing radiation at around 24 Gy), (3) mixing the platelet lyzate and heparin, (4) mixing the platelet-heparin mixture with DMEM or any other suitable cell culture medium (the medium preferably containing suitable additives, such as antibiotics and/or antimycotics), and (5) centrifuging to eliminate any aggregates (normally at approximately 900 g for approximately 10 minutes).

The cell culture composition may comprise additional components, such as conventional antibiotic and antimycotic drugs (e.g. antibiotic/antimycotic, 100× Gibco, #15240-062), nutrients, or other additives. The DMEM normally comprises the standard ingredients of low glucose DMEM, namely amino acids, salts (normally calcium chloride, potassium chloride, magnesium sulfate, sodium chloride, and monosodium phosphate), glucose, iron, and vitamins (typically folic acid, nicotinamide, riboflavin, and vitamin B12).

In a further aspect, the present invention relates to a pharmaceutical composition for use comprising between 5×10⁵ and (500,000) and 3×10⁶ (3,000,000) MSCs per ml in a pharmaceutically acceptable carrier. The MSCs in the pharmaceutical composition may advantageously be obtained by the methods disclosed herein but the MSCs may alternatively be obtained using other suitable methods.

In one embodiment, the pharmaceutical composition may further comprise plasma of blood type AB. Surprisingly, plasma of blood type AB may advantageously be used irrespective of the blood type of the patient to which the pharmaceutical composition is to be administered. Without wishing to be bound by any theory, it is surmised that the absence of antibodies against either A or B antigens is advantageous when administering the pharmaceutical composition to a patient.

The plasma (preferably of blood type AB) may be present at any concentration above 1%, preferably around 10%. The plasma may preferably be obtained fresh, normally cryo-reduced, and subsequently stored at -20°C until use. In one embodiment, the pharmaceutically acceptable carrier may be an aqueous solution comprising at least 5% w/v sodium chloride, but other pharmaceutically and physiologically acceptable carriers may also be employed. The concentration of sodium chloride in the aqueous solution is preferably around 9% w/v.

In further embodiments, the MSCs and/or the extracellular components of the pharmaceutical composition may be from an allogeneic and/or an autologous cell source. If the cells of the pharmaceutical composition are obtained from an allogeneic donor it is crucial that the donor is a young, healthy individual under 30 years of age, preferably under 25 years of age, and even more preferably under 20 years of age.

Also disclosed is a method for preparing a pharmaceutical composition comprising MSCs, comprising the following steps of culturing MSCs of crista iliaca and/or sternum origin in a cell culture composition comprising lyzed human trombocytes, passaging said MSCs not more than 5 times, and,re-suspending the MSCs so obtained in saline solution to a final concentration of between approximately 5×10⁵ and 4×10⁶ MSCs per ml.

The pharmaceutical composition may be obtained by aspirating between 20-60 ml (e.g. 20, 25, 30, 35, 40, 45, 50, 55, or 60 ml) of bone marrow from either sternum or Crista iliaca using an aspirate needle that is inserted through the skin using manual pressure until the needle hits the bone. When the needle hits the bone, with a twisting motion of the hand the needle is advanced through the bone cortex and into the marrow cavity. Once the needle is in the marrow cavity, a syringe is attached and used to aspirate liquid bone marrow. A twisting motion may preferably be performed during the aspiration to avoid excess content of blood in the sample, which might be the case if an excessively large sample from one single point is taken.

After aspiration, the cells are normally washed in a suitable liquid, for instance phosphate-buffered saline (PBS), and the washed cells may then be re-suspended in Dulbecco's modified Eagle's medium-low glucose supplemented with trombocyte (platelet) lysate and plated at a density of e.g. 160 000 cells per cm². Cultures are maintained at 37°C in a humidified atmosphere containing 5% CO₂, preferably in 175 cm² flasks. When the cultures are near confluence (>80%), the cells may be detached by treatment with trypsin and EDTA and replated at a density of 4000 cells per cm². When approximately 2×10⁶ cells or more are obtained, the cells are harvested and either cryopreserved in 10% dimethyl sulphoxide (DMSO) or washed repeatedly with PBS and re-suspended to a final concentration of between approximately 5×10⁵ and 4×10⁶ cells per ml in saline solution. The saline solution into which the MSCs are re-suspended may preferably comprise blood plasma, preferably plasma of blood type AB.

After aspiration of the MSCs from the bone marrow of a donor, it is crucial to not cultivate the MSCs for any longer period of time, preferably less than 5 passages (after obtaining the cells from the donor or after thawing), and even more preferably not more than 3 or 2 passages, in order to conserve the potency of the MSCs Various criteria for release of MSCs for clinical use need to be met, including the absence of visible clumps and the absence of contamination by pathogens (as documented by aerobic and anaerobic cultures before release), the MSCs shall preferably display a spindle-shape morphology, have a viability that preferably is greater than 95%, and immune phenotyping shall preferably show expression of CD73, CD90, and CD105 surface molecules (>90%) and absence of CD34, CD45, CD14, and CD3.

In yet additional embodiments, the pharmaceutical composition as per the present invention may further comprise extracellular vesicles (for instance exosomes) obtainable from the MSCs. Exosomes may be added to the pharmaceutical composition from a separate culture of MSCs or they may be derived from the MSCs that are themselves to be included in the pharmaceutical composition.

To optimize the properties of the pharmaceutical composition and to avoid coagulation upon injection, the pharmaceutical composition may comprise heparin or any other anticoagulation factor.

The present invention, in a further aspect, thus relates to the use of the pharmaceutical compositions according to the present invention for use in medicine, and specifically in the treatment of diseases and disorders such as acute respiratory distress syndrome (ARDS), infant respiratory distress syndrome (IRDS), pulmonary hypertension (PH), congenital heart diseases, and acute organ failure (optionally in connection with ARDS and/or IRDS), for instance heart failure, kidney failure, and/or liver failure.

Acute respiratory distress syndrome (ARDS) is an important cause of acute respiratory failure and is often associated with multiple organ failure. Several clinical disorders can precipitate ARDS, for instance viral and/or bacterial pneumonia, aspiration of gastric contents, sepsis, surgery, and major trauma. The clinical criteria for ARDS are normally the following:
- Acute onset: 20-50% of acute lung injury patients will develop ARDS within 7 days.
- Capillary wedge pressure (PCWP) ≤18 mmHg or no evidence of cardiac failure. Chest X-ray shows bilateral infiltrates.
- Refractory hypoxaemia: ARDS is present when PaO₂:FiO₂ <200.
- Physiologically, ARDS is characterized by increased permeability pulmonary edema, severe arterial hypoxemia, and impaired carbon dioxide excretion and is the result of an on-going inflammatory response.
- Clinically, up-regulation of inflammatory cytokines frequently persists in the patients.

Infant respiratory distress syndrome (IRDS) of the newborn is the most common cause of respiratory distress in premature infants, correlating with structural and functional lung immaturity. The pathophysiology is characterized by an ongoing inflammatory response giving immature type II alveolar cells that produce less surfactant, causing an increase in alveolar surface tension and a decrease in compliance. The resultant atelectasis causes pulmonary vascular constriction, hypoperfusion, and lung tissue ischemia. Hyaline membranes form through the combination of sloughed epithelium, protein, and edema. Persistent respiratory distress syndrome leads to bronchopulmonary dysplasia, characterized by typical chest radiography findings and chronic oxygen dependence.

Pulmonary hypertension (PH) is an increase in blood pressure in the pulmonary artery, pulmonary vein, and/or the pulmonary capillaries and it can be a severe disease with a markedly decreased exercise tolerance and heart failure. Evidence is accumulating to suggest that inflammation plays a significant role in the pathogenesis of PH. Endothelial cells play an important role in inflammation and immune reactions, and inflammatory cytokines cause endothelial dysfunction. Endothelial dysfunction is a hallmark of PH, consisting in reduced availability of vasodilators and antiproliferative factors and increased production of vasoconstrictors and vascular proliferative factors. Up-regulation of inflammatory cytokines and perivascular inflammatory cell infiltration have been detected in the lungs of patients with PH. Persistent PH of the newborn occurs when pulmonary vascular resistance fails to decrease soon after birth as with normal transition. The etiology may be idiopathic or secondary to meconium aspiration syndrome, pneumonia or sepsis, respiratory distress syndrome, or transient tachypnea of the newborn. The increased pulmonary hypertension gives rise to an ongoing inflammatory response in the lung.

Congenital heart disorders giving rise to cyanotic heart disease includes transposition of the great arteries and Tetralogy of Fallot (TOF). Noncyanotic heart lesions that cause a pulmonary overflow state leading to congestive heart failure. These lesions include large septal defects, patent ductus arteriosus, and coarctation of the aorta. The increased hypertension as well as cyanosis gives rise to an inflammatory response in the lung.

Thus, the common denominator between the various diseases and disorders in accordance with the present invention is the inflammatory aspect, either in (1) the pulmonary system as such, (2) emanating from the pulmonary system and involving other parts of the body such as the kidney (e.g. kidney or liver failure), or (3) emanating from some other body part and involving the pulmonary system (e.g. as in congenital heart disorders).

The present inventors have unexpectedly discovered that the administration route of the MSCs is important to achieve therapeutic efficacy. For instance, in the treatment of acute respiratory distress syndrome (ARDS), a pharmaceutical composition comprising MSCs and/or extracellular vesicles derived from said MSCs may preferably be administered via peripheral intravenous injection, central venous injection into the right atrium, injection into the right ventricle of the heart, and/or injection into the pulmonary trunk/artery. Specifically, the pharmaceutical compositions as per the present invention display considerable therapeutic efficacy in the patient group that (1) suffer from ARDS, IRDS, PH, or any other pulmonary disease or disorder within the scope of the present invention, and (2) are eligible and/or are undergoing extra-corporal membranous oxygenation (ECMO) treatment. The pharmaceutical compositions in accordance with the present invention can thus be administered both to patients that have already been placed on ECMO support, and to patients that are eligible but have not yet commenced ECMO treatment. Furthermore, as above-mentioned, subjects having certain miRNA profiles are particularly eligible for treatment. More specifically, patients having upregulated levels of at least one of the following microRNAs (miRs) respond to treatment in a surprisingly rapid and stable manner: miR-409-3P, miR-886-5P, miR-324-3P, miR-222, miR-125A-5P, miR-339-3P, and/or miR-155 (Figure 17). Furthermore, another subgroup of patients where the therapeutic efficacy is optimal is when the subject has elevated levels of caspase-cleaved cytokeratin-18 (ccK18), as an indicator of epithelial apoptosis.

In one embodiment, in patients that are placed on ECMO support, the administration mode and route of the pharmaceutical compositions may be optimized by reducing the forward flow of the ECMO to zero, either by turning off the machine and/or by clamping a suitable part of the machine to stop the flow. By reducing the flow rate to zero for a time sufficient to permit administration of the pharmaceutical composition comprising the MSCs and/or the extracellular vesicles obtainable from the MSCs the engraftment and therapeutic efficacy may be enhanced.

In a further aspect, the present invention relates to a method of preventing, treating, and/or alleviating diseases and/or disorders selected from the group comprising ARDS, IRDS, acute organ (e.g. kidney, heart, and/or liver) failure (optionally in connection with IRDS and/or ARDS), and congenital heart diseases (with pulmonary involvement). The treatment methods may comprise the steps of providing a therapeutic dose of MSCs and/or extracellular vesicles obtainable from said MSCs, and administering said therapeutic dose to a patient in need thereof. In one embodiment, the therapeutic dose of MSCs and/or exosomes obtainable from said MSCs comprises at least 500,000 MSCs per kg of body weight, preferably at least 1,000,000 MSCs per kg of body weight, or even more preferably at least 1,500,000 MSCs per kg of body weight.

### Experiments and trials

### Materials and methods

Peripheral blood mononuclear cells (PBMCs) used in *in vitro* studies were retrieved from patient and from healthy donors, isolated by density gradient-based centrifugation, and stored in 10% DMSO in liquid nitrogen until further analysis. For further purification of T-cells, a paramagnetic bead-based selection was utilized (Miltenyi Biotec, Bergisch Gladbach Germany). Bone marrow-derived MSCs (BM-MSCs) were co-cultured with allogeneic T-cells (MSC:T-cell ratio 1:5, 1:10) for 5 days. T-cells were stimulated with activating anti-CD2/CD3/CD28 antibodies (Miltenyi Biotec) in a 0.5 bead per cell ratio. PBMCs were cultured for five days in the presence of MSCs (MSC:PBMC ratio 1:5 and 1:10) and the monocytic compartment was subsequently analyzed.

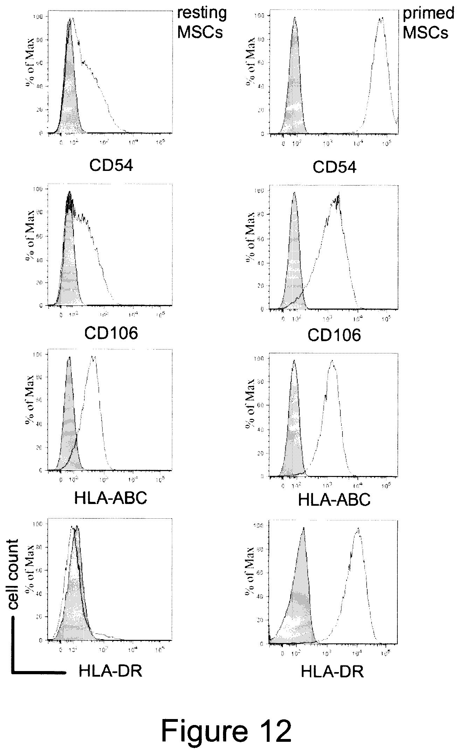

Polymorphonuclear leukocytes (PMNs) from buffy coats of healthy donors were ultrapurified under endotoxin-free conditions, as previously described. PMNs (> 95% purity) and MSCs were co-cultured for up to 40h in presence or absence of 100 ng/ml of LPS. In all cases, MSC were plated 72 hours before the start of co-cultures. In selected experiments, MSCs were pre-treated with recombinant human IFN-γ (10 ng/ml) and TNF-α (15 ng/ml) for 48h (Figure 12), gently washed twice with fresh medium to remove residual cytokines, and finally co-cultured with freshly isolated PMNs. MSC and PMN cocultures were stained with May Grunwald-Giemsa dye to observe cell morphology following reciprocal interaction.

### BAL fluid and serum analyses

Levels of pro- and anti-inflammatory cytokines in serial samples of BAL fluid and serum were assessed using a multiplex cytokine assay (Millipore, MA, US) on a Luminex machine (Luminex, Millipore). In BAL fluid, surfactant protein B concentration was determined by enzyme-linked immunosorbant assay (ELISA) (Uscn Life Science Inc, Houston, US). Caspase-cleaved K18 (ccK18) and total K18 were measured using M30-Apoptosense^{®} ELISA (Peviva AB, Bromma, Sweden) and M65 EpiDeath^{®} ELISA (Peviva AB), respectively.

### miRNA analysis

The EV purification and miRNA isolation was performed by Exosome Diagnostic Inc. (NY, US). In brief, 1.5-2 ml of blood sample from each time point was purified using the EXO50 (Exosome Diagnostics, Martinsried, Germany). The eluted RNA was processed for microRNA analysis using the Low Sample Input protocol for the TaqMan^{®} OpenArray^{®} Human MicroRNA Panel (Life Technologies, US). Megaplex^{™} RT Primers, Human Pool A and Human Pool B were used for reverse transcription followed by a pre-amplification step according to manufacturer's protocol. The pre-amplified material was diluted 1:20 in TE (pH 8.0) and analyzed on the TaqMan OpenArray Human MicroRNA Panel for final qPCR detection. In total 758 miRNAs were assayed and 200 to 300 miRNAs gave a detectable CT value for each time point.

U6 was used to normalize for sub-array-to-sub-array variation and for differences between two Megaplex pools, because this RNA was present in every sample and generated Ct values in a reliable range between 12 and 22. Delta Ct values were calculated for each miRNA by subtracting the U6 Ct value located on the same sub-array. Next, we corrected for variation of the total RNA amount. We therefore subtracted each Ct by the median Ct value of 128 miRNAs present in all samples in the first patient and by the median Ct of 79 miRNAs present in all samples in the second patient. This procedure was performed for all samples. Missing values were entered with the highest observed normalized Ct value per patient. Finally, the fold change for every miRNA relative to the normal healthy control sample was calculated.

### Expansion of mesenchymal stem cells

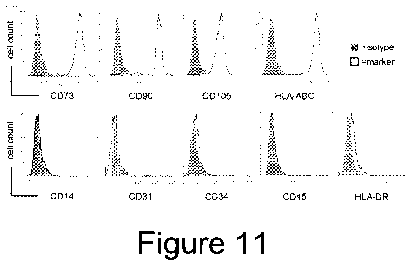

Twenty-eight ml of bone marrow was aspirated from a 49 year old HLA-mismatched third party healthy male volunteer. Clinical-grade MSCs were generated under good manufacturing practice (GMP) conditions according to a common protocol elaborated by the EBMT Developmental Committee, accredited by the Swedish National Board of Health and Welfare. Bone marrow mononuclear cells (146×10⁶) were seeded into 175 cm² flasks (Falcon, Franklin Lakes, New Jersey, USA) in Dulbecco's modified Eagles Medium-Low Glucose (DMEM-LG, Life Technologies, Gaithersburg, MD, USA) supplemented with lysed human platelets (final concentrations ranging from 10⁷ to 10⁹, normally 10⁸/mL). When the cultures were near confluence (>80%), the cells were detached by treatment with trypsin and EDTA (Invitrogen, Grand Island, NY, USA) and re-plated once at a density of 4,000 cells/cm². MSCs were harvested and cryopreserved in 10% Dimethyl Sulfoxide (WAK-Chemie Medical GmbH, Germany). After thawing, the cells were washed three times in PBS and re-suspended in 0.9 % saline solution with the addition of 10% AB plasma, to a final concentration of 2×10⁶ cells/ml. MSC release criteria for clinical use included: absence of visible clumps, spindle shape morphology, absence of contamination by pathogens (bacteria and mycoplasma) and viability >95%. MSCs expressed CD73, CD90, CD105, HLA-ABC and were negative for CD14, CD31, CD34, CD45 and HLA-DR (Figure 11).

### Assessment of immuno-modulatorv capacity

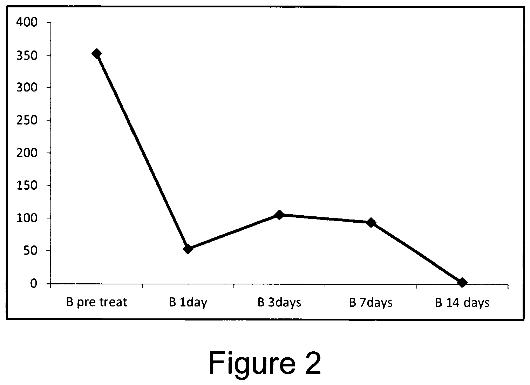

- *Ex-vivo* expanded MSCs were pre-treated (primed MSCs, pMSCs) or not (MSCs) with 10 ng/ml of IFN-γ and 15 ng/ml of TNF-α for 48 hours before used in co-cultures with PMNs with or without activation by endotoxin (100 ng/ml of lipopolysaccharides (LPS)). Following inflammatory priming, the MSCs up-regulated cell surface expression of CD54 (ICAM-1), CD106 (VCAM-1) and HLA-ABC and -DR (Fig. 2), as well as the expression of indoleamine 2,3-dioxygenase (IDO), a potent mediator of many MSC immune regulatory functions (Figures 12, 13 and 14).
- Fold increase of IDO expression after TNFα stimulation of donor cell MSCs should be <10 and fold increase of IDO expression after stimulation of INFγ should be more than >100 (p0,05). MSCs was cultured in the presence of TNF-α (15 ng/mL) or IFN-γ (10 ng/mL).
- Viability of PMN should be significantly improved (p0.05), preferably improved by at least 10%, or more preferably by at least 20%, after adding in either MSCs and LPS or pMSC with or without LPS as a co-culture. Viability and expression levels of surface markers by control or LPS-stimulated PMNs were investigated after 40 hours of direct co-culture with either resting or pMSCs. CD16 (FcγR-III) expression was used as surrogate marker of PMN viability. In the absence of LPS, PMN survival was enhanced only in presence of pMSCs. When LPS was added to the co-culture, both resting and pMSCs protected PMNs from apoptosis. Accordingly, the percentage of CD16-positive PMNs matched with the percentage of viable PMNs in all culture conditions. In parallel, expression of CD11b and CD54 is typically associated with PMN activation status. As expected, the percentage of CD11b-positive PMNs was higher in the presence of MSCs and further enhanced by LPS treatment, while CD11b relative mean fluorescence intensity (rMFI) did not change significantly, suggesting that more PMNs are becoming activated following MSC or pMSC exposure. Meaning that CD11b should be significantly improved (p 0,05) in pressens of MSCs, pMSCs with or without LPS. In contrast, CD54 rMFI should be significantly (p0,05) up-regulated by LPS treatment and this effect was enhanced by co-culture with MSCs. Overall, the higher PMN survival and activation triggered by MSCs suggests that MSCs may influence the PMN-dependent innate response through functional modifications rather than pro-apoptotic effects (Figure 14).

- Donor MSCs should preferably increase the number of myeloid-derived suppressor cells (MDSCs) by a factor 1.5, or more preferably a factor 2. Co-culturing PMNs with MSCs led to a marked increase in mature CD11b^{bright}/CD16^{bright}/CD62L^{dim} (N2-type) cells with hypersegmented nuclei consistent with granulocytic MDSCs (Figure 14). Similarly, co-culturing MSCs with healthy control human peripheral blood mononuclear cells (PBMCs) at different ratios promoted a significant increase (p0,05) CD14⁺HLA-DR^{low} monocytes resembling monocytic MDSCs (Figure 14).
- Donor MSCs should significantly increase (p 0,05), preferably 1.5-fold or even more preferably 2-fold, the number of regulatory T-cells as exemplified by CD4⁺CD25^{high} CD127^{low} regulatory T-cells (T_{Regs}), a key immune regulatory cell population, was also expanded in co-culture experiments with PBMCs (Figures 13 and 14). This corresponded with increased levels of circulating CD4⁺CD25^{high}CD127^{low} T_{Regs} that was observed in observed in treated patients' peripheral blood for up to 20 days following MSC administration (T_{Regs} among CD4⁺ T-cells in healthy controls (n=11) 3.84+/-1.60%, T_{Reg} range in patients 3.34-17.8%) (Figure 14). This finding could have resulted from an elevated thymic output as indicated by the increased proportion of CD31⁺ recent thymic emigrants among CD45RA⁺ naive T_{Regs} in patients and/or the MSC-stimulated conversion of conventional T-cells in the periphery (Figure 14).

### Male patient with virus-induced ARDS

Patient 1: A 58-year-old man with history of hypertension was hospitalized eight days after onset of high fever, cough, and dyspnea. Chest X-ray (CXR) demonstrated diffuse bilateral infiltrates and polymerase chain reaction (RT-PCR) of bronchoalveolar lavage (BAL) fluid was positive for influenza A H1N1. Oseltamivir (Tamiflu, Roche, Switzerland) was initiated but the patient deteriorated over the next two days with increased bilateral opacities on CXR and progressive respiratory failure requiring mechanical ventilation, followed by initial veno-venous (vv)- and later veno-arterial (va)-ECMO (CardioHelp, Maquet, Germany) for refractory hypoxemia. Acute kidney failure developed requiring continuous renal replacement therapy (CRRT).

Over the next four days, the patient's condition worsened with persistent hypoxemia, progressive bilateral infiltrates, and liver failure. At this juncture, following extensive discussions with the family, the hospital administration, and ethics board, HLA mismatched allogeneic bone marrow-derived MSCs obtained from a healthy volunteer were systemically infused through a central venous catheter positioned in the right atrium of the heart. The total dose (approximately 2 × 10⁶ cells/kg recipient weight) was divided into aliquots of 5 mL of cell suspension given at six time points over 20 minutes. To avoid infusion of MSCs into the ECMO circuit, the ECMO outflow cannula was clamped during each infusion, resulting in infusion inot the right atrium of the heart. At the time of infusion, the patient had no detectable influenza A H1N1 particles, as assessed by RT-PCR and had no other detectable active infection.

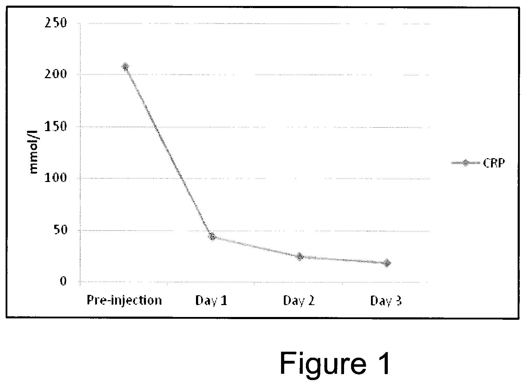

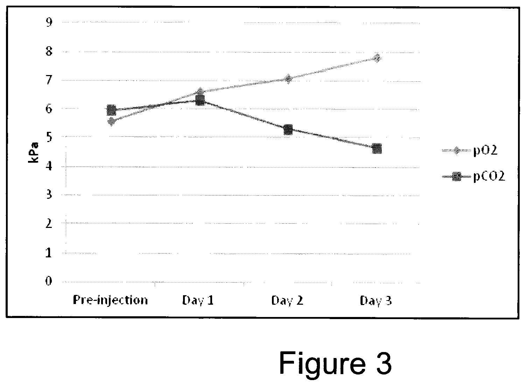

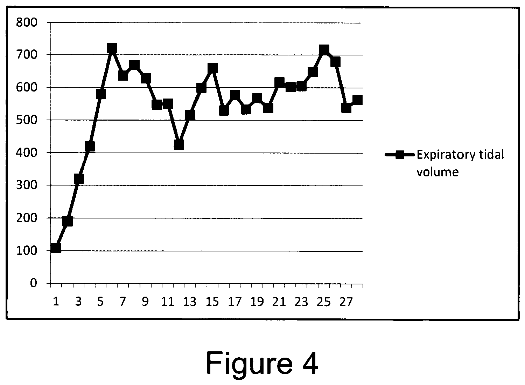

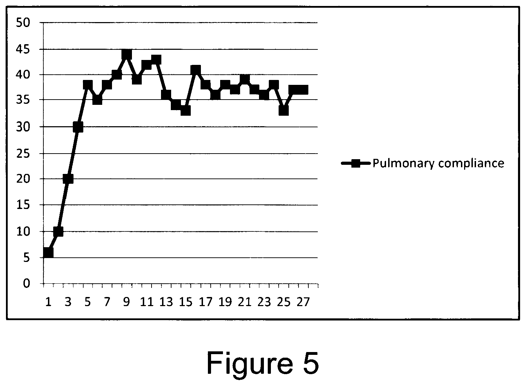

Within 24 hours clear signs of improvement are evident, both in terms of clinical chemistry parameters and pulmonary symptoms. CRP decreased strongly after administration of the MSCs (Figure 1), and so did various inflammatory cytokines, for instance the bronchoalveolar lavage (BAL) IL-6 (Figure 2), showing decreasing inflammatory response in the lung post-treatment. The partial oxygen pressure pO₂ increased significantly from pre-injection to day 3 post-injection, and the pCO₂ displayed a concomitant decrease (Figure 3). Additionally, tidal volume (Figure 4) and compliance (Figure 5) increased significantly in response to the MSC treatment.

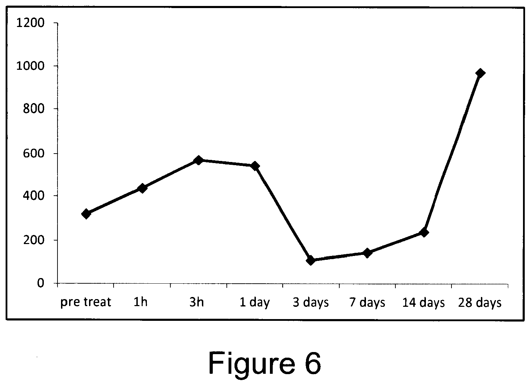

Finally, increased serum levels of RANTES approximately four weeks after treatment is indicative of stem cell mobilization in response to the MSC injection (Figure 6).

### Kidney failure in patient with virus-induced ARDS

A male patient presenting with virus-induced pneumonia develops subsequent ARDS, which in turn induces acute kidney failure requiring dialysis.

The patient is eligible for ECMO treatment but prior to commencing treatment a pharmaceutical composition comprising around 10⁶ MSCs per kg of body weight is administered to the patient (via peripheral venous injection), instead of placing the patient on ECMO support.

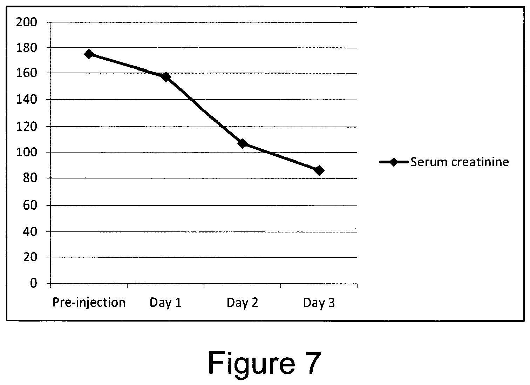

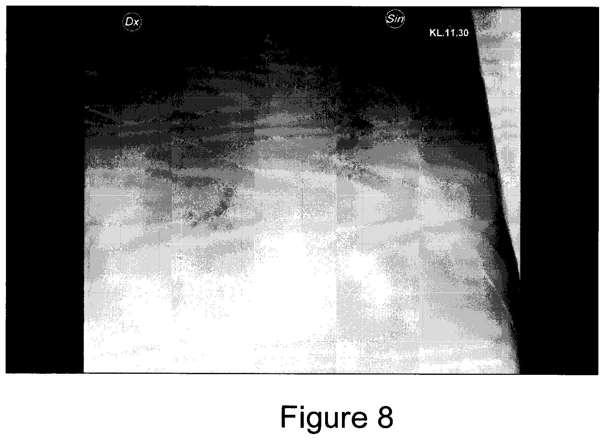

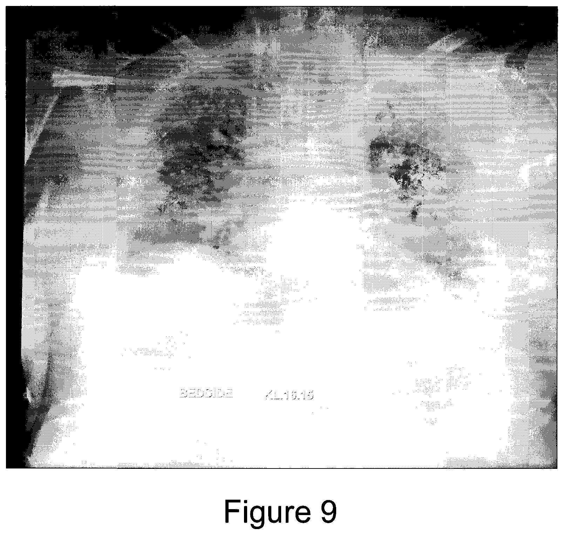

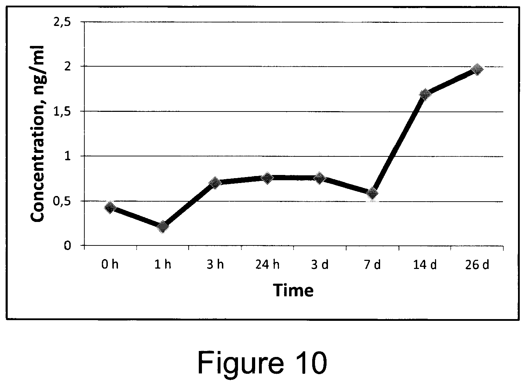

Post-injection of the MSCs the kidney failure resolved, with serum creatinine levels decreasing considerably (Figure 7) and the pulmonary symptoms were eliminated, as evidenced by lung X-ray (Figures 8,9, and 15) and recovering plasma surfactant B levels (Figure 10), with the patient subsequently being removed from ECMO treatment and dialysis.

### Treatment of infection-induced ARDS and transfusion-related acute lung injury (TRALI)

A 40-year-old man with no previous medical history was admitted to the hospital due to malaise, weight loss, night sweats, gingival hyperplasia and fever. Acute myeloid leukemia was diagnosed in the bone-marrow aspirate and induction chemotherapy was initiated. Due to caries and dental root abscesses, several teeth were extracted at the time of initiation of cytotoxic therapy. The following day, the patient became progressively hypoxemic and developed acute respiratory failure requiring intubation and mechanical ventilation. Blood and sputum cultures were negative, while CXR demonstrated bilateral infiltrates.

Despite treatment with broad spectrum antibiotics in the now neutropenic patient, progressive hypercapnia and hypoxia developed over the next two days and the patient was placed on vv-ECMO. Echocardiography demonstrated normal cardiac function. The patient was initially stable on ECMO but the clinical course was complicated by severe transfusion-dependent cytotoxic chemotherapy-induced thrombocytopenia.

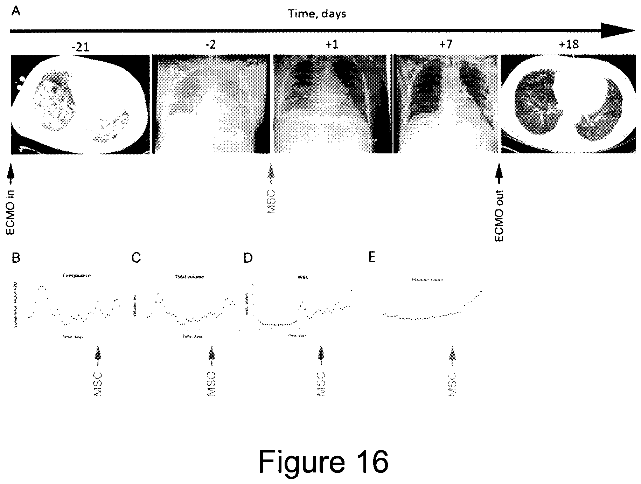

Given the large fluid volume from transfusions, CRRT was initiated in order to maintain appropriate fluid balance and reduce interstitial oedema. Fourteen days after initiation of ECMO the patient continued to deteriorate with increasing hypoxia and progressive bilateral infiltrates on CXR. Possible contributing factors included progressive ARDS, transfusion-related acute lung injury (TRALI), and/or undiagnosed infection although blood and sputum cultures remained negative. Following extensive discussion with the family, the hospital administration, and ethics board, HLA mismatched allogeneic bone marrow-derived MSCs (2 × 10⁶ cells/kg recipient weight) were similarly administered as in the first patient twenty-eight days after initiation of ECMO. As can be seen from Figure 16, there was progressive decrease in pulmonary infiltrates starting the first day after MSC infusion. By day 7, the CXR improved back to normal and the patient was weaned off ECMO the following day and extubation at day 12. Pulmonary compliance and tidal volumes improved back to normal levels during the first week. After MSC infusion, the bone-marrow function continued to be reconstituted with resolution of leukopenia and thrombocytopenia.
## Claims

1. A pharmaceutical composition comprising a population of immuno-modulatory MSCs having the following antigen profile: CD73+, CD90+, CD105+, CD34-, CD45-, CD14-, and CD3-, and a vesicle population comprising extracellular vesicles of said MSC population, wherein the extracellular vesicles are exosomes and wherein the vesicle population displays the following order of polypeptide abundance: serotransferrin < annexin A2, for use in the treatment of acute respiratory distress syndrome (ARDS), wherein the patient suffering from ARDS is eligible for and/or is undergoing extra-corporal membranous oxygenation (ECMO) treatment, and wherein the composition is administered via peripheral intravenous injection, central venous injection into the right atrium, injection into the right ventricle of the heart, and/or injection into the pulmonary trunk/artery.

2. The pharmaceutical composition for use according to claim 1, wherein the MSC population is positive for vimentin and/or Annexin A1.

3. The pharmaceutical composition for use according to claim 1 or 2, wherein the MSC population expresses a larger abundance of vimentin than of CD44.

4. The pharmaceutical composition for use according to claim any one of claims 1-3, wherein the MSC population fulfils at least one of the following immuno-modulation criteria:
(a) the MSC population is positive for at least one of the following polypeptides:
vimentin (SEQ ID No 1), caldesmon (SEQ ID No 2), annexin A1 (SEQ ID No 3), 14-3-3 protein epsilon (SEQ ID No 4), ADP ribosylation factor 1 (SEQ ID No 5), calnexin (SEQ ID No 6), ADP ribosylation factor 5 (SEQ ID No 7), transforming protein RhoA (SEQ ID No 8), CD44 (SEQ ID No 9), coactosin-like protein (SEQ ID No 10), mitogen-activated protein kinase 3 (SEQ ID No 11), insulin-like growth factor-binding protein 7 (SEQ ID No 12), N-acetyl-glucosamine-6-sulfatase (SEQ ID No 13), cellular retinoic acid-binding protein 2 (SEQ ID No 14), transcription elongation factor B polypeptide 1 (SEQ ID No 15), NEDD8 (SEQ ID No 16), fatty acid-binding protein, heart (SEQ ID No 17);
(b) the MSC population in (a) displays the following order of polypeptide abundance:
vimentin > annexin A1;
(c) the fold-increase expression of indoleamine 2,3-dioxygenase (IDO) in the MSC population is <10 when the MSCs are primed with 15 ng/ml TNF-alpha;
(d) the fold-increase expression of indoleamine 2,3-dioxygenase (IDO) in the MSC population is >100 when the MSCs are primed with 10 ng/ml IFN-gamma;
(e) the viability of polymorphonuclear neutrophils (PMNs) is increased by at least 20% when co-cultured with MSCs from the MSC population primed with IFN gamma or TNF-alpha in accordance with (c) or (d);
(f) the number of CD14⁺HLA-DR^{LOW} monocytes is increased at least 1.5-fold when healthy control human peripheral blood mononuclear cells (PBMCs) are co-cultured with the MSC population primed with IFN-gamma or TNF-alpha in accordance with (c) or (d);
(g) the number of CD4⁺CD25^{high}CD127^{LOW} regulatory T-cells (T_{Regs}) is increased at least 1.5-fold when healthy control human peripheral blood mononuclear cells (PBMCs) are co-cultured with the MSC population primed with IFN-gamma or TNF-alpha in accordance with (c) or (d).

5. The pharmaceutical composition for use according to any one of claims 1-4, further comprising plasma of blood type AB at a concentration of at least 1%, preferably 10%.

6. The pharmaceutical composition for use according to any one of claims 1-5 for use in the treatment of ARDS, wherein the patient to be treated has elevated levels of caspase-cleaved cytokeratin-18 (ccK18) as an indicator of epithelial apoptosis.

7. The pharmaceutical composition for use according to any one of claims 1-5 for use in the treatment of ARDS, wherein the patient to be treated has elevated levels of at least one of the following microRNAs (miRs): miR-409-3P, miR-886-5P, miR-324-3P, miR-222, miR-125A-5P, miR-339-3P, and/or miR-155.

## Patentansprüche

1. Pharmazeutische Zusammensetzung, die eine Population von immunmodulatorischen MSCs, die das folgende Antigenprofil aufweist: CD73+, CD90+, CD105+, CD34-, CD45-, CD14- und CD3-, und eine Vesikelpopulation umfasst, die extrazelluläre Vesikel der MSC-Population umfasst, wobei die extrazellulären Vesikel Exosome sind und wobei die Vesikelpopulation die folgende Reihenfolge von Polypeptidhäufigkeit anzeigt: Serotransferrin < Annexin A2, zur Verwendung bei der Behandlung von akutem Atemnotsyndrom (acute respiratory distress syndrome - ARDS), wobei der Patient, der an ARDS leidet, für eine extrakorporale Membranoxygenierungs(extra-corporal membranous oxygenation - ECMO)behandlung geeignet ist und/oder sich dieser unterzieht und wobei die Zusammensetzung über eine periphere intravenöse Injektion, eine zentralvenöse Injektion in den rechten Vorhof, eine Injektion in die rechte Herzkammer und/oder eine Injektion in den Lungenstamm/die Lungenarterie verabreicht wird.

2. Pharmazeutische Zusammensetzung zur Verwendung nach Anspruch 1, wobei die MSC-Population positiv für Vimentin und/oder Annexin A1 ist.

3. Pharmazeutische Zusammensetzung zur Verwendung nach Anspruch 1 oder 2, wobei die MSC-Population eine größere Häufigkeit an Vimentin exprimiert als an CD44.

4. Pharmazeutische Zusammensetzung zur Verwendung nach einem der Ansprüche 1-3, wobei die MSC-Population folgende Immunmodulationskriterien erfüllt:
(a) die MSC-Population ist positiv für die folgenden Polypeptide: Vimentin (SEQ ID Nr. 1), Caldesmon (SEQ ID Nr. 2), Annexin A1 (SEQ ID Nr. 3), 14-3-3 Protein Epsilon (SEQ ID Nr 4), ADP-Ribosylierungsfaktor 1 (SEQ ID Nr. 5), Calnexin (SEQ ID Nr. 6), ADP-Ribosylierungsfaktor 5 (SEQ ID Nr. 7), transformierendes Protein RhoA (SEQ ID Nr. 8), CD44 (SEQ ID Nr. 9), Coactosin-ähnliches Protein (SEQ ID Nr. 10), Mitogen-aktivierte Proteinkinase 3 (SEQ ID Nr. 11), insulinähnliches Wachstumsfaktor-bindendes Protein 7 (SEQ ID Nr. 12), N-Acetyl-Glucosamin-6-Sulfatase (SEQ ID Nr. 13), zelluläres Retinsäure-bindendes Protein 2 (SEQ ID Nr. 14), Transkriptionselongationsfaktor-B-Polypeptid 1 (SEQ ID Nr. 15), NEDD8 (SEQ ID Nr. 16), Fettsäure-bindendes Protein und/oder Herz (SEQ ID Nr. 17);
(b) die MSC-Population in (a) zeigt die folgende Reihenfolge von Polypeptidhäufigkeit an: Vimentin > Annexin A1;
(c) die Expression von Indolamin-2,3-Dioxygenase (IDO) in der MSC-Population ist um das <10-fache erhöht, wenn die MSCs mit 15 ng/ml TNF-alpha geprimt werden;
(d) die Expression von Indolamin-2,3-Dioxygenase (IDO) in der MSC-Population ist um das >100-fache erhöht, wenn die MSCs mit 10 ng/ml IFN-gamma geprimt werden;
(e) die Lebensfähigkeit von polymorphkernigen Neutrophilen (PMNs) wird um wenigstens 20 % erhöht, wenn sie mit MSCs aus der MSC-Population, die gemäß (c) oder (d) mit IFN-gamma oder TNF-alpha geprimt wurden, co-kultiviert werden;
(f) die Anzahl von CD14⁺HLA-DR^{NIEDRIG} Monozyten ist wenigstens um das 1,5-fache erhöht, wenn gesunde mononukleare Kontrollzellen des menschlichen peripheren Blutes (peripheral blood mononuclear cells - PBMCs) mit der MSC-Population, die mit IFN-gamma oder TNF-alpha gemäß (c) oder (d) geprimt wurden, co-kultiviert werden; und/oder
(g) die Anzahl von regulatorischen CD4⁺CD25^{hoch}CD127^{NIEDRIG}-T-Zellen (T_{Regs}) ist wenigstens um das 1,5-fache erhöht, wenn gesunde mononukleare Kontrollzellen des menschlichen peripheren Blutes (PBMCs) mit der MSC-Population, die mit IFN-gamma oder TNF-alpha gemäß (c) oder (d) geprimt wurden, co-kultiviert werden.

5. Pharmazeutische Zusammensetzung zur Verwendung nach einem der Ansprüche 1-4, die ferner Plasma der Blutgruppe AB in einer Konzentration von wenigstens 1 %, vorzugsweise 10 %, umfasst.

6. Pharmazeutische Zusammensetzung zur Verwendung nach einem der Ansprüche 1-5 zur Verwendung bei der Behandlung von ARDS, wobei der zu behandelnde Patient erhöhte Spiegel von Caspase-gespaltenem Cytokeratin-18 (ccK18) als einen Indikator für epitheliale Apoptose aufweist.

7. Pharmazeutische Zusammensetzung zur Verwendung nach einem der Ansprüche 1-5 zur Verwendung bei der Behandlung von ARDS, wobei der zu behandelnde Patient erhöhte Spiegel von folgenden microRNAs (miRs) aufweist: miR-409-3P, miR -886-5P, miR-324-3P, miR-222, miR-125A-5P, miR-339-3P und/oder miR-155.

## Revendications

1. Composition pharmaceutique comprenant une population de CSM immunomodulatrices ayant le profil antigénique suivant : CD73+, CD90+, CD105+, CD34-, CD45-, CD14- et CD3-, et une population de vésicules comprenant des vésicules extracellulaires de ladite population de CSM, dans laquelle les vésicules extracellulaires sont des exosomes et la population de vésicules présentant l'ordre d'abondance de polypeptide suivant : sérotransferrine < annexine A2, pour une utilisation dans le traitement du syndrome de détresse respiratoire aiguë (SDRA), le patient souffrant du SDRA étant éligible et/ou étant soumis au traitement d'oxygénation par membrane extracorporelle (ECMO), et la composition étant administrée par injection intraveineuse périphérique, par injection veineuse centrale dans l'atrium droit, par injection dans le ventricule droit du cœur et/ou par injection dans le tronc/l'artère pulmonaire.

2. Composition pharmaceutique destinée à être utilisée selon la revendication 1, dans laquelle la population de CSM est positive pour la vimentine et/ou l'annexine A1.

3. Composition pharmaceutique destinée à être utilisée selon la revendication 1 ou 2, dans laquelle la population de CSM exprime une plus grande abondance de vimentine que de CD44.

4. Composition pharmaceutique destinée à être utilisée selon l'une quelconque des revendications 1 à 3, dans laquelle la population de CSM remplit les critères d'immunomodulation suivants :
(a) la population de CSM est positive pour les polypeptides suivants : vimentine (SEQ ID n° 1), et/ou caldesmon (SEQ ID n° 2), et/ou annexine A1 (SEQ ID n° 3), et/ou 14-3-3 protéine epsilon (SEQ ID n° 4), et/ou facteur 1 de ribosylation ADP (SEQ ID n° 5), et/ou calnexine (SEQ ID n° 6), et/ou facteur 5 de ribosylation ADP (SEQ ID n° 7), et/ou protéine transformante RhoA (SEQ ID n° 8), et/ou CD44 (SEQ ID n° 9), et/ou protéine de type coactosine (SEQ ID n° 10), et/ou protéine kinase 3 activée par mitogène (SEQ ID n° 11), et/ou protéine 7 de liaison au facteur de croissance de type insuline (SEQ ID n° 12), et/ou N-acétyl-glucosamine-6-sulfatase (SEQ ID n° 13), et/ou protéine 2 cellulaire de liaison à l'acide rétinoïque (SEQ ID n° 14), et/ou polypeptide 1 du facteur B d'élongation de la transcription (SEQ ID n° 15), et/ou NEDD8 (SEQ ID n° 16), et/ou protéine de liaison aux acides gras, et/ou cœur (SEQ ID n° 17) ;
(b) la population de CSM en (a) présente l'ordre d'abondance de polypeptides suivant : vimentine > annexine A1 ;
(c) le facteur d'augmentation de l'expression de l'indoleamine 2,3-dioxygénase (IDO) dans la population de CSM est < 10 lorsque les CSM sont amorcées avec 15 ng/ml de TNF-alpha ;
(d) le facteur d'augmentation de l'expression de l'indoleamine 2,3-dioxygénase (IDO) dans la population de CSM est > 100 lorsque les CSM sont amorcées avec 10 ng/ml d'IFN-gamma ;
(e) la viabilité des polynucléaires neutrophiles (PMN) est augmentée d'au moins 20 % lorsqu'ils sont co-cultivés avec des CSM de la population de CSM amorcées avec de l'IFN gamma ou du TNF-alpha conformément à (c) ou à (d) ;
(f) le nombre de monocytes CD14⁺HLA-DR^{FAIBLE} est augmenté d'au moins 1,5 fois lorsque des cellules mononucléées du sang périphérique humain (PBMC) de contrôle sain sont co-cultivées avec la population de CSM amorcées avec de l'IFN-gamma ou du TNF-alpha conformément à (c) ou (d) ;
(g) le nombre de cellules T régulatrices CD4⁺CD25^{élevés}CD127^{FAIBLE} (T_{Regs}) est augmenté d'au moins 1,5 fois lorsque des cellules mononucléées du sang périphérique humain (PBMC) de contrôle sain sont co-cultivées avec la population de CSM amorcées avec de l'IFN-gamma ou du TNF-alpha conformément à (c) ou (d).

5. Composition pharmaceutique destinée à être utilisée selon l'une quelconque des revendications 1 à 4, comprenant en outre du plasma du groupe sanguin AB à une concentration d'au moins 1 %, de préférence 10 %.

6. Composition pharmaceutique destinée à être utilisée selon l'une quelconque des revendications 1 à 5 pour une utilisation dans le traitement du SDRA, dans laquelle le patient à traiter a des taux élevés de cytokératine-18 clivée par la caspase (ccK18) comme un indicateur d'apoptose épithéliale.

7. Composition pharmaceutique destinée à être utilisée selon l'une quelconque des revendications 1 à 5 pour une utilisation dans le traitement du SDRA, dans laquelle le patient à traiter a des taux élevés de micro-ARN (miR) suivants : miR-409-3P, et/ou miR-886-5P, et/ou miR-324-3P, et/ou miR-222, et/ou miR-125A-5P, et/ou miR-339-3P, et/ou miR-155.


<110> IsletOne AB
<120> MSCs in the treatment of inflammatory pulmonary diseases
<130> IO-2
<150> SE1300521-0
   <151> 2013-08-01
<160> 44
<170> PatentIn version 3.5
<210> 1
   <211> 466
   <212> PRT
   <213> Homo sapiens
<400> 1 Leu Glu
465
<210> 2
   <211> 793
   <212> PRT
   <213> Homo sapiens
<400> 2
   Met Asp Asp Phe Glu Arg Arg Arg Glu Leu Arg Arg Gln Lys Arg Glu
   1               5                   10                  15
   Glu Met Arg Leu Glu Ala Glu Arg Ile Ala Tyr Gln Arg Asn Asp Asp
               20                  25                  30
   Asp Glu Glu Glu Ala Ala Arg Glu Arg Arg Arg Arg Ala Arg Gln Glu
           35                  40                  45
   Arg Leu Arg Gln Lys Gln Glu Glu Glu Ser Leu Gly Gln Val Thr Asp
       50                  55                  60
   Gln Val Glu Val Asn Ala Gln Asn Ser Val Pro Asp Glu Glu Ala Lys
   65                  70                  75                  80
   Thr Thr Thr Thr Asn Thr Gln Val Glu Gly Asp Asp Glu Ala Ala Phe
                   85                  90                  95
   Leu Glu Arg Leu Ala Arg Arg Glu Glu Arg Arg Gln Lys Arg Leu Gln
               100                 105                 110
   Glu Ala Leu Glu Arg Gln Lys Glu Phe Asp Pro Thr Ile Thr Asp Ala
           115                 120                 125
   Ser Leu Ser Leu Pro Ser Arg Arg Met Gln Asn Asp Thr Ala Glu Asn
       130                 135                 140
   Glu Thr Thr Glu Lys Glu Glu Lys Ser Glu Ser Arg Gln Glu Arg Tyr
   145                 150                 155                 160
   Glu Ile Glu Glu Thr Glu Thr Val Thr Lys Ser Tyr Gln Lys Asn Asp
                   165                 170                 175
   Trp Arg Asp Ala Glu Glu Asn Lys Lys Glu Asp Lys Glu Lys Glu Glu
               180                 185                 190
   Glu Glu Glu Glu Lys Pro Lys Arg Gly Ser Ile Gly Glu Asn Gln Val
           195                 200                 205
   Glu Val Met Val Glu Glu Lys Thr Thr Glu Ser Gln Glu Glu Thr Val
       210                 215                 220
   Val Met Ser Leu Lys Asn Gly Gln Ile Ser Ser Glu Glu Pro Lys Gln
   225 230 235 240
   Glu Glu Glu Arg Glu Gln Gly Ser Asp Glu Ile Ser His His Glu Lys
   245 250 255
   Met Glu Glu Glu Asp Lys Glu Arg Ala Glu Ala Glu Arg Ala Arg Leu
   260 265 270
   Glu Ala Glu Glu Arg Glu Arg Ile Lys Ala Glu Gln Asp Lys Lys Ile
   275 280 285
   Ala Asp Glu Arg Ala Arg Ile Glu Ala Glu Glu Lys Ala Ala Ala Gln
   290 295 300
   Glu Arg Glu Arg Arg Glu Ala Glu Glu Arg Glu Arg Met Arg Glu Glu
   305 310 315 320
   Glu Lys Arg Ala Ala Glu Glu Arg Gln Arg Ile Lys Glu Glu Glu Lys
   325 330 335
   Arg Ala Ala Glu Glu Arg Gln Arg Ile Lys Glu Glu Glu Lys Arg Ala
   340 345 350
   Ala Glu Glu Arg Gln Arg Ile Lys Glu Glu Glu Lys Arg Ala Ala Glu
   355 360 365
   Glu Arg Gln Arg Ala Arg Ala Glu Glu Glu Glu Lys Ala Lys Val Glu
   370 375 380
   Glu Gln Lys Arg Asn Lys Gln Leu Glu Glu Lys Lys His Ala Met Gln
   385 390 395 400
   Glu Thr Lys Ile Lys Gly Glu Lys Val Glu Gln Lys Ile Glu Gly Lys
   405 410 415
   Trp Val Asn Glu Lys Lys Ala Gln Glu Asp Lys Leu Gln Thr Ala Val
   420 425 430
   Leu Lys Lys Gln Gly Glu Glu Lys Gly Thr Lys Val Gln Ala Lys Arg
   435 440 445
   Glu Lys Leu Gln Glu Asp Lys Pro Thr Phe Lys Lys Glu Glu Ile Lys
   450 455 460
   Asp Glu Lys Ile Lys Lys Asp Lys Glu Pro Lys Glu Glu Val Lys Ser
   465 470 475 480
   Phe Met Asp Arg Lys Lys Gly Phe Thr Glu Val Lys Ser Gln Asn Gly
   485 490 495
   Glu Phe Met Thr His Lys Leu Lys His Thr Glu Asn Thr Phe Ser Arg
               500                 505                 510
   Pro Gly Gly Arg Ala Ser Val Asp Thr Lys Glu Ala Glu Gly Ala Pro
           515                 520                 525
   Gln Val Glu Ala Gly Lys Arg Leu Glu Glu Leu Arg Arg Arg Arg Gly
       530                 535                 540
   Glu Thr Glu Ser Glu Glu Phe Glu Lys Leu Lys Gln Lys Gln Gln Glu
   545                 550                 555                 560
   Ala Ala Leu Glu Leu Glu Glu Leu Lys Lys Lys Arg Glu Glu Arg Arg
                   565                 570                 575
   Lys Val Leu Glu Glu Glu Glu Gln Arg Arg Lys Gln Glu Glu Ala Asp
               580                 585                 590
   Arg Lys Leu Arg Glu Glu Glu Glu Lys Arg Arg Leu Lys Glu Glu Ile
           595                 600                 605
   Glu Arg Arg Arg Ala Glu Ala Ala Glu Lys Arg Gln Lys Met Pro Glu
       610                 615                 620
   Asp Gly Leu Ser Asp Asp Lys Lys Pro Phe Lys Cys Phe Thr Pro Lys
   625                 630                 635                 640
   Gly Ser Ser Leu Lys Ile Glu Glu Arg Ala Glu Phe Leu Asn Lys Ser
                   645                 650                 655
   Val Gln Lys Ser Ser Gly Val Lys Ser Thr His Gln Ala Ala Ile Val
               660                 665                 670
   Ser Lys Ile Asp Ser Arg Leu Glu Gln Tyr Thr Ser Ala Ile Glu Gly
           675                 680                 685
   Thr Lys Ser Ala Lys Pro Thr Lys Pro Ala Ala Ser Asp Leu Pro Val
       690                 695                 700
   Pro Ala Glu Gly Val Arg Asn Ile Lys Ser Met Trp Glu Lys Gly Asn
   705                 710                 715                 720
   Val Phe Ser Ser Pro Thr Ala Ala Gly Thr Pro Asn Lys Glu Thr Ala
                   725                 730                 735
   Gly Leu Lys Val Gly Val Ser Ser Arg Ile Asn Glu Trp Leu Thr Lys
               740                 745                 750
   Thr Pro Asp Gly Asn Lys Ser Pro Ala Pro Lys Pro Ser Asp Leu Arg
           755                 760                 765
   Pro Gly Asp Val Ser Ser Lys Arg Asn Leu Trp Glu Lys Gln Ser Val
   770 775 780
   Asp Lys Val Thr Ser Pro Thr Lys Val
   785 790
<210> 3
   <211> 346
   <212> PRT
   <213> Homo sapiens
<400> 3
   Met Ala Met Val Ser Glu Phe Leu Lys Gln Ala Trp Phe Ile Glu Asn
   1 5 10 15
   Glu Glu Gln Glu Tyr Val Gln Thr Val Lys Ser Ser Lys Gly Gly Pro
   20 25 30
   Gly Ser Ala Val Ser Pro Tyr Pro Thr Phe Asn Pro Ser Ser Asp Val
   35 40 45
   Ala Ala Leu His Lys Ala Ile Met Val Lys Gly Val Asp Glu Ala Thr
   50 55 60
   Ile Ile Asp Ile Leu Thr Lys Arg Asn Asn Ala Gln Arg Gln Gln Ile
   65 70 75 80
   Lys Ala Ala Tyr Leu Gln Glu Thr Gly Lys Pro Leu Asp Glu Thr Leu
   85 90 95
   Lys Lys Ala Leu Thr Gly His Leu Glu Glu Val Val Leu Ala Leu Leu
   100 105 110
   Lys Thr Pro Ala Gln Phe Asp Ala Asp Glu Leu Arg Ala Ala Met Lys
   115 120 125
   Gly Leu Gly Thr Asp Glu Asp Thr Leu Ile Glu Ile Leu Ala Ser Arg
   130 135 140
   Thr Asn Lys Glu Ile Arg Asp Ile Asn Arg Val Tyr Arg Glu Glu Leu
   145 150 155 160
   Lys Arg Asp Leu Ala Lys Asp Ile Thr Ser Asp Thr Ser Gly Asp Phe
   165 170 175
   Arg Asn Ala Leu Leu Ser Leu Ala Lys Gly Asp Arg Ser Glu Asp Phe
   180 185 190
   Gly Val Asn Glu Asp Leu Ala Asp Ser Asp Ala Arg Ala Leu Tyr Glu
   195 200 205
   Ala Gly Glu Arg Arg Lys Gly Thr Asp Val Asn Val Phe Asn Thr Ile
       210                 215                 220
   Leu Thr Thr Arg Ser Tyr Pro Gln Leu Arg Arg Val Phe Gln Lys Tyr
   225                 230                 235                 240
   Thr Lys Tyr Ser Lys His Asp Met Asn Lys Val Leu Asp Leu Glu Leu
                   245                 250                 255
   Lys Gly Asp Ile Glu Lys Cys Leu Thr Ala Ile Val Lys Cys Ala Thr
               260                 265                 270
   Ser Lys Pro Ala Phe Phe Ala Glu Lys Leu His Gln Ala Met Lys Gly
           275                 280                 285
   Val Gly Thr Arg His Lys Ala Leu Ile Arg Ile Met Val Ser Arg Ser
       290                 295                 300
   Glu Ile Asp Met Asn Asp Ile Lys Ala Phe Tyr Gln Lys Met Tyr Gly
   305                 310                 315                 320
   Ile Ser Leu Cys Gln Ala Ile Leu Asp Glu Thr Lys Gly Asp Tyr Glu
                   325                 330                 335
   Lys Ile Leu Val Ala Leu Cys Gly Gly Asn
               340                 345
<210> 4
   <211> 255
   <212> PRT
   <213> Homo sapiens
<400> 4
   Met Asp Asp Arg Glu Asp Leu Val Tyr Gln Ala Lys Leu Ala Glu Gln
   1               5                   10                  15
   Ala Glu Arg Tyr Asp Glu Met Val Glu Ser Met Lys Lys Val Ala Gly
               20                  25                  30
   Met Asp Val Glu Leu Thr Val Glu Glu Arg Asn Leu Leu Ser Val Ala
           35                  40                  45
   Tyr Lys Asn Val Ile Gly Ala Arg Arg Ala Ser Trp Arg Ile Ile Ser
       50                  55                  60
   Ser Ile Glu Gln Lys Glu Glu Asn Lys Gly Gly Glu Asp Lys Leu Lys
   65                  70                  75                  80
   Met Ile Arg Glu Tyr Arg Gln Met Val Glu Thr Glu Leu Lys Leu Ile
                   85                  90                  95
   Cys Cys Asp Ile Leu Asp Val Leu Asp Lys His Leu Ile Pro Ala Ala
               100                 105                 110
   Asn Thr Gly Glu Ser Lys Val Phe Tyr Tyr Lys Met Lys Gly Asp Tyr
           115                 120                 125
   His Arg Tyr Leu Ala Glu Phe Ala Thr Gly Asn Asp Arg Lys Glu Ala
       130                 135                 140
   Ala Glu Asn Ser Leu Val Ala Tyr Lys Ala Ala Ser Asp Ile Ala Met
   145                 150                 155                 160
   Thr Glu Leu Pro Pro Thr His Pro Ile Arg Leu Gly Leu Ala Leu Asn
                   165                 170                 175
   Phe Ser Val Phe Tyr Tyr Glu Ile Leu Asn Ser Pro Asp Arg Ala Cys
               180                 185                 190
   Arg Leu Ala Lys Ala Ala Phe Asp Asp Ala Ile Ala Glu Leu Asp Thr
           195                 200                 205
   Leu Ser Glu Glu Ser Tyr Lys Asp Ser Thr Leu Ile Met Gln Leu Leu
       210                 215                 220
   Arg Asp Asn Leu Thr Leu Trp Thr Ser Asp Met Gln Gly Asp Gly Glu
   225                 230                 235                 240
   Glu Gln Asn Lys Glu Ala Leu Gln Asp Val Glu Asp Glu Asn Gln
                   245                 250                 255
<210> 5
   <211> 181
   <212> PRT
   <213> Homo sapiens
<400> 5
   Met Gly Asn Ile Phe Ala Asn Leu Phe Lys Gly Leu Phe Gly Lys Lys
   1               5                   10                  15
   Glu Met Arg Ile Leu Met Val Gly Leu Asp Ala Ala Gly Lys Thr Thr
               20                  25                  30
   Ile Leu Tyr Lys Leu Lys Leu Gly Glu Ile Val Thr Thr Ile Pro Thr
           35                  40                  45
   Ile Gly Phe Asn Val Glu Thr Val Glu Tyr Lys Asn Ile Ser Phe Thr
       50                  55                  60
   Val Trp Asp Val Gly Gly Gln Asp Lys Ile Arg Pro Leu Trp Arg His
   65                  70                  75                  80
   Tyr Phe Gln Asn Thr Gln Gly Leu Ile Phe Val Val Asp Ser Asn Asp
                   85                  90                  95
   Arg Glu Arg Val Asn Glu Ala Arg Glu Glu Leu Met Arg Met Leu Ala
               100                 105                 110
   Glu Asp Glu Leu Arg Asp Ala Val Leu Leu Val Phe Ala Asn Lys Gln
           115                 120                 125
   Asp Leu Pro Asn Ala Met Asn Ala Ala Glu Ile Thr Asp Lys Leu Gly
       130                 135                 140
   Leu His Ser Leu Arg His Arg Asn Trp Tyr Ile Gln Ala Thr Cys Ala
   145                 150                 155                 160
   Thr Ser Gly Asp Gly Leu Tyr Glu Gly Leu Asp Trp Leu Ser Asn Gln
                   165                 170                 175
   Leu Arg Asn Gln Lys
               180
<210> 6
   <211> 592
   <212> PRT
   <213> Homo sapiens
<400> 6
   Met Glu Gly Lys Trp Leu Leu Cys Met Leu Leu Val Leu Gly Thr Ala
   1               5                   10                  15
   Ile Val Glu Ala His Asp Gly His Asp Asp Asp Val Ile Asp Ile Glu
               20                  25                  30
   Asp Asp Leu Asp Asp Val Ile Glu Glu Val Glu Asp Ser Lys Pro Asp
           35                  40                  45
   Thr Thr Ala Pro Pro Ser Ser Pro Lys Val Thr Tyr Lys Ala Pro Val
       50                  55                  60
   Pro Thr Gly Glu Val Tyr Phe Ala Asp Ser Phe Asp Arg Gly Thr Leu
   65                  70                  75                  80
   Ser Gly Trp Ile Leu Ser Lys Ala Lys Lys Asp Asp Thr Asp Asp Glu
                   85                  90                  95
   Ile Ala Lys Tyr Asp Gly Lys Trp Glu Val Glu Glu Met Lys Glu Ser
               100                 105                 110
   Lys Leu Pro Gly Asp Lys Gly Leu Val Leu Met Ser Arg Ala Lys His
           115                 120                 125
   His Ala Ile Ser Ala Lys Leu Asn Lys Pro Phe Leu Phe Asp Thr Lys
       130                 135                 140
   Pro Leu Ile Val Gln Tyr Glu Val Asn Phe Gln Asn Gly Ile Glu Cys
   145                 150                 155                 160
   Gly Gly Ala Tyr Val Lys Leu Leu Ser Lys Thr Pro Glu Leu Asn Leu
                   165                 170                 175
   Asp Gln Phe His Asp Lys Thr Pro Tyr Thr Ile Met Phe Gly Pro Asp
               180                 185                 190
   Lys Cys Gly Glu Asp Tyr Lys Leu His Phe Ile Phe Arg His Lys Asn
           195                 200                 205
   Pro Lys Thr Gly Ile Tyr Glu Glu Lys His Ala Lys Arg Pro Asp Ala
       210                 215                 220
   Asp Leu Lys Thr Tyr Phe Thr Asp Lys Lys Thr His Leu Tyr Thr Leu
   225                 230                 235                 240
   Ile Leu Asn Pro Asp Asn Ser Phe Glu Ile Leu Val Asp Gln Ser Val
                   245                 250                 255
   Val Asn Ser Gly Asn Leu Leu Asn Asp Met Thr Pro Pro Val Asn Pro
               260                 265                 270
   Ser Arg Glu Ile Glu Asp Pro Glu Asp Arg Lys Pro Glu Asp Trp Asp
           275                 280                 285
   Glu Arg Pro Lys Ile Pro Asp Pro Glu Ala Val Lys Pro Asp Asp Trp
       290                 295                 300
   Asp Glu Asp Ala Pro Ala Lys Ile Pro Asp Glu Glu Ala Thr Lys Pro
   305                 310                 315                 320
   Glu Gly Trp Leu Asp Asp Glu Pro Glu Tyr Val Pro Asp Pro Asp Ala
                   325                 330                 335
   Glu Lys Pro Glu Asp Trp Asp Glu Asp Met Asp Gly Glu Trp Glu Ala
               340                 345                 350
   Pro Gln Ile Ala Asn Pro Arg Cys Glu Ser Ala Pro Gly Cys Gly Val
           355                 360                 365
   Trp Gln Arg Pro Val Ile Asp Asn Pro Asn Tyr Lys Gly Lys Trp Lys
       370                 375                 380
   Pro Pro Met Ile Asp Asn Pro Ser Tyr Gln Gly Ile Trp Lys Pro Arg
   385                 390                 395                 400
   Lys Ile Pro Asn Pro Asp Phe Phe Glu Asp Leu Glu Pro Phe Arg Met
                   405                 410                 415
   Thr Pro Phe Ser Ala Ile Gly Leu Glu Leu Trp Ser Met Thr Ser Asp
   420 425 430
   Ile Phe Phe Asp Asn Phe Ile Ile Cys Ala Asp Arg Arg Ile Val Asp
   435 440 445
   Asp Trp Ala Asn Asp Gly Trp Gly Leu Lys Lys Ala Ala Asp Gly Ala
   450 455 460
   Ala Glu Pro Gly Val Val Gly Gln Met Ile Glu Ala Ala Glu Glu Arg
   465 470 475 480
   Pro Trp Leu Trp Val Val Tyr Ile Leu Thr Val Ala Leu Pro Val Phe
   485 490 495
   Leu Val Ile Leu Phe Cys Cys Ser Gly Lys Lys Gln Thr Ser Gly Met
   500 505 510
   Glu Tyr Lys Lys Thr Asp Ala Pro Gln Pro Asp Val Lys Glu Glu Glu
   515 520 525
   Glu Glu Lys Glu Glu Glu Lys Asp Lys Gly Asp Glu Glu Glu Glu Gly
   530 535 540
   Glu Glu Lys Leu Glu Glu Lys Gln Lys Ser Asp Ala Glu Glu Asp Gly
   545 550 555 560
   Gly Thr Val Ser Gln Glu Glu Glu Asp Arg Lys Pro Lys Ala Glu Glu
   565 570 575
   Asp Glu Ile Leu Asn Arg Ser Pro Arg Asn Arg Lys Pro Arg Arg Glu
   580 585 590
<210> 7
   <211> 180
   <212> PRT
   <213> Homo sapiens
<400> 7
   Met Gly Leu Thr Val Ser Ala Leu Phe Ser Arg Ile Phe Gly Lys Lys
   1 5 10 15
   Gln Met Arg Ile Leu Met Val Gly Leu Asp Ala Ala Gly Lys Thr Thr
   20 25 30
   Ile Leu Tyr Lys Leu Lys Leu Gly Glu Ile Val Thr Thr Ile Pro Thr
   35 40 45
   Ile Gly Phe Asn Val Glu Thr Val Glu Tyr Lys Asn Ile Cys Phe Thr
   50 55 60
   Val Trp Asp Val Gly Gly Gln Asp Lys Ile Arg Pro Leu Trp Arg His
   65                  70                  75                  80
   Tyr Phe Gln Asn Thr Gln Gly Leu Ile Phe Val Val Asp Ser Asn Asp
                   85                  90                  95
   Arg Glu Arg Val Gln Glu Ser Ala Asp Glu Leu Gln Lys Met Leu Gln
               100                 105                 110
   Glu Asp Glu Leu Arg Asp Ala Val Leu Leu Val Phe Ala Asn Lys Gln
           115                 120                 125
   Asp Met Pro Asn Ala Met Pro Val Ser Glu Leu Thr Asp Lys Leu Gly
       130                 135                 140
   Leu Gln His Leu Arg Ser Arg Thr Trp Tyr Val Gln Ala Thr Cys Ala
   145                 150                 155                 160
   Thr Gln Gly Thr Gly Leu Tyr Asp Gly Leu Asp Trp Leu Ser His Glu
                   165                 170                 175
   Leu Ser Lys Arg
               180
<210> 8
   <211> 193
   <212> PRT
   <213> Homo sapiens
<400> 8
   Met Ala Ala Ile Arg Lys Lys Leu Val Ile Val Gly Asp Gly Ala Cys
   1               5                   10                  15
   Gly Lys Thr Cys Leu Leu Ile Val Phe Ser Lys Asp Gln Phe Pro Glu
               20                  25                  30
   Val Tyr Val Pro Thr Val Phe Glu Asn Tyr Val Ala Asp Ile Glu Val
           35                  40                  45
   Asp Gly Lys Gln Val Glu Leu Ala Leu Trp Asp Thr Ala Gly Gln Glu
       50                  55                  60
   Asp Tyr Asp Arg Leu Arg Pro Leu Ser Tyr Pro Asp Thr Asp Val Ile
   65                  70                  75                  80
   Leu Met Cys Phe Ser Ile Asp Ser Pro Asp Ser Leu Glu Asn Ile Pro
                   85                  90                  95
   Glu Lys Trp Thr Pro Glu Val Lys His Phe Cys Pro Asn Val Pro Ile
               100                 105                 110
   Ile Leu Val Gly Asn Lys Lys Asp Leu Arg Asn Asp Glu His Thr Arg
           115                 120                 125
   Arg Glu Leu Ala Lys Met Lys Gln Glu Pro Val Lys Pro Glu Glu Gly
       130                 135                 140
   Arg Asp Met Ala Asn Arg Ile Gly Ala Phe Gly Tyr Met Glu Cys Ser
   145                 150                 155                 160
   Ala Lys Thr Lys Asp Gly Val Arg Glu Val Phe Glu Met Ala Thr Arg
                   165                 170                 175
   Ala Ala Leu Gln Ala Arg Arg Gly Lys Lys Lys Ser Gly Cys Leu Val
               180                 185                 190
   Leu
<210> 9
   <211> 206
   <212> PRT
   <213> Homo sapiens
<400> 9
   Met Asp Lys Phe Trp Trp His Ala Ala Trp Gly Leu Cys Leu Val Pro
   1 5 10 15
   Leu Ser Leu Ala Gln Ile Asp Leu Asn Ile Thr Cys Arg Phe Ala Gly
   20 25 30
   Val Phe His Val Glu Lys Asn Gly Arg Tyr Ser Ile Ser Arg Thr Glu
   35 40 45
   Ala Ala Asp Leu Cys Lys Ala Phe Asn Ser Thr Leu Pro Thr Met Ala
   50 55 60
   Gln Met Glu Lys Ala Leu Ser Ile Gly Phe Glu Thr Cys Arg Tyr Gly
   65 70 75 80
   Phe Ile Glu Gly His Val Val Ile Pro Arg Ile His Pro Asn Ser Ile
   85 90 95
   Cys Gly Ala Asn Thr Thr Ser Gly Pro Ile Arg Thr Pro Gln Ile Pro
   100 105 110
   Glu Trp Leu Ile Ile Leu Ala Ser Leu Leu Ala Leu Ala Leu Ile Leu
   115 120 125
   Ala Val Cys Ile Ala Val Asn Ser Arg Arg Arg Cys Gly Gln Lys Lys
   130 135 140
   Lys Leu Val Ile Asn Ser Gly Asn Gly Ala Val Glu Asp Arg Lys Pro
   145 150 155 160
   Ser Gly Leu Asn Gly Glu Ala Ser Lys Ser Gln Glu Met Val His Leu
   165 170 175
   Val Asn Lys Glu Ser Ser Glu Thr Pro Asp Gln Phe Met Thr Ala Asp
   180 185 190
   Glu Thr Arg Asn Leu Gln Asn Val Asp Met Lys Ile Gly Val
   195 200 205
<210> 10
   <211> 142
   <212> PRT
   <213> Homo sapiens
<400> 10
   Met Ala Thr Lys Ile Asp Lys Glu Ala Cys Arg Ala Ala Tyr Asn Leu
   1               5                   10                  15
   Val Arg Asp Asp Gly Ser Ala Val Ile Trp Val Thr Phe Lys Tyr Asp
               20                  25                  30
   Gly Ser Thr Ile Val Pro Gly Glu Gln Gly Ala Glu Tyr Gln His Phe
           35                  40                  45
   Ile Gln Gln Cys Thr Asp Asp Val Arg Leu Phe Ala Phe Val Arg Phe
       50                  55                  60
   Thr Thr Gly Asp Ala Met Ser Lys Arg Ser Lys Phe Ala Leu Ile Thr
   65                  70                  75                  80
   Trp Ile Gly Glu Asn Val Ser Gly Leu Gln Arg Ala Lys Thr Gly Thr
                   85                  90                  95
   Asp Lys Thr Leu Val Lys Glu Val Val Gln Asn Phe Ala Lys Glu Phe
               100                 105                 110
   Val Ile Ser Asp Arg Lys Glu Leu Glu Glu Asp Phe Ile Lys Ser Glu
           115                 120                 125
   Leu Lys Lys Ala Gly Gly Ala Asn Tyr Asp Ala Gln Thr Glu
       130                 135                 140
<210> 11
   <211> 379
   <212> PRT
   <213> Homo sapiens
<400> 11
   Met Ala Ala Ala Ala Ala Gln Gly Gly Gly Gly Gly Glu Pro Arg Arg
   1               5                   10                  15
   Thr Glu Gly Val Gly Pro Gly Val Pro Gly Glu Val Glu Met Val Lys
               20                  25                  30
   Gly Gln Pro Phe Asp Val Gly Pro Arg Tyr Thr Gln Leu Gln Tyr Ile
           35                  40                  45
   Gly Glu Gly Ala Tyr Gly Met Val Ser Ser Ala Tyr Asp His Val Arg
       50                  55                  60
   Lys Thr Arg Val Ala Ile Lys Lys Ile Ser Pro Phe Glu His Gln Thr
   65                  70                  75                  80
   Tyr Cys Gln Arg Thr Leu Arg Glu Ile Gln Ile Leu Leu Arg Phe Arg
                   85                  90                  95
   His Glu Asn Val Ile Gly Ile Arg Asp Ile Leu Arg Ala Ser Thr Leu
               100                 105                 110
   Glu Ala Met Arg Asp Val Tyr Ile Val Gln Asp Leu Met Glu Thr Asp
           115                 120                 125
   Leu Tyr Lys Leu Leu Lys Ser Gln Gln Leu Ser Asn Asp His Ile Cys
       130                 135                 140
   Tyr Phe Leu Tyr Gln Ile Leu Arg Gly Leu Lys Tyr Ile His Ser Ala
   145                 150                 155                 160
   Asn Val Leu His Arg Asp Leu Lys Pro Ser Asn Leu Leu Ile Asn Thr
                   165                 170                 175
   Thr Cys Asp Leu Lys Ile Cys Asp Phe Gly Leu Ala Arg Ile Ala Asp
               180                 185                 190
   Pro Glu His Asp His Thr Gly Phe Leu Thr Glu Tyr Val Ala Thr Arg
           195                 200                 205
   Trp Tyr Arg Ala Pro Glu Ile Met Leu Asn Ser Lys Gly Tyr Thr Lys
       210                 215                 220
   Ser Ile Asp Ile Trp Ser Val Gly Cys Ile Leu Ala Glu Met Leu Ser
   225                 230                 235                 240
   Asn Arg Pro Ile Phe Pro Gly Lys His Tyr Leu Asp Gln Leu Asn His
                   245                 250                 255
   Ile Leu Gly Ile Leu Gly Ser Pro Ser Gln Glu Asp Leu Asn Cys Ile
               260                 265                 270
   Ile Asn Met Lys Ala Arg Asn Tyr Leu Gln Ser Leu Pro Ser Lys Thr
           275                 280                 285
   Lys Val Ala Trp Ala Lys Leu Phe Pro Lys Ser Asp Ser Lys Ala Leu
       290                 295                 300
   Asp Leu Leu Asp Arg Met Leu Thr Phe Asn Pro Asn Lys Arg Ile Thr
   305                 310                 315                 320
   Val Glu Glu Ala Leu Ala His Pro Tyr Leu Glu Gln Tyr Tyr Asp Pro
                   325                 330                 335
   Thr Asp Glu Pro Val Ala Glu Glu Pro Phe Thr Phe Ala Met Glu Leu
               340                 345                 350
   Asp Asp Leu Pro Lys Glu Arg Leu Lys Glu Leu Ile Phe Gln Glu Thr
           355                 360                 365
   Ala Arg Phe Gln Pro Gly Val Leu Glu Ala Pro
       370                 375
<210> 12
   <211> 282
   <212> PRT
   <213> Homo sapiens
<400> 12
   Met Glu Arg Pro Ser Leu Arg Ala Leu Leu Leu Gly Ala Ala Gly Leu
   1               5                   10                  15
   Leu Leu Leu Leu Leu Pro Leu Ser Ser Ser Ser Ser Ser Asp Thr Cys
               20                  25                  30
   Gly Pro Cys Glu Pro Ala Ser Cys Pro Pro Leu Pro Pro Leu Gly Cys
           35                  40                  45
   Leu Leu Gly Glu Thr Arg Asp Ala Cys Gly Cys Cys Pro Met Cys Ala
       50                  55                  60
   Arg Gly Glu Gly Glu Pro Cys Gly Gly Gly Gly Ala Gly Arg Gly Tyr
   65                  70                  75                  80
   Cys Ala Pro Gly Met Glu Cys Val Lys Ser Arg Lys Arg Arg Lys Gly
                   85                  90                  95
   Lys Ala Gly Ala Ala Ala Gly Gly Pro Gly Val Ser Gly Val Cys Val
               100                 105                 110
   Cys Lys Ser Arg Tyr Pro Val Cys Gly Ser Asp Gly Thr Thr Tyr Pro
           115                 120                 125
   Ser Gly Cys Gln Leu Arg Ala Ala Ser Gln Arg Ala Glu Ser Arg Gly
       130                 135                 140
   Glu Lys Ala Ile Thr Gln Val Ser Lys Gly Thr Cys Glu Gln Gly Pro
   145 150 155 160
   Ser Ile Val Thr Pro Pro Lys Asp Ile Trp Asn Val Thr Gly Ala Gln
   165 170 175
   Val Tyr Leu Ser Cys Glu Val Ile Gly Ile Pro Thr Pro Val Leu Ile
   180 185 190
   Trp Asn Lys Val Lys Arg Gly His Tyr Gly Val Gln Arg Thr Glu Leu
   195 200 205
   Leu Pro Gly Asp Arg Asp Asn Leu Ala Ile Gln Thr Arg Gly Gly Pro
   210 215 220
   Glu Lys His Glu Val Thr Gly Trp Val Leu Val Ser Pro Leu Ser Lys
   225 230 235 240
   Glu Asp Ala Gly Glu Tyr Glu Cys His Ala Ser Asn Ser Gln Gly Gln
   245 250 255
   Ala Ser Ala Ser Ala Lys Ile Thr Val Val Asp Ala Leu His Glu Ile
   260 265 270
   Pro Val Lys Lys Gly Glu Gly Ala Glu Leu
   275 280
<210> 13
   <211> 552
   <212> PRT
   <213> Homo sapiens
<400> 13
<210> 14
   <211> 138
   <212> PRT
   <213> Homo sapiens
<400> 14
   Met Pro Asn Phe Ser Gly Asn Trp Lys Ile Ile Arg Ser Glu Asn Phe
   1 5 10 15
   Glu Glu Leu Leu Lys Val Leu Gly Val Asn Val Met Leu Arg Lys Ile
   20 25 30
   Ala Val Ala Ala Ala Ser Lys Pro Ala Val Glu Ile Lys Gln Glu Gly
   35 40 45
   Asp Thr Phe Tyr Ile Lys Thr Ser Thr Thr Val Arg Thr Thr Glu Ile
   50 55 60
   Asn Phe Lys Val Gly Glu Glu Phe Glu Glu Gln Thr Val Asp Gly Arg
   65                  70                  75                  80
   Pro Cys Lys Ser Leu Val Lys Trp Glu Ser Glu Asn Lys Met Val Cys
                   85                  90                  95
   Glu Gln Lys Leu Leu Lys Gly Glu Gly Pro Lys Thr Ser Trp Thr Arg
               100                 105                 110
   Glu Leu Thr Asn Asp Gly Glu Leu Ile Leu Thr Met Thr Ala Asp Asp
           115                 120                 125
   Val Val Cys Thr Arg Val Tyr Val Arg Glu
       130                 135
<210> 15
   <211> 89
   <212> PRT
   <213> Homo sapiens
<400> 15
   Met Asp Gly Glu Glu Lys Thr Tyr Gly Gly Cys Glu Gly Pro Asp Ala
   1               5                   10                  15
   Met Tyr Val Lys Leu Ile Ser Ser Asp Gly His Glu Phe Ile Val Lys
               20                  25                  30
   Arg Glu His Ala Leu Thr Ser Gly Thr Ile Lys Ala Met Leu Ser Gly
           35                  40                  45
   Pro Gly Gln Phe Ala Glu Asn Glu Thr Asn Glu Val Asn Phe Arg Glu
       50                  55                  60
   Ile Pro Ser His Val Leu Ser Lys Val Cys Met Tyr Phe Thr Tyr Lys
   65                  70                  75                  80
   Val Arg Tyr Thr Asn Ser Ser Thr Glu
                   85
<210> 16
   <211> 50
   <212> PRT
   <213> Homo sapiens
<400> 16
   Met Leu Ile Lys Val Lys Thr Leu Thr Gly Lys Glu Ile Glu Ile Asp
   1 5 10 15
   Ile Glu Pro Thr Asp Lys Val Glu Arg Ile Lys Glu Arg Val Glu Glu
   20 25 30
   Lys Glu Gly Ile Pro Pro Gln Gln Gln Arg Leu Ile Tyr Ser Gly Lys
   35 40 45
   Gln Met
   50
<210> 17
   <211> 132
   <212> PRT
   <213> Homo sapiens
<400> 17
   Met Val Asp Ala Phe Leu Gly Thr Trp Lys Leu Val Asp Ser Lys Asn
   1 5 10 15
   Phe Asp Asp Tyr Met Lys Ser Leu Gly Val Gly Phe Ala Thr Arg Gln
   20 25 30
   Val Ala Ser Met Thr Lys Pro Thr Thr Ile Ile Glu Lys Asn Gly Asp
   35 40 45
   Ile Leu Thr Leu Lys Thr His Ser Thr Phe Lys Asn Thr Glu Ile Ser
   50 55 60
   Phe Lys Leu Gly Val Glu Phe Asp Glu Thr Thr Ala Asp Asp Arg Lys
   65 70 75 80
   Val Lys Ser Ile Val Thr Leu Asp Gly Gly Lys Leu Val His Leu Gln
   85 90 95
   Lys Trp Asp Gly Gln Glu Thr Thr Leu Val Arg Glu Leu Ile Asp Gly
   100 105 110
   Lys Leu Ile Leu Thr Leu Thr His Gly Thr Ala Val Cys Thr Arg Thr
   115 120 125
   Tyr Glu Lys Glu
   130
<210> 18
   <211> 698
   <212> PRT
   <213> Homo sapiens
<400> 18
   Met Arg Leu Ala Val Gly Ala Leu Leu Val Cys Ala Val Leu Gly Leu
   1               5                   10                  15
   Cys Leu Ala Val Pro Asp Lys Thr Val Arg Trp Cys Ala Val Ser Glu
               20                  25                  30
   His Glu Ala Thr Lys Cys Gln Ser Phe Arg Asp His Met Lys Ser Val
           35                  40                  45
   Ile Pro Ser Asp Gly Pro Ser Val Ala Cys Val Lys Lys Ala Ser Tyr
       50                  55                  60
   Leu Asp Cys Ile Arg Ala Ile Ala Ala Asn Glu Ala Asp Ala Val Thr
   65                  70                  75                  80
   Leu Asp Ala Gly Leu Val Tyr Asp Ala Tyr Leu Ala Pro Asn Asn Leu
                   85                  90                  95
   Lys Pro Val Val Ala Glu Phe Tyr Gly Ser Lys Glu Asp Pro Gln Thr
               100                 105                 110
   Phe Tyr Tyr Ala Val Ala Val Val Lys Lys Asp Ser Gly Phe Gln Met
           115                 120                 125
   Asn Gln Leu Arg Gly Lys Lys Ser Cys His Thr Gly Leu Gly Arg Ser
       130                 135                 140
   Ala Gly Trp Asn Ile Pro Ile Gly Leu Leu Tyr Cys Asp Leu Pro Glu
   145                 150                 155                 160
   Pro Arg Lys Pro Leu Glu Lys Ala Val Ala Asn Phe Phe Ser Gly Ser
                   165                 170                 175
   Cys Ala Pro Cys Ala Asp Gly Thr Asp Phe Pro Gln Leu Cys Gln Leu
               180                 185                 190
   Cys Pro Gly Cys Gly Cys Ser Thr Leu Asn Gln Tyr Phe Gly Tyr Ser
           195                 200                 205
   Gly Ala Phe Lys Cys Leu Lys Asp Gly Ala Gly Asp Val Ala Phe Val
       210                 215                 220
   Lys His Ser Thr Ile Phe Glu Asn Leu Ala Asn Lys Ala Asp Arg Asp
   225                 230                 235                 240
   Gln Tyr Glu Leu Leu Cys Leu Asp Asn Thr Arg Lys Pro Val Asp Glu
                   245                 250                 255
   Tyr Lys Asp Cys His Leu Ala Gln Val Pro Ser His Thr Val Val Ala
               260                 265                 270
   Arg Ser Met Gly Gly Lys Glu Asp Leu Ile Trp Glu Leu Leu Asn Gln
           275                 280                 285
   Ala Gln Glu His Phe Gly Lys Asp Lys Ser Lys Glu Phe Gln Leu Phe
       290                 295                 300
   Ser Ser Pro His Gly Lys Asp Leu Leu Phe Lys Asp Ser Ala His Gly
   305                 310                 315                 320
   Phe Leu Lys Val Pro Pro Arg Met Asp Ala Lys Met Tyr Leu Gly Tyr
                   325                 330                 335
   Glu Tyr Val Thr Ala Ile Arg Asn Leu Arg Glu Gly Thr Cys Pro Glu
               340                 345                 350
   Ala Pro Thr Asp Glu Cys Lys Pro Val Lys Trp Cys Ala Leu Ser His
           355                 360                 365
   His Glu Arg Leu Lys Cys Asp Glu Trp Ser Val Asn Ser Val Gly Lys
       370                 375                 380
   Ile Glu Cys Val Ser Ala Glu Thr Thr Glu Asp Cys Ile Ala Lys Ile
   385                 390                 395                 400
   Met Asn Gly Glu Ala Asp Ala Met Ser Leu Asp Gly Gly Phe Val Tyr
                   405                 410                 415
   Ile Ala Gly Lys Cys Gly Leu Val Pro Val Leu Ala Glu Asn Tyr Asn
               420                 425                 430
   Lys Ser Asp Asn Cys Glu Asp Thr Pro Glu Ala Gly Tyr Phe Ala Ile
           435                 440                 445
   Ala Val Val Lys Lys Ser Ala Ser Asp Leu Thr Trp Asp Asn Leu Lys
       450                 455                 460
   Gly Lys Lys Ser Cys His Thr Ala Val Gly Arg Thr Ala Gly Trp Asn
   465                 470                 475                 480
   Ile Pro Met Gly Leu Leu Tyr Asn Lys Ile Asn His Cys Arg Phe Asp
                   485                 490                 495
   Glu Phe Phe Ser Glu Gly Cys Ala Pro Gly Ser Lys Lys Asp Ser Ser
               500                 505                 510
   Leu Cys Lys Leu Cys Met Gly Ser Gly Leu Asn Leu Cys Glu Pro Asn
           515                 520                 525
   Asn Lys Glu Gly Tyr Tyr Gly Tyr Thr Gly Ala Phe Arg Cys Leu Val
       530                 535                 540
   Glu Lys Gly Asp Val Ala Phe Val Lys His Gln Thr Val Pro Gln Asn
   545                 550                 555                 560
   Thr Gly Gly Lys Asn Pro Asp Pro Trp Ala Lys Asn Leu Asn Glu Lys
                   565                 570                 575
   Asp Tyr Glu Leu Leu Cys Leu Asp Gly Thr Arg Lys Pro Val Glu Glu
               580                 585                 590
   Tyr Ala Asn Cys His Leu Ala Arg Ala Pro Asn His Ala Val Val Thr
   595 600 605
   Arg Lys Asp Lys Glu Ala Cys Val His Lys Ile Leu Arg Gln Gln Gln
   610 615 620
   His Leu Phe Gly Ser Asn Val Thr Asp Cys Ser Gly Asn Phe Cys Leu
   625 630 635 640
   Phe Arg Ser Glu Thr Lys Asp Leu Leu Phe Arg Asp Asp Thr Val Cys
   645 650 655
   Leu Ala Lys Leu His Asp Arg Asn Thr Tyr Glu Lys Tyr Leu Gly Glu
   660 665 670
   Glu Tyr Val Lys Ala Val Gly Asn Leu Arg Lys Cys Ser Thr Ser Ser
   675 680 685
   Leu Leu Glu Ala Cys Thr Phe Arg Arg Pro
   690 695
<210> 19
   <211> 3396
   <212> PRT
   <213> Homo sapiens
<400> 19
   Met Phe Ile Asn Ile Lys Ser Ile Leu Trp Met Cys Ser Thr Leu Ile
   1 5 10 15
   Val Thr His Ala Leu His Lys Val Lys Val Gly Lys Ser Pro Pro Val
   20 25 30
   Arg Gly Ser Leu Ser Gly Lys Val Ser Leu Pro Cys His Phe Ser Thr
   35 40 45
   Met Pro Thr Leu Pro Pro Ser Tyr Asn Thr Ser Glu Phe Leu Arg Ile
   50 55 60
   Lys Trp Ser Lys Ile Glu Val Asp Lys Asn Gly Lys Asp Leu Lys Glu
   65 70 75 80
   Thr Thr Val Leu Val Ala Gln Asn Gly Asn Ile Lys Ile Gly Gln Asp
   85 90 95
   Tyr Lys Gly Arg Val Ser Val Pro Thr His Pro Glu Ala Val Gly Asp
   100 105 110
   Ala Ser Leu Thr Val Val Lys Leu Leu Ala Ser Asp Ala Gly Leu Tyr
   115 120 125
   Arg Cys Asp Val Met Tyr Gly Ile Glu Asp Thr Gln Asp Thr Val Ser
   130 135 140
   Leu Thr Val Asp Gly Val Val Phe His Tyr Arg Ala Ala Thr Ser Arg
   145 150 155 160
   Tyr Thr Leu Asn Phe Glu Ala Ala Gln Lys Ala Cys Leu Asp Val Gly
   165 170 175
   Ala Val Ile Ala Thr Pro Glu Gln Leu Phe Ala Ala Tyr Glu Asp Gly
   180 185 190
   Phe Glu Gln Cys Asp Ala Gly Trp Leu Ala Asp Gln Thr Val Arg Tyr
   195 200 205
   Pro Ile Arg Ala Pro Arg Val Gly Cys Tyr Gly Asp Lys Met Gly Lys
   210 215 220
   Ala Gly Val Arg Thr Tyr Gly Phe Arg Ser Pro Gln Glu Thr Tyr Asp
   225 230 235 240
   Val Tyr Cys Tyr Val Asp His Leu Asp Gly Asp Val Phe His Leu Thr
   245 250 255
   Val Pro Ser Lys Phe Thr Phe Glu Glu Ala Ala Lys Glu Cys Glu Asn
   260 265 270
   Gln Asp Ala Arg Leu Ala Thr Val Gly Glu Leu Gln Ala Ala Trp Arg
   275 280 285
   Asn Gly Phe Asp Gln Cys Asp Tyr Gly Trp Leu Ser Asp Ala Ser Val
   290 295 300
   Arg His Pro Val Thr Val Ala Arg Ala Gln Cys Gly Gly Gly Leu Leu
   305 310 315 320
   Gly Val Arg Thr Leu Tyr Arg Phe Glu Asn Gln Thr Gly Phe Pro Pro
   325 330 335
   Pro Asp Ser Arg Phe Asp Ala Tyr Cys Phe Lys Pro Lys Glu Ala Thr
   340 345 350
   Thr Ile Asp Leu Ser Ile Leu Ala Glu Thr Ala Ser Pro Ser Leu Ser
   355 360 365
   Lys Glu Pro Gln Met Val Ser Asp Arg Thr Thr Pro Ile Ile Pro Leu
   370 375 380
   Val Asp Glu Leu Pro Val Ile Pro Thr Glu Phe Pro Pro Val Gly Asn
   385 390 395 400
   Ile Val Ser Phe Glu Gln Lys Ala Thr Val Gln Pro Gln Ala Ile Thr
   405 410 415
   Asp Ser Leu Ala Thr Lys Leu Pro Thr Pro Thr Gly Ser Thr Lys Lys
   420 425 430
   Pro Trp Asp Met Asp Asp Tyr Ser Pro Ser Ala Ser Gly Pro Leu Gly
   435 440 445
   Lys Leu Asp Ile Ser Glu Ile Lys Glu Glu Val Leu Gln Ser Thr Thr
   450 455 460
   Gly Val Ser His Tyr Ala Thr Asp Ser Trp Asp Gly Val Val Glu Asp
   465 470 475 480
   Lys Gln Thr Gln Glu Ser Val Thr Gln Ile Glu Gln Ile Glu Val Gly
   485 490 495
   Pro Leu Val Thr Ser Met Glu Ile Leu Lys His Ile Pro Ser Lys Glu
   500 505 510
   Phe Pro Val Thr Glu Thr Pro Leu Val Thr Ala Arg Met Ile Leu Glu
   515 520 525
   Ser Lys Thr Glu Lys Lys Met Val Ser Thr Val Ser Glu Leu Val Thr
   530 535 540
   Thr Gly His Tyr Gly Phe Thr Leu Gly Glu Glu Asp Asp Glu Asp Arg
   545 550 555 560
   Thr Leu Thr Val Gly Ser Asp Glu Ser Thr Leu Ile Phe Asp Gln Ile
   565 570 575
   Pro Glu Val Ile Thr Val Ser Lys Thr Ser Glu Asp Thr Ile His Thr
   580 585 590
   His Leu Glu Asp Leu Glu Ser Val Ser Ala Ser Thr Thr Val Ser Pro
   595 600 605
   Leu Ile Met Pro Asp Asn Asn Gly Ser Ser Met Asp Asp Trp Glu Glu
   610 615 620
   Arg Gln Thr Ser Gly Arg Ile Thr Glu Glu Phe Leu Gly Lys Tyr Leu
   625 630 635 640
   Ser Thr Thr Pro Phe Pro Ser Gln His Arg Thr Glu Ile Glu Leu Phe
   645 650 655
   Pro Tyr Ser Gly Asp Lys Ile Leu Val Glu Gly Ile Ser Thr Val Ile
   660 665 670
   Tyr Pro Ser Leu Gln Thr Glu Met Thr His Arg Arg Glu Arg Thr Glu
   675 680 685
   Thr Leu Ile Pro Glu Met Arg Thr Asp Thr Tyr Thr Asp Glu Ile Gln
   690 695 700
   Glu Glu Ile Thr Lys Ser Pro Phe Met Gly Lys Thr Glu Glu Glu Val
   705 710 715 720
   Phe Ser Gly Met Lys Leu Ser Thr Ser Leu Ser Glu Pro Ile His Val
   725 730 735
   Thr Glu Ser Ser Val Glu Met Thr Lys Ser Phe Asp Phe Pro Thr Leu
   740 745 750
   Ile Thr Lys Leu Ser Ala Glu Pro Thr Glu Val Arg Asp Met Glu Glu
   755 760 765
   Asp Phe Thr Ala Thr Pro Gly Thr Thr Lys Tyr Asp Glu Asn Ile Thr
   770 775 780
   Thr Val Leu Leu Ala His Gly Thr Leu Ser Val Glu Ala Ala Thr Val
   785 790 795 800
   Ser Lys Trp Ser Trp Asp Glu Asp Asn Thr Thr Ser Lys Pro Leu Glu
   805 810 815
   Ser Thr Glu Pro Ser Ala Ser Ser Lys Leu Pro Pro Ala Leu Leu Thr
   820 825 830
   Thr Val Gly Met Asn Gly Lys Asp Lys Asp Ile Pro Ser Phe Thr Glu
   835 840 845
   Asp Gly Ala Asp Glu Phe Thr Leu Ile Pro Asp Ser Thr Gln Lys Gln
   850 855 860
   Leu Glu Glu Val Thr Asp Glu Asp Ile Ala Ala His Gly Lys Phe Thr
   865 870 875 880
   Ile Arg Phe Gln Pro Thr Thr Ser Thr Gly Ile Ala Glu Lys Ser Thr
   885 890 895
   Leu Arg Asp Ser Thr Thr Glu Glu Lys Val Pro Pro Ile Thr Ser Thr
   900 905 910
   Glu Gly Gln Val Tyr Ala Thr Met Glu Gly Ser Ala Leu Gly Glu Val
   915 920 925
   Glu Asp Val Asp Leu Ser Lys Pro Val Ser Thr Val Pro Gln Phe Ala
   930 935 940
   His Thr Ser Glu Val Glu Gly Leu Ala Phe Val Ser Tyr Ser Ser Thr
   945 950 955 960
   Gln Glu Pro Thr Thr Tyr Val Asp Ser Ser His Thr Ile Pro Leu Ser
   965 970 975
   Val Ile Pro Lys Thr Asp Trp Gly Val Leu Val Pro Ser Val Pro Ser
   980 985 990
   Glu Asp Glu Val Leu Gly Glu Pro Ser Gln Asp Ile Leu Val Ile Asp
   995 1000 1005
   Gln Thr Arg Leu Glu Ala Thr Ile Ser Pro Glu Thr Met Arg Thr
   1010 1015 1020
   Thr Lys Ile Thr Glu Gly Thr Thr Gln Glu Glu Phe Pro Trp Lys
   1025 1030 1035
   Glu Gln Thr Ala Glu Lys Pro Val Pro Ala Leu Ser Ser Thr Ala
   1040 1045 1050
   Trp Thr Pro Lys Glu Ala Val Thr Pro Leu Asp Glu Gln Glu Gly
   1055 1060 1065
   Asp Gly Ser Ala Tyr Thr Val Ser Glu Asp Glu Leu Leu Thr Gly
   1070 1075 1080
   Ser Glu Arg Val Pro Val Leu Glu Thr Thr Pro Val Gly Lys Ile
   1085 1090 1095
   Asp His Ser Val Ser Tyr Pro Pro Gly Ala Val Thr Glu His Lys
   1100 1105 1110
   Val Lys Thr Asp Glu Val Val Thr Leu Thr Pro Arg Ile Gly Pro
   1115 1120 1125
   Lys Val Ser Leu Ser Pro Gly Pro Glu Gln Lys Tyr Glu Thr Glu
   1130 1135 1140
   Gly Ser Ser Thr Thr Gly Phe Thr Ser Ser Leu Ser Pro Phe Ser
   1145 1150 1155
   Thr His Ile Thr Gln Leu Met Glu Glu Thr Thr Thr Glu Lys Thr
   1160 1165 1170
   Ser Leu Glu Asp Ile Asp Leu Gly Ser Gly Leu Phe Glu Lys Pro
   1175 1180 1185
   Lys Ala Thr Glu Leu Ile Glu Phe Ser Thr Ile Lys Val Thr Val
   1190 1195 1200
   Pro Ser Asp Ile Thr Thr Ala Phe Ser Ser Val Asp Arg Leu His
   1205 1210 1215
   Thr Thr Ser Ala Phe Lys Pro Ser Ser Ala Ile Thr Lys Lys Pro
   1220 1225 1230
   Pro Leu Ile Asp Arg Glu Pro Gly Glu Glu Thr Thr Ser Asp Met
   1235 1240 1245
   Val Ile Ile Gly Glu Ser Thr Ser His Val Pro Pro Thr Thr Leu
   1250 1255 1260
   Glu Asp Ile Val Ala Lys Glu Thr Glu Thr Asp Ile Asp Arg Glu
   1265 1270 1275
   Tyr Phe Thr Thr Ser Ser Pro Pro Ala Thr Gln Pro Thr Arg Pro
   1280 1285 1290
   Pro Thr Val Glu Asp Lys Glu Ala Phe Gly Pro Gln Ala Leu Ser
   1295 1300 1305
   Thr Pro Gln Pro Pro Ala Ser Thr Lys Phe His Pro Asp Ile Asn
   1310 1315 1320
   Val Tyr Ile Ile Glu Val Arg Glu Asn Lys Thr Gly Arg Met Ser
   1325 1330 1335
   Asp Leu Ser Val Ile Gly His Pro Ile Asp Ser Glu Ser Lys Glu
   1340 1345 1350
   Asp Glu Pro Cys Ser Glu Glu Thr Asp Pro Val His Asp Leu Met
   1355 1360 1365
   Ala Glu Ile Leu Pro Glu Phe Pro Asp Ile Ile Glu Ile Asp Leu
   1370 1375 1380
   Tyr His Ser Glu Glu Asn Glu Glu Glu Glu Glu Glu Cys Ala Asn
   1385 1390 1395
   Ala Thr Asp Val Thr Thr Thr Pro Ser Val Gln Tyr Ile Asn Gly
   1400 1405 1410
   Lys His Leu Val Thr Thr Val Pro Lys Asp Pro Glu Ala Ala Glu
   1415 1420 1425
   Ala Arg Arg Gly Gln Phe Glu Ser Val Ala Pro Ser Gln Asn Phe
   1430 1435 1440
   Ser Asp Ser Ser Glu Ser Asp Thr His Pro Phe Val Ile Ala Lys
   1445 1450 1455
   Thr Glu Leu Ser Thr Ala Val Gln Pro Asn Glu Ser Thr Glu Thr
   1460 1465 1470
   Thr Glu Ser Leu Glu Val Thr Trp Lys Pro Glu Thr Tyr Pro Glu
   1475 1480 1485
   Thr Ser Glu His Phe Ser Gly Gly Glu Pro Asp Val Phe Pro Thr
   1490 1495 1500
   Val Pro Phe His Glu Glu Phe Glu Ser Gly Thr Ala Lys Lys Gly
   1505 1510 1515
   Ala Glu Ser Val Thr Glu Arg Asp Thr Glu Val Gly His Gln Ala
   1520 1525 1530
   His Glu His Thr Glu Pro Val Ser Leu Phe Pro Glu Glu Ser Ser
   1535 1540 1545
   Gly Glu Ile Ala Ile Asp Gln Glu Ser Gln Lys Ile Ala Phe Ala
   1550 1555 1560
   Arg Ala Thr Glu Val Thr Phe Gly Glu Glu Val Glu Lys Ser Thr
   1565 1570 1575
   Ser Val Thr Tyr Thr Pro Thr Ile Val Pro Ser Ser Ala Ser Ala
   1580 1585 1590
   Tyr Val Ser Glu Glu Glu Ala Val Thr Leu Ile Gly Asn Pro Trp
   1595 1600 1605
   Pro Asp Asp Leu Leu Ser Thr Lys Glu Ser Trp Val Glu Ala Thr
   1610 1615 1620
   Pro Arg Gln Val Val Glu Leu Ser Gly Ser Ser Ser Ile Pro Ile
   1625 1630 1635
   Thr Glu Gly Ser Gly Glu Ala Glu Glu Asp Glu Asp Thr Met Phe
   1640 1645 1650
   Thr Met Val Thr Asp Leu Ser Gln Arg Asn Thr Thr Asp Thr Leu
   1655 1660 1665
   Ile Thr Leu Asp Thr Ser Arg Ile Ile Thr Glu Ser Phe Phe Glu
   1670 1675 1680
   Val Pro Ala Thr Thr Ile Tyr Pro Val Ser Glu Gln Pro Ser Ala
   1685 1690 1695
   Lys Val Val Pro Thr Lys Phe Val Ser Glu Thr Asp Thr Ser Glu
   1700 1705 1710
   Trp Ile Ser Ser Thr Thr Val Glu Glu Lys Lys Arg Lys Glu Glu
   1715 1720 1725
   Glu Gly Thr Thr Gly Thr Ala Ser Thr Phe Glu Val Tyr Ser Ser
   1730 1735 1740
   Thr Gln Arg Ser Asp Gln Leu Ile Leu Pro Phe Glu Leu Glu Ser
   1745 1750 1755
   Pro Asn Val Ala Thr Ser Ser Asp Ser Gly Thr Arg Lys Ser Phe
   1760 1765 1770
   Met Ser Leu Thr Thr Pro Thr Gln Ser Glu Arg Glu Met Thr Asp
   1775 1780 1785
   Ser Thr Pro Val Phe Thr Glu Thr Asn Thr Leu Glu Asn Leu Gly
   1790 1795 1800
   Ala Gln Thr Thr Glu His Ser Ser Ile His Gln Pro Gly Val Gln
   1805 1810 1815
   Glu Gly Leu Thr Thr Leu Pro Arg Ser Pro Ala Ser Val Phe Met
   1820 1825 1830
   Glu Gln Gly Ser Gly Glu Ala Ala Ala Asp Pro Glu Thr Thr Thr
   1835 1840 1845
   Val Ser Ser Phe Ser Leu Asn Val Glu Tyr Ala Ile Gln Ala Glu
   1850 1855 1860
   Lys Glu Val Ala Gly Thr Leu Ser Pro His Val Glu Thr Thr Phe
   1865 1870 1875
   Ser Thr Glu Pro Thr Gly Leu Val Leu Ser Thr Val Met Asp Arg
   1880 1885 1890
   Val Val Ala Glu Asn Ile Thr Gln Thr Ser Arg Glu Ile Val Ile
   1895 1900 1905
   Ser Glu Arg Leu Gly Glu Pro Asn Tyr Gly Ala Glu Ile Arg Gly
   1910 1915 1920
   Phe Ser Thr Gly Phe Pro Leu Glu Glu Asp Phe Ser Gly Asp Phe
   1925 1930 1935
   Arg Glu Tyr Ser Thr Val Ser His Pro Ile Ala Lys Glu Glu Thr
   1940 1945 1950
   Val Met Met Glu Gly Ser Gly Asp Ala Ala Phe Arg Asp Thr Gln
   1955 1960 1965
   Thr Ser Pro Ser Thr Val Pro Thr Ser Val His Ile Ser His Ile
   1970 1975 1980
   Ser Asp Ser Glu Gly Pro Ser Ser Thr Met Val Ser Thr Ser Ala
   1985 1990 1995
   Phe Pro Trp Glu Glu Phe Thr Ser Ser Ala Glu Gly Ser Gly Glu
   2000 2005 2010
   Gln Leu Val Thr Val Ser Ser Ser Val Val Pro Val Leu Pro Ser
   2015 2020 2025
   Ala Val Gln Lys Phe Ser Gly Thr Ala Ser Ser Ile Ile Asp Glu
   2030 2035 2040
   Gly Leu Gly Glu Val Gly Thr Val Asn Glu Ile Asp Arg Arg Ser
   2045 2050 2055
   Thr Ile Leu Pro Thr Ala Glu Val Glu Gly Thr Lys Ala Pro Val
   2060 2065 2070
   Glu Lys Glu Glu Val Lys Val Ser Gly Thr Val Ser Thr Asn Phe
   2075 2080 2085
   Pro Gln Thr Ile Glu Pro Ala Lys Leu Trp Ser Arg Gln Glu Val
   2090 2095 2100
   Asn Pro Val Arg Gln Glu Ile Glu Ser Glu Thr Thr Ser Glu Glu
   2105 2110 2115
   Gln Ile Gln Glu Glu Lys Ser Phe Glu Ser Pro Gln Asn Ser Pro
   2120 2125 2130
   Ala Thr Glu Gln Thr Ile Phe Asp Ser Gln Thr Phe Thr Glu Thr
   2135 2140 2145
   Glu Leu Lys Thr Thr Asp Tyr Ser Val Leu Thr Thr Lys Lys Thr
   2150 2155 2160
   Tyr Ser Asp Asp Lys Glu Met Lys Glu Glu Asp Thr Ser Leu Val
   2165 2170 2175
   Asn Met Ser Thr Pro Asp Pro Asp Ala Asn Gly Leu Glu Ser Tyr
   2180 2185 2190
   Thr Thr Leu Pro Glu Ala Thr Glu Lys Ser His Phe Phe Leu Ala
   2195 2200 2205
   Thr Ala Leu Val Thr Glu Ser Ile Pro Ala Glu His Val Val Thr
   2210 2215 2220
   Asp Ser Pro Ile Lys Lys Glu Glu Ser Thr Lys His Phe Pro Lys
   2225 2230 2235
   Gly Met Arg Pro Thr Ile Gln Glu Ser Asp Thr Glu Leu Leu Phe
   2240 2245 2250
   Ser Gly Leu Gly Ser Gly Glu Glu Val Leu Pro Thr Leu Pro Thr
   2255 2260 2265
   Glu Ser Val Asn Phe Thr Glu Val Glu Gln Ile Asn Asn Thr Leu
   2270 2275 2280
   Tyr Pro His Thr Ser Gln Val Glu Ser Thr Ser Ser Asp Lys Ile
   2285 2290 2295
   Glu Asp Phe Asn Arg Met Glu Asn Val Ala Lys Glu Val Gly Pro
   2300 2305 2310
   Leu Val Ser Gln Thr Asp Ile Phe Glu Gly Ser Gly Ser Val Thr
   2315 2320 2325
   Ser Thr Thr Leu Ile Glu Ile Leu Ser Asp Thr Gly Ala Glu Gly
   2330 2335 2340
   Pro Thr Val Ala Pro Leu Pro Phe Ser Thr Asp Ile Gly His Pro
   2345 2350 2355
   Gln Asn Gln Thr Val Arg Trp Ala Glu Glu Ile Gln Thr Ser Arg
   2360 2365 2370
   Pro Gln Thr Ile Thr Glu Gln Asp Ser Asn Lys Asn Ser Ser Thr
   2375 2380 2385
   Ala Glu Ile Asn Glu Thr Thr Thr Ser Ser Thr Asp Phe Leu Ala
   2390 2395 2400
   Arg Ala Tyr Gly Phe Glu Met Ala Lys Glu Phe Val Thr Ser Ala
   2405 2410 2415
   Pro Lys Pro Ser Asp Leu Tyr Tyr Glu Pro Ser Gly Glu Gly Ser
   2420 2425 2430
   Gly Glu Val Asp Ile Val Asp Ser Phe His Thr Ser Ala Thr Thr
   2435 2440 2445
   Gln Ala Thr Arg Gln Glu Ser Ser Thr Thr Phe Val Ser Asp Gly
   2450 2455 2460
   Ser Leu Glu Lys His Pro Glu Val Pro Ser Ala Lys Ala Val Thr
   2465 2470 2475
   Ala Asp Gly Phe Pro Thr Val Ser Val Met Leu Pro Leu His Ser
   2480 2485 2490
   Glu Gln Asn Lys Ser Ser Pro Asp Pro Thr Ser Thr Leu Ser Asn
   2495 2500 2505
   Thr Val Ser Tyr Glu Arg Ser Thr Asp Gly Ser Phe Gln Asp Arg
   2510 2515 2520
   Phe Arg Glu Phe Glu Asp Ser Thr Leu Lys Pro Asn Arg Lys Lys
   2525 2530 2535
   Pro Thr Glu Asn Ile Ile Ile Asp Leu Asp Lys Glu Asp Lys Asp
   2540 2545 2550
   Leu Ile Leu Thr Ile Thr Glu Ser Thr Ile Leu Glu Ile Leu Pro
   2555 2560 2565
   Glu Leu Thr Ser Asp Lys Asn Thr Ile Ile Asp Ile Asp His Thr
   2570 2575 2580
   Lys Pro Val Tyr Glu Asp Ile Leu Gly Met Gln Thr Asp Ile Asp
   2585 2590 2595
   Thr Glu Val Pro Ser Glu Pro His Asp Ser Asn Asp Glu Ser Asn
   2600 2605 2610
   Asp Asp Ser Thr Gln Val Gln Glu Ile Tyr Glu Ala Ala Val Asn
   2615 2620 2625
   Leu Ser Leu Thr Glu Glu Thr Phe Glu Gly Ser Ala Asp Val Leu
   2630 2635 2640
   Ala Ser Tyr Thr Gln Ala Thr His Asp Glu Ser Met Thr Tyr Glu
   2645 2650 2655
   Asp Arg Ser Gln Leu Asp His Met Gly Phe His Phe Thr Thr Gly
   2660 2665 2670
   Ile Pro Ala Pro Ser Thr Glu Thr Glu Leu Asp Val Leu Leu Pro
   2675 2680 2685
   Thr Ala Thr Ser Leu Pro Ile Pro Arg Lys Ser Ala Thr Val Ile
   2690 2695 2700
   Pro Glu Ile Glu Gly Ile Lys Ala Glu Ala Lys Ala Leu Asp Asp
   2705 2710 2715
   Met Phe Glu Ser Ser Thr Leu Ser Asp Gly Gln Ala Ile Ala Asp
   2720 2725 2730
   Gln Ser Glu Ile Ile Pro Thr Leu Gly Gln Phe Glu Arg Thr Gln
   2735 2740 2745
   Glu Glu Tyr Glu Asp Lys Lys His Ala Gly Pro Ser Phe Gln Pro
   2750 2755 2760
   Glu Phe Ser Ser Gly Ala Glu Glu Ala Leu Val Asp His Thr Pro
   2765 2770 2775
   Tyr Leu Ser Ile Ala Thr Thr His Leu Met Asp Gln Ser Val Thr
   2780 2785 2790
   Glu Val Pro Asp Val Met Glu Gly Ser Asn Pro Pro Tyr Tyr Thr
   2795 2800 2805
   Asp Thr Thr Leu Ala Val Ser Thr Phe Ala Lys Leu Ser Ser Gln
   2810 2815 2820
   Thr Pro Ser Ser Pro Leu Thr Ile Tyr Ser Gly Ser Glu Ala Ser
   2825 2830 2835
   Gly His Thr Glu Ile Pro Gln Pro Ser Ala Leu Pro Gly Ile Asp
   2840 2845 2850
   Val Gly Ser Ser Val Met Ser Pro Gln Asp Ser Phe Lys Glu Ile
   2855 2860 2865
   His Val Asn Ile Glu Ala Thr Phe Lys Pro Ser Ser Glu Glu Tyr
   2870 2875 2880
   Leu His Ile Thr Glu Pro Pro Ser Leu Ser Pro Asp Thr Lys Leu
   2885 2890 2895
   Glu Pro Ser Glu Asp Asp Gly Lys Pro Glu Leu Leu Glu Glu Met
   2900 2905 2910
   Glu Ala Ser Pro Thr Glu Leu Ile Ala Val Glu Gly Thr Glu Ile
   2915 2920 2925
   Leu Gln Asp Phe Gln Asn Lys Thr Asp Gly Gln Val Ser Gly Glu
   2930 2935 2940
   Ala Ile Lys Met Phe Pro Thr Ile Lys Thr Pro Glu Ala Gly Thr
   2945 2950 2955
   Val Ile Thr Thr Ala Asp Glu Ile Glu Leu Glu Gly Ala Thr Gln
   2960 2965 2970
   Trp Pro His Ser Thr Ser Ala Ser Ala Thr Tyr Gly Val Glu Ala
   2975 2980 2985
   Gly Val Val Pro Trp Leu Ser Pro Gln Thr Ser Glu Arg Pro Thr
   2990 2995 3000
   Leu Ser Ser Ser Pro Glu Ile Asn Pro Glu Thr Gln Ala Ala Leu
   3005 3010 3015
   Ile Arg Gly Gln Asp Ser Thr Ile Ala Ala Ser Glu Gln Gln Val
   3020 3025 3030
   Ala Ala Arg Ile Leu Asp Ser Asn Asp Gln Ala Thr Val Asn Pro
   3035 3040 3045
   Val Glu Phe Asn Thr Glu Val Ala Thr Pro Pro Phe Ser Leu Leu
   3050 3055 3060
   Glu Thr Ser Asn Glu Thr Asp Phe Leu Ile Gly Ile Asn Glu Glu
   3065 3070 3075
   Ser Val Glu Gly Thr Ala Ile Tyr Leu Pro Gly Pro Asp Arg Cys
   3080 3085 3090
   Lys Met Asn Pro Cys Leu Asn Gly Gly Thr Cys Tyr Pro Thr Glu
   3095 3100 3105
   Thr Ser Tyr Val Cys Thr Cys Val Pro Gly Tyr Ser Gly Asp Gln
   3110 3115 3120
   Cys Glu Leu Asp Phe Asp Glu Cys His Ser Asn Pro Cys Arg Asn
   3125 3130 3135
   Gly Ala Thr Cys Val Asp Gly Phe Asn Thr Phe Arg Cys Leu Cys
   3140 3145 3150
   Leu Pro Ser Tyr Val Gly Ala Leu Cys Glu Gln Asp Thr Glu Thr
   3155 3160 3165
   Cys Asp Tyr Gly Trp His Lys Phe Gln Gly Gln Cys Tyr Lys Tyr
   3170 3175 3180
   Phe Ala His Arg Arg Thr Trp Asp Ala Ala Glu Arg Glu Cys Arg
   3185 3190 3195
   Leu Gln Gly Ala His Leu Thr Ser Ile Leu Ser His Glu Glu Gln
   3200 3205 3210
   Met Phe Val Asn Arg Val Gly His Asp Tyr Gln Trp Ile Gly Leu
   3215 3220 3225
   Asn Asp Lys Met Phe Glu His Asp Phe Arg Trp Thr Asp Gly Ser
   3230 3235 3240
   Thr Leu Gln Tyr Glu Asn Trp Arg Pro Asn Gln Pro Asp Ser Phe
   3245 3250 3255
   Phe Ser Ala Gly Glu Asp Cys Val Val Ile Ile Trp His Glu Asn
   3260 3265 3270
   Gly Gln Trp Asn Asp Val Pro Cys Asn Tyr His Leu Thr Tyr Thr
   3275 3280 3285
   Cys Lys Lys Gly Thr Val Ala Cys Gly Gln Pro Pro Val Val Glu
   3290 3295 3300
   Asn Ala Lys Thr Phe Gly Lys Met Lys Pro Arg Tyr Glu Ile Asn
   3305 3310 3315
   Ser Leu Ile Arg Tyr His Cys Lys Asp Gly Phe Ile Gln Arg His
   3320 3325 3330
   Leu Pro Thr Ile Arg Cys Leu Gly Asn Gly Arg Trp Ala Ile Pro
   3335 3340 3345
   Lys Ile Thr Cys Met Asn Pro Ser Ala Tyr Gln Arg Thr Tyr Ser
   3350 3355 3360
   Met Lys Tyr Phe Lys Asn Ser Ser Ser Ala Lys Asp Asn Ser Ile
   3365 3370 3375
   Asn Thr Ser Lys His Asp His Arg Trp Ser Arg Arg Trp Gln Glu
   3380 3385 3390
   Ser Arg Arg
   3395
<210> 21
   <211> 480
   <212> PRT
   <213> Homo sapiens
<400> 21
<210> 22
   <211> 291
   <212> PRT
   <213> Homo sapiens
<400> 22
   Met Gln Arg Ala Arg Pro Thr Leu Trp Ala Ala Ala Leu Thr Leu Leu
   1               5                   10                  15
   Val Leu Leu Arg Gly Pro Pro Val Ala Arg Ala Gly Ala Ser Ser Ala
               20                  25                  30
   Gly Leu Gly Pro Val Val Arg Cys Glu Pro Cys Asp Ala Arg Ala Leu
           35                  40                  45
   Ala Gln Cys Ala Pro Pro Pro Ala Val Cys Ala Glu Leu Val Arg Glu
       50                  55                  60
   Pro Gly Cys Gly Cys Cys Leu Thr Cys Ala Leu Ser Glu Gly Gln Pro
   65                  70                  75                  80
   Cys Gly Ile Tyr Thr Glu Arg Cys Gly Ser Gly Leu Arg Cys Gln Pro
                   85                  90                  95
   Ser Pro Asp Glu Ala Arg Pro Leu Gln Ala Leu Leu Asp Gly Arg Gly
               100                 105                 110
   Leu Cys Val Asn Ala Ser Ala Val Ser Arg Leu Arg Ala Tyr Leu Leu
           115                 120                 125
   Pro Ala Pro Pro Ala Pro Gly Asn Ala Ser Glu Ser Glu Glu Asp Arg
       130                 135                 140
   Ser Ala Gly Ser Val Glu Ser Pro Ser Val Ser Ser Thr His Arg Val
   145                 150                 155                 160
   Ser Asp Pro Lys Phe His Pro Leu His Ser Lys Ile Ile Ile Ile Lys
                   165                 170                 175
   Lys Gly His Ala Lys Asp Ser Gln Arg Tyr Lys Val Asp Tyr Glu Ser
               180                 185                 190
   Gln Ser Thr Asp Thr Gln Asn Phe Ser Ser Glu Ser Lys Arg Glu Thr
           195                 200                 205
   Glu Tyr Gly Pro Cys Arg Arg Glu Met Glu Asp Thr Leu Asn His Leu
       210                 215                 220
   Lys Phe Leu Asn Val Leu Ser Pro Arg Gly Val His Ile Pro Asn Cys
   225                 230                 235                 240
   Asp Lys Lys Gly Phe Tyr Lys Lys Lys Gln Cys Arg Pro Ser Lys Gly
                   245                 250                 255
   Arg Lys Arg Gly Phe Cys Trp Cys Val Asp Lys Tyr Gly Gln Pro Leu
               260                 265                 270
   Pro Gly Tyr Thr Thr Lys Gly Lys Glu Asp Val His Cys Tyr Ser Met
   275 280 285
   Gln Ser Lys
   290
<210> 23
   <211> 349
   <212> PRT
   <213> Homo sapiens
<400> 23
   Met Thr Ala Ala Ser Met Gly Pro Val Arg Val Ala Phe Val Val Leu
   1 5 10 15
   Leu Ala Leu Cys Ser Arg Pro Ala Val Gly Gln Asn Cys Ser Gly Pro
   20 25 30
   Cys Arg Cys Pro Asp Glu Pro Ala Pro Arg Cys Pro Ala Gly Val Ser
   35 40 45
   Leu Val Leu Asp Gly Cys Gly Cys Cys Arg Val Cys Ala Lys Gln Leu
   50 55 60
   Gly Glu Leu Cys Thr Glu Arg Asp Pro Cys Asp Pro His Lys Gly Leu
   65 70 75 80
   Phe Cys His Phe Gly Ser Pro Ala Asn Arg Lys Ile Gly Val Cys Thr
   85 90 95
   Ala Lys Asp Gly Ala Pro Cys Ile Phe Gly Gly Thr Val Tyr Arg Ser
   100 105 110
   Gly Glu Ser Phe Gln Ser Ser Cys Lys Tyr Gln Cys Thr Cys Leu Asp
   115 120 125
   Gly Ala Val Gly Cys Met Pro Leu Cys Ser Met Asp Val Arg Leu Pro
   130 135 140
   Ser Pro Asp Cys Pro Phe Pro Arg Arg Val Lys Leu Pro Gly Lys Cys
   145 150 155 160
   Cys Glu Glu Trp Val Cys Asp Glu Pro Lys Asp Gln Thr Val Val Gly
   165 170 175
   Pro Ala Leu Ala Ala Tyr Arg Leu Glu Asp Thr Phe Gly Pro Asp Pro
   180 185 190
   Thr Met Ile Arg Ala Asn Cys Leu Val Gln Thr Thr Glu Trp Ser Ala
   195 200 205
   Cys Ser Lys Thr Cys Gly Met Gly Ile Ser Thr Arg Val Thr Asn Asp
       210                 215                 220
   Asn Ala Ser Cys Arg Leu Glu Lys Gln Ser Arg Leu Cys Met Val Arg
   225                 230                 235                 240
   Pro Cys Glu Ala Asp Leu Glu Glu Asn Ile Lys Lys Gly Lys Lys Cys
                   245                 250                 255
   Ile Arg Thr Pro Lys Ile Ser Lys Pro Ile Lys Phe Glu Leu Ser Gly
               260                 265                 270
   Cys Thr Ser Met Lys Thr Tyr Arg Ala Lys Phe Cys Gly Val Cys Thr
           275                 280                 285
   Asp Gly Arg Cys Cys Thr Pro His Arg Thr Thr Thr Leu Pro Val Glu
       290                 295                 300
   Phe Lys Cys Pro Asp Gly Glu Val Met Lys Lys Asn Met Met Phe Ile
   305                 310                 315                 320
   Lys Thr Cys Ala Cys His Tyr Asn Cys Pro Gly Asp Asn Asp Ile Phe
                   325                 330                 335
   Glu Ser Leu Tyr Tyr Arg Lys Met Tyr Gly Asp Met Ala
               340                 345
<210> 24
   <211> 1134
   <212> PRT
   <213> Homo sapiens
<400> 24
<210> 25
   <211> 5890
   <212> PRT
   <213> Homo sapiens
<400> 25
   Met Glu Lys Glu Glu Thr Thr Arg Glu Leu Leu Leu Pro Asn Trp Gln
   1               5                   10                  15
   Gly Ser Gly Ser His Gly Leu Thr Ile Ala Gln Arg Asp Asp Gly Val
   20 25 30
   Phe Val Gln Glu Val Thr Gln Asn Ser Pro Ala Ala Arg Thr Gly Val
   35 40 45
   Val Lys Glu Gly Asp Gln Ile Val Gly Ala Thr Ile Tyr Phe Asp Asn
   50 55 60
   Leu Gln Ser Gly Glu Val Thr Gln Leu Leu Asn Thr Met Gly His His
   65 70 75 80
   Thr Val Gly Leu Lys Leu His Arg Lys Gly Asp Arg Ser Pro Glu Pro
   85 90 95
   Gly Gln Thr Trp Thr Arg Glu Val Phe Ser Ser Cys Ser Ser Glu Val
   100 105 110
   Val Leu Ser Gly Asp Asp Glu Glu Tyr Gln Arg Ile Tyr Thr Thr Lys
   115 120 125
   Ile Lys Pro Arg Leu Lys Ser Glu Asp Gly Val Glu Gly Asp Leu Gly
   130 135 140
   Glu Thr Gln Ser Arg Thr Ile Thr Val Thr Arg Arg Val Thr Ala Tyr
   145 150 155 160
   Thr Val Asp Val Thr Gly Arg Glu Gly Ala Lys Asp Ile Asp Ile Ser
   165 170 175
   Ser Pro Glu Phe Lys Ile Lys Ile Pro Arg His Glu Leu Thr Glu Ile
   180 185 190
   Ser Asn Val Asp Val Glu Thr Gln Ser Gly Lys Thr Val Ile Arg Leu
   195 200 205
   Pro Ser Gly Ser Gly Ala Ala Ser Pro Thr Gly Ser Ala Val Asp Ile
   210 215 220
   Arg Ala Gly Ala Ile Ser Ala Ser Gly Pro Glu Leu Gln Gly Ala Gly
   225 230 235 240
   His Ser Lys Leu Gln Val Thr Met Pro Gly Ile Lys Val Gly Gly Ser
   245 250 255
   Gly Val Asn Val Asn Ala Lys Gly Leu Asp Leu Gly Gly Arg Gly Gly
   260 265 270
   Val Gln Val Pro Ala Val Asp Ile Ser Ser Ser Leu Gly Gly Arg Ala
   275 280 285
   Val Glu Val Gln Gly Pro Ser Leu Glu Ser Gly Asp His Gly Lys Ile
   290 295 300
   Lys Phe Pro Thr Met Lys Val Pro Lys Phe Gly Val Ser Thr Gly Arg
   305 310 315 320
   Glu Gly Gln Thr Pro Lys Ala Gly Leu Arg Val Ser Ala Pro Glu Val
   325 330 335
   Ser Val Gly His Lys Gly Gly Lys Pro Gly Leu Thr Ile Gln Ala Pro
   340 345 350
   Gln Leu Glu Val Ser Val Pro Ser Ala Asn Ile Glu Gly Leu Glu Gly
   355 360 365
   Lys Leu Lys Gly Pro Gln Ile Thr Gly Pro Ser Leu Glu Gly Asp Leu
   370 375 380
   Gly Leu Lys Gly Ala Lys Pro Gln Gly His Ile Gly Val Asp Ala Ser
   385 390 395 400
   Ala Pro Gln Ile Gly Gly Ser Ile Thr Gly Pro Ser Val Glu Val Gln
   405 410 415
   Ala Pro Asp Ile Asp Val Gln Gly Pro Gly Ser Lys Leu Asn Val Pro
   420 425 430
   Lys Met Lys Val Pro Lys Phe Ser Val Ser Gly Ala Lys Gly Glu Glu
   435 440 445
   Thr Gly Ile Asp Val Thr Leu Pro Thr Gly Glu Val Thr Val Pro Gly
   450 455 460
   Val Ser Gly Asp Val Ser Leu Pro Glu Ile Ala Thr Gly Gly Leu Glu
   465 470 475 480
   Gly Lys Met Lys Gly Thr Lys Val Lys Thr Pro Glu Met Ile Ile Gln
   485 490 495
   Lys Pro Lys Ile Ser Met Gln Asp Val Asp Leu Ser Leu Gly Ser Pro
   500 505 510
   Lys Leu Lys Gly Asp Ile Lys Val Ser Ala Pro Gly Val Gln Gly Asp
   515 520 525
   Val Lys Gly Pro Gln Val Ala Leu Lys Gly Ser Arg Val Asp Ile Glu
   530 535 540
   Thr Pro Asn Leu Glu Gly Thr Leu Thr Gly Pro Arg Leu Gly Ser Pro
   545 550 555 560
   Ser Gly Lys Thr Gly Thr Cys Arg Ile Ser Met Ser Glu Val Asp Leu
                   565                 570                 575
   Asn Val Ala Ala Pro Lys Val Lys Gly Gly Val Asp Val Thr Leu Pro
               580                 585                 590
   Arg Val Glu Gly Lys Val Lys Val Pro Glu Val Asp Val Arg Gly Pro
           595                 600                 605
   Lys Val Asp Val Ser Ala Pro Asp Val Glu Ala His Gly Pro Glu Trp
       610                 615                 620
   Asn Leu Lys Met Pro Lys Met Lys Met Pro Thr Phe Ser Thr Pro Gly
   625                 630                 635                 640
   Ala Lys Gly Glu Gly Pro Asp Val His Met Thr Leu Pro Lys Gly Asp
                   645                 650                 655
   Ile Ser Ile Ser Gly Pro Lys Val Asn Val Glu Ala Pro Asp Val Asn
               660                 665                 670
   Leu Glu Gly Leu Gly Gly Lys Leu Lys Gly Pro Asp Val Lys Leu Pro
           675                 680                 685
   Asp Met Ser Val Lys Thr Pro Lys Ile Ser Met Pro Asp Val Asp Leu
       690                 695                 700
   His Val Lys Gly Thr Lys Val Lys Gly Glu Tyr Asp Val Thr Val Pro
   705                 710                 715                 720
   Lys Leu Glu Gly Glu Leu Lys Gly Pro Lys Val Asp Ile Asp Ala Pro
                   725                 730                 735
   Asp Val Asp Val His Gly Pro Asp Trp His Leu Lys Met Pro Lys Met
               740                 745                 750
   Lys Met Pro Lys Phe Ser Val Pro Gly Phe Lys Ala Glu Gly Pro Glu
           755                 760                 765
   Val Asp Val Asn Leu Pro Lys Ala Asp Val Asp Ile Ser Gly Pro Lys
       770                 775                 780
   Ile Asp Val Thr Ala Pro Asp Val Ser Ile Glu Glu Pro Glu Gly Lys
   785                 790                 795                 800
   Leu Lys Gly Pro Lys Phe Lys Met Pro Glu Met Asn Ile Lys Val Pro
                   805                 810                 815
   Lys Ile Ser Met Pro Asp Val Asp Leu His Leu Lys Gly Pro Asn Val
               820                 825                 830
   Lys Gly Glu Tyr Asp Val Thr Met Pro Lys Val Glu Ser Glu Ile Lys
           835                 840                 845
   Val Pro Asp Val Glu Leu Lys Ser Ala Lys Met Asp Ile Asp Val Pro
       850                 855                 860
   Asp Val Glu Val Gln Gly Pro Asp Trp His Leu Lys Met Pro Lys Met
   865                 870                 875                 880
   Lys Met Pro Lys Phe Ser Met Pro Gly Phe Lys Ala Glu Gly Pro Glu
                   885                 890                 895
   Val Asp Val Asn Leu Pro Lys Ala Asp Val Asp Ile Ser Gly Pro Lys
               900                 905                 910
   Val Gly Val Glu Val Pro Asp Val Asn Ile Glu Gly Pro Glu Gly Lys
           915                 920                 925
   Leu Lys Gly Pro Lys Phe Lys Met Pro Glu Met Asn Ile Lys Ala Pro
       930                 935                 940
   Lys Ile Ser Met Pro Asp Val Asp Leu His Met Lys Gly Pro Lys Val
   945                 950                 955                 960
   Lys Gly Glu Tyr Asp Met Thr Val Pro Lys Leu Glu Gly Asp Leu Lys
                   965                 970                 975
   Gly Pro Lys Val Asp Val Ser Ala Pro Asp Val Glu Met Gln Gly Pro
               980                 985                 990
   Asp Trp Asn Leu Lys Met Pro Lys  Ile Lys Met Pro Lys  Phe Ser Met
           995                 1000                1005
   Pro Ser  Leu Lys Gly Glu Gly  Pro Glu Phe Asp Val  Asn Leu Ser
       1010                1015                1020
   Lys Ala  Asn Val Asp Ile Ser  Ala Pro Lys Val Asp  Thr Asn Ala
       1025                1030                1035
   Pro Asp  Leu Ser Leu Glu Gly  Pro Glu Gly Lys Leu  Lys Gly Pro
       1040                1045                1050
   Lys Phe  Lys Met Pro Glu Met  His Phe Arg Ala Pro  Lys Met Ser
       1055                1060                1065
   Leu Pro  Asp Val Asp Leu Asp  Leu Lys Gly Pro Lys  Met Lys Gly
       1070                1075                1080
   Asn Val  Asp Ile Ser Ala Pro  Lys Ile Glu Gly Glu  Met Gln Val
       1085                1090                1095
   Pro Asp Val Asp Ile Arg Gly Pro Lys Val Asp Ile Lys Ala Pro
   1100 1105 1110
   Asp Val Glu Gly Gln Gly Leu Asp Trp Ser Leu Lys Ile Pro Lys
   1115 1120 1125
   Met Lys Met Pro Lys Phe Ser Met Pro Ser Leu Lys Gly Glu Gly
   1130 1135 1140
   Pro Glu Val Asp Val Asn Leu Pro Lys Ala Asp Val Val Val Ser
   1145 1150 1155
   Gly Pro Lys Val Asp Ile Glu Ala Pro Asp Val Ser Leu Glu Gly
   1160 1165 1170
   Pro Glu Gly Lys Leu Lys Gly Pro Lys Phe Lys Met Pro Glu Met
   1175 1180 1185
   His Phe Lys Thr Pro Lys Ile Ser Met Pro Asp Val Asp Leu His
   1190 1195 1200
   Leu Lys Gly Pro Lys Val Lys Gly Asp Val Asp Val Ser Val Pro
   1205 1210 1215
   Lys Val Glu Gly Glu Met Lys Val Pro Asp Val Glu Ile Lys Gly
   1220 1225 1230
   Pro Lys Met Asp Ile Asp Ala Pro Asp Val Glu Val Gln Gly Pro
   1235 1240 1245
   Asp Trp His Leu Lys Met Pro Lys Met Lys Met Pro Lys Phe Ser
   1250 1255 1260
   Met Pro Gly Phe Lys Gly Glu Gly Arg Glu Val Asp Val Asn Leu
   1265 1270 1275
   Pro Lys Ala Asp Ile Asp Val Ser Gly Pro Lys Val Asp Val Glu
   1280 1285 1290
   Val Pro Asp Val Ser Leu Glu Gly Pro Glu Gly Lys Leu Lys Gly
   1295 1300 1305
   Pro Lys Phe Lys Met Pro Glu Met His Phe Lys Ala Pro Lys Ile
   1310 1315 1320
   Ser Met Pro Asp Val Asp Leu Asn Leu Lys Gly Pro Lys Leu Lys
   1325 1330 1335
   Gly Asp Val Asp Val Ser Leu Pro Glu Val Glu Gly Glu Met Lys
   1340 1345 1350
   Val Pro Asp Val Asp Ile Lys Gly Pro Lys Val Asp Ile Ser Ala
       1355                    1360                    1365
   Pro Asp Val Asp Val His Gly Pro Asp Trp His Leu Lys Met Pro
       1370                    1375                    1380
   Lys Val Lys Met Pro Lys Phe Ser Met Pro Gly Phe Lys Gly Glu
       1385                    1390                    1395
   Gly Pro Glu Val Asp Val Lys Leu Pro Lys Ala Asp Val Asp Val
       1400                    1405                    1410
   Ser Gly Pro Lys Met Asp Ala Glu Val Pro Asp Val Asn Ile Glu
       1415                    1420                    1425
   Gly Pro Asp Ala Lys Leu Lys Gly Pro Lys Phe Lys Met Pro Glu
       1430                    1435                    1440
   Met Ser Ile Lys Pro Gln Lys Ile Ser Ile Pro Asp Val Gly Leu
       1445                    1450                    1455
   His Leu Lys Gly Pro Lys Met Lys Gly Asp Tyr Asp Val Thr Val
       1460                    1465                    1470
   Pro Lys Val Glu Gly Glu Ile Lys Ala Pro Asp Val Asp Ile Lys
       1475                    1480                    1485
   Gly Pro Lys Val Asp Ile Asn Ala Pro Asp Val Glu Val His Gly
       1490                    1495                    1500
   Pro Asp Trp His Leu Lys Met Pro Lys Val Lys Met Pro Lys Phe
       1505                    1510                    1515
   Ser Met Pro Gly Phe Lys Gly Glu Gly Pro Glu Val Asp Met Asn
       1520                    1525                    1530
   Leu Pro Lys Ala Asp Leu Gly Val Ser Gly Pro Lys Val Asp Ile
       1535                    1540                    1545
   Asp Val Pro Asp Val Asn Leu Glu Ala Pro Glu Gly Lys Leu Lys
       1550                    1555                    1560
   Gly Pro Lys Phe Lys Met Pro Ser Met Asn Ile Gln Thr His Lys
       1565                    1570                    1575
   Ile Ser Met Pro Asp Val Gly Leu Asn Leu Lys Ala Pro Lys Leu
       1580                    1585                    1590
   Lys Thr Asp Val Asp Val Ser Leu Pro Lys Val Glu Gly Asp Leu
       1595                    1600                    1605
   Lys Gly Pro Glu Ile Asp Val Lys Ala Pro Lys Met Asp Val Asn
       1610                1615                1620
   Val Gly Asp Ile Asp Ile Glu Gly Pro Glu Gly Lys Leu Lys Gly
       1625                1630                1635
   Pro Lys Phe Lys Met Pro Glu Met His Phe Lys Ala Pro Lys Ile
       1640                1645                1650
   Ser Met Pro Asp Val Asp Leu His Leu Lys Gly Pro Lys Val Lys
       1655                1660                1665
   Gly Asp Met Asp Val Ser Val Pro Lys Val Glu Gly Glu Met Lys
       1670                1675                1680
   Val Pro Asp Val Asp Ile Lys Gly Pro Lys Val Asp Ile Asp Ala
       1685                1690                1695
   Pro Asp Val Glu Val His Asp Pro Asp Trp His Leu Lys Met Pro
       1700                1705                1710
   Lys Met Lys Met Pro Lys Phe Ser Met Pro Gly Phe Lys Ala Glu
       1715                1720                1725
   Gly Pro Glu Val Asp Val Asn Leu Pro Lys Ala Asp Ile Asp Val
       1730                1735                1740
   Ser Gly Pro Ser Val Asp Thr Asp Ala Pro Asp Leu Asp Ile Glu
       1745                1750                1755
   Gly Pro Glu Gly Lys Leu Lys Gly Ser Lys Phe Lys Met Pro Lys
       1760                1765                1770
   Leu Asn Ile Lys Ala Pro Lys Val Ser Met Pro Asp Val Asp Leu
       1775                1780                1785
   Asn Leu Lys Gly Pro Lys Leu Lys Gly Glu Ile Asp Ala Ser Val
       1790                1795                1800
   Pro Glu Leu Glu Gly Asp Leu Arg Gly Pro Gln Val Asp Val Lys
       1805                1810                1815
   Gly Pro Phe Val Glu Ala Glu Val Pro Asp Val Asp Leu Glu Cys
       1820                1825                1830
   Pro Asp Ala Lys Leu Lys Gly Pro Lys Phe Lys Met Pro Glu Met
       1835                1840                1845
   His Phe Lys Ala Pro Lys Ile Ser Met Pro Asp Val Asp Leu His
       1850                1855                1860
   Leu Lys Gly Pro Lys Val Lys Gly Asp Ala Asp Val Ser Val Pro
   1865 1870 1875
   Lys Leu Glu Gly Asp Leu Thr Gly Pro Ser Val Gly Val Glu Val
   1880 1885 1890
   Pro Asp Val Glu Leu Glu Cys Pro Asp Ala Lys Leu Lys Gly Pro
   1895 1900 1905
   Lys Phe Lys Met Pro Asp Met His Phe Lys Ala Pro Lys Ile Ser
   1910 1915 1920
   Met Pro Asp Val Asp Leu His Leu Lys Gly Pro Lys Val Lys Gly
   1925 1930 1935
   Asp Val Asp Val Ser Val Pro Lys Leu Glu Gly Asp Leu Thr Gly
   1940 1945 1950
   Pro Ser Val Gly Val Glu Val Pro Asp Val Glu Leu Glu Cys Pro
   1955 1960 1965
   Asp Ala Lys Leu Lys Gly Pro Lys Phe Lys Met Pro Glu Met His
   1970 1975 1980
   Phe Lys Thr Pro Lys Ile Ser Met Pro Asp Val Asp Leu His Leu
   1985 1990 1995
   Lys Gly Pro Lys Val Lys Gly Asp Met Asp Val Ser Val Pro Lys
   2000 2005 2010
   Val Glu Gly Glu Met Lys Val Pro Asp Val Asp Ile Lys Gly Pro
   2015 2020 2025
   Lys Met Asp Ile Asp Ala Pro Asp Val Asp Val His Gly Pro Asp
   2030 2035 2040
   Trp His Leu Lys Met Pro Lys Met Lys Met Pro Lys Phe Ser Met
   2045 2050 2055
   Pro Gly Phe Lys Ala Glu Gly Pro Glu Val Asp Val Asn Leu Pro
   2060 2065 2070
   Lys Ala Asp Val Val Val Ser Gly Pro Lys Val Asp Val Glu Val
   2075 2080 2085
   Pro Asp Val Ser Leu Glu Gly Pro Glu Gly Lys Leu Lys Gly Pro
   2090 2095 2100
   Lys Leu Lys Met Pro Glu Met His Phe Lys Ala Pro Lys Ile Ser
   2105 2110 2115
   Met Pro Asp Val Asp Leu His Leu Lys Gly Pro Lys Val Lys Gly
   2120 2125 2130
   Asp Val Asp Val Ser Leu Pro Lys Leu Glu Gly Asp Leu Thr Gly
   2135 2140 2145
   Pro Ser Val Asp Val Glu Val Pro Asp Val Glu Leu Glu Cys Pro
   2150 2155 2160
   Asp Ala Lys Leu Lys Gly Pro Lys Phe Lys Met Pro Glu Met His
   2165 2170 2175
   Phe Lys Thr Pro Lys Ile Ser Met Pro Asp Val Asn Leu Asn Leu
   2180 2185 2190
   Lys Gly Pro Lys Val Lys Gly Asp Met Asp Val Ser Val Pro Lys
   2195 2200 2205
   Val Glu Gly Glu Met Lys Val Pro Asp Val Asp Ile Arg Gly Pro
   2210 2215 2220
   Lys Val Asp Ile Asp Ala Pro Asp Val Asp Val His Gly Pro Asp
   2225 2230 2235
   Trp His Leu Lys Met Pro Lys Met Lys Met Pro Lys Phe Ser Met
   2240 2245 2250
   Pro Gly Phe Lys Gly Glu Gly Pro Glu Val Asp Val Asn Leu Pro
   2255 2260 2265
   Lys Ala Asp Val Asp Val Ser Gly Pro Lys Val Asp Val Glu Val
   2270 2275 2280
   Pro Asp Val Ser Leu Glu Gly Pro Glu Gly Lys Leu Lys Gly Pro
   2285 2290 2295
   Lys Phe Lys Met Pro Glu Met His Phe Lys Thr Pro Lys Ile Ser
   2300 2305 2310
   Met Pro Asp Val Asp Phe Asn Leu Lys Gly Pro Lys Ile Lys Gly
   2315 2320 2325
   Asp Val Asp Val Ser Ala Pro Lys Leu Glu Gly Glu Leu Lys Gly
   2330 2335 2340
   Pro Glu Leu Asp Val Lys Gly Pro Lys Leu Asp Ala Asp Met Pro
   2345 2350 2355
   Glu Val Ala Val Glu Gly Pro Asn Gly Lys Trp Lys Thr Pro Lys
   2360 2365 2370
   Phe Lys Met Pro Asp Met His Phe Lys Ala Pro Lys Ile Ser Met
       2375                2380                2385
   Pro Asp Leu Asp Leu His Leu Lys Ser Pro Lys Ala Lys Gly Glu
       2390                2395                2400
   Val Asp Val Asp Val Pro Lys Leu Glu Gly Asp Leu Lys Gly Pro
       2405                2410                2415
   His Val Asp Val Ser Gly Pro Asp Ile Asp Ile Glu Gly Pro Glu
       2420                2425                2430
   Gly Lys Leu Lys Gly Pro Lys Phe Lys Met Pro Asp Met His Phe
       2435                2440                2445
   Lys Ala Pro Asn Ile Ser Met Pro Asp Val Asp Leu Asn Leu Lys
       2450                2455                2460
   Gly Pro Lys Ile Lys Gly Asp Val Asp Val Ser Val Pro Glu Val
       2465                2470                2475
   Glu Gly Lys Leu Glu Val Pro Asp Met Asn Ile Arg Gly Pro Lys
       2480                2485                2490
   Val Asp Val Asn Ala Pro Asp Val Gln Ala Pro Asp Trp His Leu
       2495                2500                2505
   Lys Met Pro Lys Met Lys Met Pro Lys Phe Ser Met Pro Gly Phe
       2510                2515                2520
   Lys Ala Glu Gly Pro Glu Val Asp Val Asn Leu Pro Lys Ala Asp
       2525                2530                2535
   Val Asp Ile Ser Gly Pro Lys Val Asp Ile Glu Gly Pro Asp Val
       2540                2545                2550
   Asn Ile Glu Gly Pro Glu Gly Lys Leu Lys Gly Pro Lys Leu Lys
       2555                2560                2565
   Met Pro Glu Met Asn Ile Lys Ala Pro Lys Ile Ser Met Pro Asp
       2570                2575                2580
   Phe Asp Leu His Leu Lys Gly Pro Lys Val Lys Gly Asp Val Asp
       2585                2590                2595
   Val Ser Leu Pro Lys Val Glu Gly Asp Leu Lys Gly Pro Glu Val
       2600                2605                2610
   Asp Ile Lys Gly Pro Lys Val Asp Ile Asn Ala Pro Asp Val Gly
       2615                2620                2625
   Val Gln Gly Pro Asp Trp His Leu Lys Met Pro Lys Val Lys Met
   2630 2635 2640
   Pro Lys Phe Ser Met Pro Gly Phe Lys Gly Glu Gly Pro Asp Gly
   2645 2650 2655
   Asp Val Lys Leu Pro Lys Ala Asp Ile Asp Val Ser Gly Pro Lys
   2660 2665 2670
   Val Asp Ile Glu Gly Pro Asp Val Asn Ile Glu Gly Pro Glu Gly
   2675 2680 2685
   Lys Leu Lys Gly Pro Lys Phe Lys Met Pro Glu Met Asn Ile Lys
   2690 2695 2700
   Ala Pro Lys Ile Ser Met Pro Asp Ile Asp Leu Asn Leu Lys Gly
   2705 2710 2715
   Pro Lys Val Lys Gly Asp Val Asp Val Ser Leu Pro Lys Val Glu
   2720 2725 2730
   Gly Asp Leu Lys Gly Pro Glu Val Asp Ile Lys Gly Pro Lys Val
   2735 2740 2745
   Asp Ile Asp Ala Pro Asp Val Asp Val His Gly Pro Asp Trp His
   2750 2755 2760
   Leu Lys Met Pro Lys Ile Lys Met Pro Lys Ile Ser Met Pro Gly
   2765 2770 2775
   Phe Lys Gly Glu Gly Pro Asp Val Asp Val Asn Leu Pro Lys Ala
   2780 2785 2790
   Asp Ile Asp Val Ser Gly Pro Lys Val Asp Val Glu Cys Pro Asp
   2795 2800 2805
   Val Asn Ile Glu Gly Pro Glu Gly Lys Trp Lys Ser Pro Lys Phe
   2810 2815 2820
   Lys Met Pro Glu Met His Phe Lys Thr Pro Lys Ile Ser Met Pro
   2825 2830 2835
   Asp Ile Asp Leu Asn Leu Thr Gly Pro Lys Ile Lys Gly Asp Val
   2840 2845 2850
   Asp Val Thr Gly Pro Lys Val Glu Gly Asp Leu Lys Gly Pro Glu
   2855 2860 2865
   Val Asp Leu Lys Gly Pro Lys Val Asp Ile Asp Val Pro Asp Val
   2870 2875 2880
   Asn Val Gln Gly Pro Asp Trp His Leu Lys Met Pro Lys Met Lys
   2885 2890 2895
   Met Pro Lys Phe Ser Met Pro Gly Phe Lys Ala Glu Gly Pro Glu
   2900 2905 2910
   Val Asp Val Asn Leu Pro Lys Ala Asp Val Asp Val Ser Gly Pro
   2915 2920 2925
   Lys Val Asp Val Glu Gly Pro Asp Val Asn Ile Glu Gly Pro Glu
   2930 2935 2940
   Gly Lys Leu Lys Gly Pro Lys Phe Lys Met Pro Glu Met Asn Ile
   2945 2950 2955
   Lys Ala Pro Lys Ile Pro Met Pro Asp Phe Asp Leu His Leu Lys
   2960 2965 2970
   Gly Pro Lys Val Lys Gly Asp Val Asp Ile Ser Leu Pro Lys Val
   2975 2980 2985
   Glu Gly Asp Leu Lys Gly Pro Glu Val Asp Ile Arg Gly Pro Gln
   2990 2995 3000
   Val Asp Ile Asp Val Pro Asp Val Gly Val Gln Gly Pro Asp Trp
   3005 3010 3015
   His Leu Lys Met Pro Lys Val Lys Met Pro Lys Phe Ser Met Pro
   3020 3025 3030
   Gly Phe Lys Gly Glu Gly Pro Asp Val Asp Val Asn Leu Pro Lys
   3035 3040 3045
   Ala Asp Leu Asp Val Ser Gly Pro Lys Val Asp Ile Asp Val Pro
   3050 3055 3060
   Asp Val Asn Ile Glu Gly Pro Glu Gly Lys Leu Lys Gly Pro Lys
   3065 3070 3075
   Phe Lys Met Pro Glu Met Asn Ile Lys Ala Pro Lys Ile Ser Met
   3080 3085 3090
   Pro Asp Ile Asp Leu Asn Leu Lys Gly Pro Lys Val Lys Gly Asp
   3095 3100 3105
   Met Asp Val Ser Leu Pro Lys Val Glu Gly Asp Met Lys Val Pro
   3110 3115 3120
   Asp Val Asp Ile Lys Gly Pro Lys Val Asp Ile Asn Ala Pro Asp
   3125 3130 3135
   Val Asp Val Gln Gly Pro Asp Trp His Leu Lys Met Pro Lys Ile
   3140 3145 3150
   Lys Met Pro Lys Ile Ser Met Pro Gly Phe Lys Gly Glu Gly Pro
   3155 3160 3165
   Glu Val Asp Val Asn Leu Pro Lys Ala Asp Leu Asp Val Ser Gly
   3170 3175 3180
   Pro Lys Val Asp Val Asp Val Pro Asp Val Asn Ile Glu Gly Pro
   3185 3190 3195
   Asp Ala Lys Leu Lys Gly Pro Lys Phe Lys Met Pro Glu Met Asn
   3200 3205 3210
   Ile Lys Ala Pro Lys Ile Ser Met Pro Asp Leu Asp Leu Asn Leu
   3215 3220 3225
   Lys Gly Pro Lys Met Lys Gly Glu Val Asp Val Ser Leu Ala Asn
   3230 3235 3240
   Val Glu Gly Asp Leu Lys Gly Pro Ala Leu Asp Ile Lys Gly Pro
   3245 3250 3255
   Lys Ile Asp Val Asp Ala Pro Asp Ile Asp Ile His Gly Pro Asp
   3260 3265 3270
   Ala Lys Leu Lys Gly Pro Lys Leu Lys Met Pro Asp Met His Val
   3275 3280 3285
   Asn Met Pro Lys Ile Ser Met Pro Glu Ile Asp Leu Asn Leu Lys
   3290 3295 3300
   Gly Ser Lys Leu Lys Gly Asp Val Asp Val Ser Gly Pro Lys Leu
   3305 3310 3315
   Glu Gly Asp Ile Lys Ala Pro Ser Leu Asp Ile Lys Gly Pro Glu
   3320 3325 3330
   Val Asp Val Ser Gly Pro Lys Leu Asn Ile Glu Gly Lys Ser Lys
   3335 3340 3345
   Lys Ser Arg Phe Lys Leu Pro Lys Phe Asn Phe Ser Gly Ser Lys
   3350 3355 3360
   Val Gln Thr Pro Glu Val Asp Val Lys Gly Lys Lys Pro Asp Ile
   3365 3370 3375
   Asp Ile Thr Gly Pro Lys Val Asp Ile Asn Ala Pro Asp Val Glu
   3380 3385 3390
   Val Gln Gly Lys Val Lys Gly Ser Lys Phe Lys Met Pro Phe Leu
   3395 3400 3405
   Ser Ile Ser Ser Pro Lys Val Ser Met Pro Asp Val Glu Leu Asn
   3410 3415 3420
   Leu Lys Ser Pro Lys Val Lys Gly Asp Leu Asp Ile Ala Gly Pro
   3425 3430 3435
   Asn Leu Glu Gly Asp Phe Lys Gly Pro Lys Val Asp Ile Lys Ala
   3440 3445 3450
   Pro Glu Val Asn Leu Asn Ala Pro Asp Val Asp Val His Gly Pro
   3455 3460 3465
   Asp Trp Asn Leu Lys Met Pro Lys Met Lys Met Pro Lys Phe Ser
   3470 3475 3480
   Val Ser Gly Leu Lys Ala Glu Gly Pro Asp Val Ala Val Asp Leu
   3485 3490 3495
   Pro Lys Gly Asp Ile Asn Ile Glu Gly Pro Ser Met Asn Ile Glu
   3500 3505 3510
   Gly Pro Asp Leu Asn Val Glu Gly Pro Glu Gly Gly Leu Lys Gly
   3515 3520 3525
   Pro Lys Phe Lys Met Pro Asp Met Asn Ile Lys Ala Pro Lys Ile
   3530 3535 3540
   Ser Met Pro Asp Ile Asp Leu Asn Leu Lys Gly Pro Lys Val Lys
   3545 3550 3555
   Gly Asp Val Asp Ile Ser Leu Pro Lys Leu Glu Gly Asp Leu Lys
   3560 3565 3570
   Gly Pro Glu Val Asp Ile Lys Gly Pro Lys Val Asp Ile Asn Ala
   3575 3580 3585
   Pro Asp Val Asp Val His Gly Pro Asp Trp His Leu Lys Met Pro
   3590 3595 3600
   Lys Val Lys Met Pro Lys Phe Ser Met Pro Gly Phe Lys Gly Glu
   3605 3610 3615
   Gly Pro Glu Val Asp Val Thr Leu Pro Lys Ala Asp Ile Asp Ile
   3620 3625 3630
   Ser Gly Pro Asn Val Asp Val Asp Val Pro Asp Val Asn Ile Glu
   3635 3640 3645
   Gly Pro Asp Ala Lys Leu Lys Gly Pro Lys Phe Lys Met Pro Glu
   3650 3655 3660
   Met Asn Ile Lys Ala Pro Lys Ile Ser Met Pro Asp Phe Asp Leu
   3665 3670 3675
   Asn Leu Lys Gly Pro Lys Met Lys Gly Asp Val Val Val Ser Leu
   3680 3685 3690
   Pro Lys Val Glu Gly Asp Leu Lys Gly Pro Glu Val Asp Ile Lys
   3695 3700 3705
   Gly Pro Lys Val Asp Ile Asp Thr Pro Asp Ile Asn Ile Glu Gly
   3710 3715 3720
   Ser Glu Gly Lys Phe Lys Gly Pro Lys Phe Lys Ile Pro Glu Met
   3725 3730 3735
   His Leu Lys Ala Pro Lys Ile Ser Met Pro Asp Ile Asp Leu Asn
   3740 3745 3750
   Leu Lys Gly Pro Lys Val Lys Gly Asp Val Asp Val Ser Leu Pro
   3755 3760 3765
   Lys Met Glu Gly Asp Leu Lys Gly Pro Glu Val Asp Ile Lys Gly
   3770 3775 3780
   Pro Lys Val Asp Ile Asn Ala Pro Asp Val Asp Val Gln Gly Pro
   3785 3790 3795
   Asp Trp His Leu Lys Met Pro Lys Val Lys Met Pro Lys Phe Ser
   3800 3805 3810
   Met Pro Gly Phe Lys Gly Glu Gly Pro Asp Val Asp Val Asn Leu
   3815 3820 3825
   Pro Lys Ala Asp Leu Asp Val Ser Gly Pro Lys Val Asp Ile Asp
   3830 3835 3840
   Val Pro Asp Val Asn Ile Glu Gly Pro Glu Gly Lys Leu Lys Gly
   3845 3850 3855
   Pro Lys Phe Lys Met Pro Glu Met Asn Ile Lys Ala Pro Lys Ile
   3860 3865 3870
   Ser Met Pro Asp Ile Asp Leu Asn Leu Lys Gly Pro Lys Val Lys
   3875 3880 3885
   Gly Asp Met Asp Val Ser Leu Pro Lys Val Glu Gly Asp Met Gln
   3890 3895 3900
   Val Pro Asp Leu Asp Ile Lys Gly Pro Lys Val Asp Ile Asn Ala
       3905                3910                3915
   Pro Asp Val Asp Val Arg Gly Pro Asp Trp His Leu Lys Met Pro
       3920                3925                3930
   Lys Ile Lys Met Pro Lys Ile Ser Met Pro Gly Phe Lys Gly Glu
       3935                3940                3945
   Gly Pro Glu Val Asp Val Asn Leu Pro Lys Ala Asp Leu Asp Val
       3950                3955                3960
   Ser Gly Pro Lys Val Asp Val Asp Val Pro Asp Val Asn Ile Glu
       3965                3970                3975
   Gly Pro Asp Ala Lys Leu Lys Gly Pro Lys Phe Lys Met Pro Glu
       3980                3985                3990
   Met Asn Ile Lys Ala Pro Lys Ile Ser Met Pro Asp Phe Asp Leu
       3995                4000                4005
   His Leu Lys Gly Pro Lys Val Lys Gly Asp Val Asp Val Ser Leu
       4010                4015                4020
   Pro Lys Met Glu Gly Asp Leu Lys Ala Pro Glu Val Asp Ile Lys
       4025                4030                4035
   Gly Pro Lys Val Asp Ile Asp Ala Pro Asp Val Asp Val His Gly
       4040                4045                4050
   Pro Asp Trp His Leu Lys Met Pro Lys Val Lys Met Pro Lys Phe
       4055                4060                4065
   Ser Met Pro Gly Phe Lys Gly Glu Gly Pro Glu Val Asp Val Asn
       4070                4075                4080
   Leu Pro Lys Ala Asp Ile Asp Val Ser Gly Pro Lys Val Asp Ile
       4085                4090                4095
   Asp Thr Pro Asp Ile Asp Ile His Gly Pro Glu Gly Lys Leu Lys
       4100                4105                4110
   Gly Pro Lys Phe Lys Met Pro Asp Leu His Leu Lys Ala Pro Lys
       4115                4120                4125
   Ile Ser Met Pro Glu Val Asp Leu Asn Leu Lys Gly Pro Lys Met
       4130                4135                4140
   Lys Gly Asp Val Asp Val Ser Leu Pro Lys Val Glu Gly Asp Leu
       4145                4150                4155
   Lys Gly Pro Glu Val Asp Ile Lys Gly Pro Lys Val Asp Ile Asp
   4160 4165 4170
   Val Pro Asp Val Asp Val Gln Gly Pro Asp Trp His Leu Lys Met
   4175 4180 4185
   Pro Lys Val Lys Met Pro Lys Phe Ser Met Pro Gly Phe Lys Gly
   4190 4195 4200
   Glu Gly Pro Asp Val Asp Val Asn Leu Pro Lys Ala Asp Leu Asp
   4205 4210 4215
   Val Ser Gly Pro Lys Val Asp Ile Asp Val Pro Asp Val Asn Ile
   4220 4225 4230
   Glu Gly Pro Asp Ala Lys Leu Lys Gly Pro Lys Phe Lys Met Pro
   4235 4240 4245
   Glu Met Asn Ile Lys Ala Pro Lys Ile Ser Met Pro Asp Phe Asp
   4250 4255 4260
   Leu His Leu Lys Gly Pro Lys Val Lys Gly Asp Val Asp Val Ser
   4265 4270 4275
   Leu Pro Lys Val Glu Gly Asp Leu Lys Gly Pro Glu Val Asp Ile
   4280 4285 4290
   Lys Gly Pro Lys Val Asp Ile Asp Ala Pro Asp Val Asp Val His
   4295 4300 4305
   Gly Pro Asp Trp His Leu Lys Met Pro Lys Val Lys Met Pro Lys
   4310 4315 4320
   Phe Ser Met Pro Gly Phe Lys Gly Glu Gly Pro Asp Val Asp Val
   4325 4330 4335
   Thr Leu Pro Lys Ala Asp Ile Glu Ile Ser Gly Pro Lys Val Asp
   4340 4345 4350
   Ile Asp Ala Pro Asp Val Ser Ile Glu Gly Pro Asp Ala Lys Leu
   4355 4360 4365
   Lys Gly Pro Lys Phe Lys Met Pro Glu Met Asn Ile Lys Ala Pro
   4370 4375 4380
   Lys Ile Ser Met Pro Asp Ile Asp Phe Asn Leu Lys Gly Pro Lys
   4385 4390 4395
   Val Lys Gly Asp Val Asp Val Ser Leu Pro Lys Val Glu Gly Asp
   4400 4405 4410
   Leu Lys Gly Pro Glu Ile Asp Ile Lys Gly Pro Ser Leu Asp Ile
   4415 4420 4425
   Asp Thr Pro Asp Val Asn Ile Glu Gly Pro Glu Gly Lys Leu Lys
   4430 4435 4440
   Gly Pro Lys Phe Lys Met Pro Glu Met Asn Ile Lys Ala Pro Lys
   4445 4450 4455
   Ile Ser Met Pro Asp Phe Asp Leu His Leu Lys Gly Pro Lys Val
   4460 4465 4470
   Lys Gly Asp Val Asp Val Ser Leu Pro Lys Val Glu Ser Asp Leu
   4475 4480 4485
   Lys Gly Pro Glu Val Asp Ile Glu Gly Pro Glu Gly Lys Leu Lys
   4490 4495 4500
   Gly Pro Lys Phe Lys Met Pro Asp Val His Phe Lys Ser Pro Gln
   4505 4510 4515
   Ile Ser Met Ser Asp Ile Asp Leu Asn Leu Lys Gly Pro Lys Ile
   4520 4525 4530
   Lys Gly Asp Met Asp Ile Ser Val Pro Lys Leu Glu Gly Asp Leu
   4535 4540 4545
   Lys Gly Pro Lys Val Asp Val Lys Gly Pro Lys Val Gly Ile Asp
   4550 4555 4560
   Thr Pro Asp Ile Asp Ile His Gly Pro Glu Gly Lys Leu Lys Gly
   4565 4570 4575
   Pro Lys Phe Lys Met Pro Asp Leu His Leu Lys Ala Pro Lys Ile
   4580 4585 4590
   Ser Met Pro Glu Val Asp Leu Asn Leu Lys Gly Pro Lys Val Lys
   4595 4600 4605
   Gly Asp Met Asp Ile Ser Leu Pro Lys Val Glu Gly Asp Leu Lys
   4610 4615 4620
   Gly Pro Glu Val Asp Ile Arg Asp Pro Lys Val Asp Ile Asp Val
   4625 4630 4635
   Pro Asp Val Asp Val Gln Gly Pro Asp Trp His Leu Lys Met Pro
   4640 4645 4650
   Lys Val Lys Met Pro Lys Phe Ser Met Pro Gly Phe Lys Gly Glu
   4655 4660 4665
   Gly Pro Asp Val Asp Val Asn Leu Pro Lys Ala Asp Ile Asp Val
   4670 4675 4680
   Ser Gly Pro Lys Val Asp Val Asp Val Pro Asp Val Asn Ile Glu
   4685 4690 4695
   Gly Pro Asp Ala Lys Leu Lys Gly Pro Lys Phe Lys Met Pro Glu
   4700 4705 4710
   Met Ser Ile Lys Ala Pro Lys Ile Ser Met Pro Asp Ile Asp Leu
   4715 4720 4725
   Asn Leu Lys Gly Pro Lys Val Lys Gly Asp Val Asp Val Thr Leu
   4730 4735 4740
   Pro Lys Val Glu Gly Asp Leu Lys Gly Pro Glu Ala Asp Ile Lys
   4745 4750 4755
   Gly Pro Lys Val Asp Ile Asn Thr Pro Asp Val Asp Val His Gly
   4760 4765 4770
   Pro Asp Trp His Leu Lys Met Pro Lys Val Lys Met Pro Lys Phe
   4775 4780 4785
   Ser Met Pro Gly Phe Lys Gly Glu Gly Pro Asp Val Asp Val Ser
   4790 4795 4800
   Leu Pro Lys Ala Asp Ile Asp Val Ser Gly Pro Lys Val Asp Val
   4805 4810 4815
   Asp Ile Pro Asp Val Asn Ile Glu Gly Pro Asp Ala Lys Leu Lys
   4820 4825 4830
   Gly Pro Lys Phe Lys Met Pro Glu Ile Asn Ile Lys Ala Pro Lys
   4835 4840 4845
   Ile Ser Ile Pro Asp Val Asp Leu Asp Leu Lys Gly Pro Lys Val
   4850 4855 4860
   Lys Gly Asp Phe Asp Val Ser Val Pro Lys Val Glu Gly Thr Leu
   4865 4870 4875
   Lys Gly Pro Glu Val Asp Leu Lys Gly Pro Arg Leu Asp Phe Glu
   4880 4885 4890
   Gly Pro Asp Ala Lys Leu Ser Gly Pro Ser Leu Lys Met Pro Ser
   4895 4900 4905
   Leu Glu Ile Ser Ala Pro Lys Val Thr Ala Pro Asp Val Asp Leu
   4910 4915 4920
   His Leu Lys Ala Pro Lys Ile Gly Phe Ser Gly Pro Lys Leu Glu
   4925 4930 4935
   Gly Gly Glu Val Asp Leu Lys Gly Pro Lys Val Glu Ala Pro Ser
   4940 4945 4950
   Leu Asp Val His Met Asp Ser Pro Asp Ile Asn Ile Glu Gly Pro
   4955 4960 4965
   Asp Val Lys Ile Pro Lys Phe Lys Lys Pro Lys Phe Gly Phe Gly
   4970 4975 4980
   Ala Lys Ser Pro Lys Ala Asp Ile Lys Ser Pro Ser Leu Asp Val
   4985 4990 4995
   Thr Val Pro Glu Ala Glu Leu Asn Leu Glu Thr Pro Glu Ile Ser
   5000 5005 5010
   Val Gly Gly Lys Gly Lys Lys Ser Lys Phe Lys Met Pro Lys Ile
   5015 5020 5025
   His Met Ser Gly Pro Lys Ile Lys Ala Lys Lys Gln Gly Phe Asp
   5030 5035 5040
   Leu Asn Val Pro Gly Gly Glu Ile Asp Ala Ser Leu Lys Ala Pro
   5045 5050 5055
   Asp Val Asp Val Asn Ile Ala Gly Pro Asp Ala Ala Leu Lys Val
   5060 5065 5070
   Asp Val Lys Ser Pro Lys Thr Lys Lys Thr Met Phe Gly Lys Met
   5075 5080 5085
   Tyr Phe Pro Asp Val Glu Phe Asp Ile Lys Ser Pro Lys Phe Lys
   5090 5095 5100
   Ala Glu Ala Pro Leu Pro Ser Pro Lys Leu Glu Gly Glu Leu Gln
   5105 5110 5115
   Ala Pro Asp Leu Glu Leu Ser Leu Pro Ala Ile His Val Glu Gly
   5120 5125 5130
   Leu Asp Ile Lys Ala Lys Ala Pro Lys Val Lys Met Pro Asp Val
   5135 5140 5145
   Asp Ile Ser Val Pro Lys Ile Glu Gly Asp Leu Lys Gly Pro Lys
   5150 5155 5160
   Val Gln Ala Asn Leu Gly Ala Pro Asp Ile Asn Ile Glu Gly Leu
   5165 5170 5175
   Asp Ala Lys Val Lys Thr Pro Ser Phe Gly Ile Ser Ala Pro Gln
   5180 5185 5190
   Val Ser Ile Pro Asp Val Asn Val Asn Leu Lys Gly Pro Lys Ile
   5195 5200 5205
   Lys Gly Asp Val Pro Ser Val Gly Leu Glu Gly Pro Asp Val Asp
   5210 5215 5220
   Leu Gln Gly Pro Glu Ala Lys Ile Lys Phe Pro Lys Phe Ser Met
   5225 5230 5235
   Pro Lys Ile Gly Ile Pro Gly Val Lys Met Glu Gly Gly Gly Ala
   5240 5245 5250
   Glu Val His Ala Gln Leu Pro Ser Leu Glu Gly Asp Leu Arg Gly
   5255 5260 5265
   Pro Asp Val Lys Leu Glu Gly Pro Asp Val Ser Leu Lys Gly Pro
   5270 5275 5280
   Gly Val Asp Leu Pro Ser Val Asn Leu Ser Met Pro Lys Val Ser
   5285 5290 5295
   Gly Pro Asp Leu Asp Leu Asn Leu Lys Gly Pro Ser Leu Lys Gly
   5300 5305 5310
   Asp Leu Asp Ala Ser Val Pro Ser Met Lys Val His Ala Pro Gly
   5315 5320 5325
   Leu Asn Leu Ser Gly Val Gly Gly Lys Met Gln Val Gly Gly Asp
   5330 5335 5340
   Gly Val Lys Val Pro Gly Ile Asp Ala Thr Thr Lys Leu Asn Val
   5345 5350 5355
   Gly Ala Pro Asp Val Thr Leu Arg Gly Pro Ser Leu Gln Gly Asp
   5360 5365 5370
   Leu Ala Val Ser Gly Asp Ile Lys Cys Pro Lys Val Ser Val Gly
   5375 5380 5385
   Ala Pro Asp Leu Ser Leu Glu Ala Ser Glu Gly Ser Ile Lys Leu
   5390 5395 5400
   Pro Lys Met Lys Leu Pro Gln Phe Gly Ile Ser Thr Pro Gly Ser
   5405 5410 5415
   Asp Leu His Val Asn Ala Lys Gly Pro Gln Val Ser Gly Glu Leu
   5420 5425 5430
   Lys Gly Pro Gly Val Asp Val Asn Leu Lys Gly Pro Arg Ile Ser
   5435 5440 5445
   Ala Pro Asn Val Asp Phe Asn Leu Glu Gly Pro Lys Val Lys Gly
   5450 5455 5460
   Ser Leu Gly Ala Thr Gly Glu Ile Lys Gly Pro Thr Val Gly Gly
   5465 5470 5475
   Gly Leu Pro Gly Ile Gly Val Gln Gly Leu Glu Gly Asn Leu Gln
   5480 5485 5490
   Met Pro Gly Ile Lys Ser Ser Gly Cys Asp Val Asn Leu Pro Gly
   5495 5500 5505
   Val Asn Val Lys Leu Pro Thr Gly Gln Ile Ser Gly Pro Glu Ile
   5510 5515 5520
   Lys Gly Gly Leu Lys Gly Ser Glu Val Gly Phe His Gly Ala Ala
   5525 5530 5535
   Pro Asp Ile Ser Val Lys Gly Pro Ala Phe Asn Met Ala Ser Pro
   5540 5545 5550
   Glu Ser Asp Phe Gly Ile Asn Leu Lys Gly Pro Lys Ile Lys Gly
   5555 5560 5565
   Gly Ala Asp Val Ser Gly Gly Val Ser Ala Pro Asp Ile Ser Leu
   5570 5575 5580
   Gly Glu Gly His Leu Ser Val Lys Gly Ser Gly Gly Glu Trp Lys
   5585 5590 5595
   Gly Pro Gln Val Ser Ser Ala Leu Asn Leu Asp Thr Ser Lys Phe
   5600 5605 5610
   Ala Gly Gly Leu His Phe Ser Gly Pro Lys Val Glu Gly Gly Val
   5615 5620 5625
   Lys Gly Gly Gln Ile Gly Leu Gln Ala Pro Gly Leu Ser Val Ser
   5630 5635 5640
   Gly Pro Gln Gly His Leu Glu Ser Gly Ser Gly Lys Val Thr Phe
   5645 5650 5655
   Pro Lys Met Lys Ile Pro Lys Phe Thr Phe Ser Gly Arg Glu Leu
   5660 5665 5670
   Val Gly Arg Glu Met Gly Val Asp Val His Phe Pro Lys Ala Glu
   5675 5680 5685
   Ala Ser Ile Gln Ala Gly Ala Gly Asp Gly Glu Trp Glu Glu Ser
   5690 5695 5700
   Glu Val Lys Leu Lys Lys Ser Lys Ile Lys Met Pro Lys Phe Asn
   5705 5710 5715
   Phe Ser Lys Pro Lys Gly Lys Gly Gly Val Thr Gly Ser Pro Glu
   5720 5725 5730
   Ala Ser Ile Ser Gly Ser Lys Gly Asp Leu Lys Ser Ser Lys Ala
   5735 5740 5745
   Ser Leu Gly Ser Leu Glu Gly Glu Ala Glu Ala Glu Ala Ser Ser
   5750 5755 5760
   Pro Lys Gly Lys Phe Ser Leu Phe Lys Ser Lys Lys Pro Arg His
   5765 5770 5775
   Arg Ser Asn Ser Phe Ser Asp Glu Arg Glu Phe Ser Gly Pro Ser
   5780 5785 5790
   Thr Pro Thr Gly Thr Leu Glu Phe Glu Gly Gly Glu Val Ser Leu
   5795 5800 5805
   Glu Gly Gly Lys Val Lys Gly Lys His Gly Lys Leu Lys Phe Gly
   5810 5815 5820
   Thr Phe Gly Gly Leu Gly Ser Lys Ser Lys Gly His Tyr Glu Val
   5825 5830 5835
   Thr Gly Ser Asp Asp Glu Thr Gly Lys Leu Gln Gly Ser Gly Val
   5840 5845 5850
   Ser Leu Ala Ser Lys Lys Ser Arg Leu Ser Ser Ser Ser Ser Asn
   5855 5860 5865
   Asp Ser Gly Asn Lys Val Gly Ile Gln Leu Pro Glu Val Glu Leu
   5870 5875 5880
   Ser Val Ser Thr Lys Lys Glu
   5885 5890
<210> 26
   <211> 2468
   <212> PRT
   <213> Homo sapiens
<400> 26
   Met Ala Thr Val Val Val Glu Ala Thr Glu Pro Glu Pro Ser Gly Ser
   1 5 10 15
   Ile Ala Asn Pro Ala Ala Ser Thr Ser Pro Ser Leu Ser His Arg Phe
   20 25 30
   Leu Asp Ser Lys Phe Tyr Leu Leu Val Val Val Gly Glu Ile Val Thr
   35 40 45
   Glu Glu His Leu Arg Arg Ala Ile Gly Asn Ile Glu Leu Gly Ile Arg
   50 55 60
   Ser Trp Asp Thr Asn Leu Ile Glu Cys Asn Leu Asp Gln Glu Leu Lys
   65 70 75 80
   Leu Phe Val Ser Arg His Ser Ala Arg Phe Ser Pro Glu Val Pro Gly
   85 90 95
   Gln Lys Ile Leu His His Arg Ser Asp Val Leu Glu Thr Val Val Leu
   100 105 110
   Ile Asn Pro Ser Asp Glu Ala Val Ser Thr Glu Val Arg Leu Met Ile
   115 120 125
   Thr Asp Ala Ala Arg His Lys Leu Leu Val Leu Thr Gly Gln Cys Phe
   130 135 140
   Glu Asn Thr Gly Glu Leu Ile Leu Gln Ser Gly Ser Phe Ser Phe Gln
   145 150 155 160
   Asn Phe Ile Glu Ile Phe Thr Asp Gln Glu Ile Gly Glu Leu Leu Ser
   165 170 175
   Thr Thr His Pro Ala Asn Lys Ala Ser Leu Thr Leu Phe Cys Pro Glu
   180 185 190
   Glu Gly Asp Trp Lys Asn Ser Asn Leu Asp Arg His Asn Leu Gln Asp
   195 200 205
   Phe Ile Asn Ile Lys Leu Asn Ser Ala Ser Ile Leu Pro Glu Met Glu
   210 215 220
   Gly Leu Ser Glu Phe Thr Glu Tyr Leu Ser Glu Ser Val Glu Val Pro
   225 230 235 240
   Ser Pro Phe Asp Ile Leu Glu Pro Pro Thr Ser Gly Gly Phe Leu Lys
   245 250 255
   Leu Ser Lys Pro Cys Cys Tyr Ile Phe Pro Gly Gly Arg Gly Asp Ser
   260 265 270
   Ala Leu Phe Ala Val Asn Gly Phe Asn Met Leu Ile Asn Gly Gly Ser
   275 280 285
   Glu Arg Lys Ser Cys Phe Trp Lys Leu Ile Arg His Leu Asp Arg Val
       290                 295                 300
   Asp Ser Ile Leu Leu Thr His Ile Gly Asp Asp Asn Leu Pro Gly Ile
   305                 310                 315                 320
   Asn Ser Met Leu Gln Arg Lys Ile Ala Glu Leu Glu Glu Glu Gln Ser
                   325                 330                 335
   Gln Gly Ser Thr Thr Asn Ser Asp Trp Met Lys Asn Leu Ile Ser Pro
               340                 345                 350
   Asp Leu Gly Val Val Phe Leu Asn Val Pro Glu Asn Leu Lys Asn Pro
           355                 360                 365
   Glu Pro Asn Ile Lys Met Lys Arg Ser Ile Glu Glu Ala Cys Phe Thr
       370                 375                 380
   Leu Gln Tyr Leu Asn Lys Leu Ser Met Lys Pro Glu Pro Leu Phe Arg
   385                 390                 395                 400
   Ser Val Gly Asn Thr Ile Asp Pro Val Ile Leu Phe Gln Lys Met Gly
                   405                 410                 415
   Val Gly Lys Leu Glu Met Tyr Val Leu Asn Pro Val Lys Ser Ser Lys
               420                 425                 430
   Glu Met Gln Tyr Phe Met Gln Gln Trp Thr Gly Thr Asn Lys Asp Lys
           435                 440                 445
   Ala Glu Phe Ile Leu Pro Asn Gly Gln Glu Val Asp Leu Pro Ile Ser
       450                 455                 460
   Tyr Leu Thr Ser Val Ser Ser Leu Ile Val Trp His Pro Ala Asn Pro
   465                 470                 475                 480
   Ala Glu Lys Ile Ile Arg Val Leu Phe Pro Gly Asn Ser Thr Gln Tyr
                   485                 490                 495
   Asn Ile Leu Glu Gly Leu Glu Lys Leu Lys His Leu Asp Phe Leu Lys
               500                 505                 510
   Gln Pro Leu Ala Thr Gln Lys Asp Leu Thr Gly Gln Val Pro Thr Pro
           515                 520                 525
   Val Val Lys Gln Thr Lys Leu Lys Gln Arg Ala Asp Ser Arg Glu Ser
       530                 535                 540
   Leu Lys Pro Ala Ala Lys Pro Leu Pro Ser Lys Ser Val Arg Lys Glu
   545                 550                 555                 560
   Ser Lys Glu Glu Thr Pro Glu Val Thr Lys Val Asn His Val Glu Lys
                   565                 570                 575
   Pro Pro Lys Val Glu Ser Lys Glu Lys Val Met Val Lys Lys Asp Lys
               580                 585                 590
   Pro Ile Lys Thr Glu Thr Lys Pro Ser Val Thr Glu Lys Glu Val Pro
           595                 600                 605
   Ser Lys Glu Glu Pro Ser Pro Val Lys Ala Glu Val Ala Glu Lys Gln
       610                 615                 620
   Ala Thr Asp Val Lys Pro Lys Ala Ala Lys Glu Lys Thr Val Lys Lys
   625                 630                 635                 640
   Glu Thr Lys Val Lys Pro Glu Asp Lys Lys Glu Glu Lys Glu Lys Pro
                   645                 650                 655
   Lys Lys Glu Val Ala Lys Lys Glu Asp Lys Thr Pro Ile Lys Lys Glu
               660                 665                 670
   Glu Lys Pro Lys Lys Glu Glu Val Lys Lys Glu Val Lys Lys Glu Ile
           675                 680                 685
   Lys Lys Glu Glu Lys Lys Glu Pro Lys Lys Glu Val Lys Lys Glu Thr
       690                 695                 700
   Pro Pro Lys Glu Val Lys Lys Glu Val Lys Lys Glu Glu Lys Lys Glu
   705                 710                 715                 720
   Val Lys Lys Glu Glu Lys Glu Pro Lys Lys Glu Ile Lys Lys Leu Pro
                   725                 730                 735
   Lys Asp Ala Lys Lys Ser Ser Thr Pro Leu Ser Glu Ala Lys Lys Pro
               740                 745                 750
   Ala Ala Leu Lys Pro Lys Val Pro Lys Lys Glu Glu Ser Val Lys Lys
           755                 760                 765
   Asp Ser Val Ala Ala Gly Lys Pro Lys Glu Lys Gly Lys Ile Lys Val
       770                 775                 780
   Ile Lys Lys Glu Gly Lys Ala Ala Glu Ala Val Ala Ala Ala Val Gly
   785                 790                 795                 800
   Thr Gly Ala Thr Thr Ala Ala Val Met Ala Ala Ala Gly Ile Ala Ala
                   805                 810                 815
   Ile Gly Pro Ala Lys Glu Leu Glu Ala Glu Arg Ser Leu Met Ser Ser
               820                 825                 830
   Pro Glu Asp Leu Thr Lys Asp Phe Glu Glu Leu Lys Ala Glu Glu Val
           835                 840                 845
   Asp Val Thr Lys Asp Ile Lys Pro Gln Leu Glu Leu Ile Glu Asp Glu
       850                 855                 860
   Glu Lys Leu Lys Glu Thr Glu Pro Val Glu Ala Tyr Val Ile Gln Lys
   865                 870                 875                 880
   Glu Arg Glu Val Thr Lys Gly Pro Ala Glu Ser Pro Asp Glu Gly Ile
                   885                 890                 895
   Thr Thr Thr Glu Gly Glu Gly Glu Cys Glu Gln Thr Pro Glu Glu Leu
               900                 905                 910
   Glu Pro Val Glu Lys Gln Gly Val Asp Asp Ile Glu Lys Phe Glu Asp
           915                 920                 925
   Glu Gly Ala Gly Phe Glu Glu Ser Ser Glu Thr Gly Asp Tyr Glu Glu
       930                 935                 940
   Lys Ala Glu Thr Glu Glu Ala Glu Glu Pro Glu Glu Asp Gly Glu Glu
   945                 950                 955                 960
   His Val Cys Val Ser Ala Ser Lys His Ser Pro Thr Glu Asp Glu Glu
                   965                 970                 975
   Ser Ala Lys Ala Glu Ala Asp Ala Tyr Ile Arg Glu Lys Arg Glu Ser
               980                 985                 990
   Val Ala Ser Gly Asp Asp Arg Ala  Glu Glu Asp Met Asp  Glu Ala Ile
           995                 1000                 1005
   Glu Lys  Gly Glu Ala Glu Gln  Ser Glu Glu Glu Ala  Asp Glu Glu
       1010                 1015                 1020
   Asp Lys  Ala Glu Asp Ala Arg  Glu Glu Glu Tyr Glu  Pro Glu Lys
       1025                 1030                 1035
   Met Glu  Ala Glu Asp Tyr Val  Met Ala Val Val Asp  Lys Ala Ala
       1040                 1045                 1050
   Glu Ala  Gly Gly Ala Glu Glu  Gln Tyr Gly Phe Leu  Thr Thr Pro
       1055                 1060                 1065
   Thr Lys  Gln Leu Gly Ala Gln  Ser Pro Gly Arg Glu  Pro Ala Ser
       1070                 1075                 1080
   Ser Ile  His Asp Glu Thr Leu  Pro Gly Gly Ser Glu  Ser Glu Ala
       1085                 1090                 1095
   Thr Ala Ser Asp Glu Glu Asn Arg Glu Asp Gln Pro Glu Glu Phe
   1100 1105 1110
   Thr Ala Thr Ser Gly Tyr Thr Gln Ser Thr Ile Glu Ile Ser Ser
   1115 1120 1125
   Glu Pro Thr Pro Met Asp Glu Met Ser Thr Pro Arg Asp Val Met
   1130 1135 1140
   Ser Asp Glu Thr Asn Asn Glu Glu Thr Glu Ser Pro Ser Gln Glu
   1145 1150 1155
   Phe Val Asn Ile Thr Lys Tyr Glu Ser Ser Leu Tyr Ser Gln Glu
   1160 1165 1170
   Tyr Ser Lys Pro Ala Asp Val Thr Pro Leu Asn Gly Phe Ser Glu
   1175 1180 1185
   Gly Ser Lys Thr Asp Ala Thr Asp Gly Lys Asp Tyr Asn Ala Ser
   1190 1195 1200
   Ala Ser Thr Ile Ser Pro Pro Ser Ser Met Glu Glu Asp Lys Phe
   1205 1210 1215
   Ser Arg Ser Ala Leu Arg Asp Ala Tyr Cys Ser Glu Val Lys Ala
   1220 1225 1230
   Ser Thr Thr Leu Asp Ile Lys Asp Ser Ile Ser Ala Val Ser Ser
   1235 1240 1245
   Glu Lys Val Ser Pro Ser Lys Ser Pro Ser Leu Ser Pro Ser Pro
   1250 1255 1260
   Pro Ser Pro Leu Glu Lys Thr Pro Leu Gly Glu Arg Ser Val Asn
   1265 1270 1275
   Phe Ser Leu Thr Pro Asn Glu Ile Lys Val Ser Ala Glu Ala Glu
   1280 1285 1290
   Val Ala Pro Val Ser Pro Glu Val Thr Gln Glu Val Val Glu Glu
   1295 1300 1305
   His Cys Ala Ser Pro Glu Asp Lys Thr Leu Glu Val Val Ser Pro
   1310 1315 1320
   Ser Gln Ser Val Thr Gly Ser Ala Gly His Thr Pro Tyr Tyr Gln
   1325 1330 1335
   Ser Pro Thr Asp Glu Lys Ser Ser His Leu Pro Thr Glu Val Ile
   1340 1345 1350
   Glu Lys Pro Pro Ala Val Pro Val Ser Phe Glu Phe Ser Asp Ala
   1355 1360 1365
   Lys Asp Glu Asn Glu Arg Ala Ser Val Ser Pro Met Asp Glu Pro
   1370 1375 1380
   Val Pro Asp Ser Glu Ser Pro Ile Glu Lys Val Leu Ser Pro Leu
   1385 1390 1395
   Arg Ser Pro Pro Leu Ile Gly Ser Glu Ser Ala Tyr Glu Ser Phe
   1400 1405 1410
   Leu Ser Ala Asp Asp Lys Ala Ser Gly Arg Gly Ala Glu Ser Pro
   1415 1420 1425
   Phe Glu Glu Lys Ser Gly Lys Gln Gly Ser Pro Asp Gln Val Ser
   1430 1435 1440
   Pro Val Ser Glu Met Thr Ser Thr Ser Leu Tyr Gln Asp Lys Gln
   1445 1450 1455
   Glu Gly Lys Ser Thr Asp Phe Ala Pro Ile Lys Glu Asp Phe Gly
   1460 1465 1470
   Gln Glu Lys Lys Thr Asp Asp Val Glu Ala Met Ser Ser Gln Pro
   1475 1480 1485
   Ala Leu Ala Leu Asp Glu Arg Lys Leu Gly Asp Val Ser Pro Thr
   1490 1495 1500
   Gln Ile Asp Val Ser Gln Phe Gly Ser Phe Lys Glu Asp Thr Lys
   1505 1510 1515
   Met Ser Ile Ser Glu Gly Thr Val Ser Asp Lys Ser Ala Thr Pro
   1520 1525 1530
   Val Asp Glu Gly Val Ala Glu Asp Thr Tyr Ser His Met Glu Gly
   1535 1540 1545
   Val Ala Ser Val Ser Thr Ala Ser Val Ala Thr Ser Ser Phe Pro
   1550 1555 1560
   Glu Pro Thr Thr Asp Asp Val Ser Pro Ser Leu His Ala Glu Val
   1565 1570 1575
   Gly Ser Pro His Ser Thr Glu Val Asp Asp Ser Leu Ser Val Ser
   1580 1585 1590
   Val Val Gln Thr Pro Thr Thr Phe Gln Glu Thr Glu Met Ser Pro
   1595 1600 1605
   Ser Lys Glu Glu Cys Pro Arg Pro Met Ser Ile Ser Pro Pro Asp
   1610 1615 1620
   Phe Ser Pro Lys Thr Ala Lys Ser Arg Thr Pro Val Gln Asp His
   1625 1630 1635
   Arg Ser Glu Gln Ser Ser Met Ser Ile Glu Phe Gly Gln Glu Ser
   1640 1645 1650
   Pro Glu Gln Ser Leu Ala Met Asp Phe Ser Arg Gln Ser Pro Asp
   1655 1660 1665
   His Pro Thr Val Gly Ala Gly Val Leu His Ile Thr Glu Asn Gly
   1670 1675 1680
   Pro Thr Glu Val Asp Tyr Ser Pro Ser Asp Met Gln Asp Ser Ser
   1685 1690 1695
   Leu Ser His Lys Ile Pro Pro Met Glu Glu Pro Ser Tyr Thr Gln
   1700 1705 1710
   Asp Asn Asp Leu Ser Glu Leu Ile Ser Val Ser Gln Val Glu Ala
   1715 1720 1725
   Ser Pro Ser Thr Ser Ser Ala His Thr Pro Ser Gln Ile Ala Ser
   1730 1735 1740
   Pro Leu Gln Glu Asp Thr Leu Ser Asp Val Ala Pro Pro Arg Asp
   1745 1750 1755
   Met Ser Leu Tyr Ala Ser Leu Thr Ser Glu Lys Val Gln Ser Leu
   1760 1765 1770
   Glu Gly Glu Lys Leu Ser Pro Lys Ser Asp Ile Ser Pro Leu Thr
   1775 1780 1785
   Pro Arg Glu Ser Ser Pro Leu Tyr Ser Pro Thr Phe Ser Asp Ser
   1790 1795 1800
   Thr Ser Ala Val Lys Glu Lys Thr Ala Thr Cys His Ser Ser Ser
   1805 1810 1815
   Ser Pro Pro Ile Asp Ala Ala Ser Ala Glu Pro Tyr Gly Phe Arg
   1820 1825 1830
   Ala Ser Val Leu Phe Asp Thr Met Gln His His Leu Ala Leu Asn
   1835 1840 1845
   Arg Asp Leu Ser Thr Pro Gly Leu Glu Lys Asp Ser Gly Gly Lys
   1850 1855 1860
   Thr Pro Gly Asp Phe Ser Tyr Ala Tyr Gln Lys Pro Glu Glu Thr
   1865 1870 1875
   Thr Arg Ser Pro Asp Glu Glu Asp Tyr Asp Tyr Glu Ser Tyr Glu
   1880 1885 1890
   Lys Thr Thr Arg Thr Ser Asp Val Gly Gly Tyr Tyr Tyr Glu Lys
   1895 1900 1905
   Ile Glu Arg Thr Thr Lys Ser Pro Ser Asp Ser Gly Tyr Ser Tyr
   1910 1915 1920
   Glu Thr Ile Gly Lys Thr Thr Lys Thr Pro Glu Asp Gly Asp Tyr
   1925 1930 1935
   Ser Tyr Glu Ile Ile Glu Lys Thr Thr Arg Thr Pro Glu Glu Gly
   1940 1945 1950
   Gly Tyr Ser Tyr Asp Ile Ser Glu Lys Thr Thr Ser Pro Pro Glu
   1955 1960 1965
   Val Ser Gly Tyr Ser Tyr Glu Lys Thr Glu Arg Ser Arg Arg Leu
   1970 1975 1980
   Leu Asp Asp Ile Ser Asn Gly Tyr Asp Asp Ser Glu Asp Gly Gly
   1985 1990 1995
   His Thr Leu Gly Asp Pro Ser Tyr Ser Tyr Glu Thr Thr Glu Lys
   2000 2005 2010
   Ile Thr Ser Phe Pro Glu Ser Glu Gly Tyr Ser Tyr Glu Thr Ser
   2015 2020 2025
   Thr Lys Thr Thr Arg Thr Pro Asp Thr Ser Thr Tyr Cys Tyr Glu
   2030 2035 2040
   Thr Ala Glu Lys Ile Thr Arg Thr Pro Gln Ala Ser Thr Tyr Ser
   2045 2050 2055
   Tyr Glu Thr Ser Asp Leu Cys Tyr Thr Ala Glu Lys Lys Ser Pro
   2060 2065 2070
   Ser Glu Ala Arg Gln Asp Val Asp Leu Cys Leu Val Ser Ser Cys
   2075 2080 2085
   Glu Tyr Lys His Pro Lys Thr Glu Leu Ser Pro Ser Phe Ile Asn
   2090 2095 2100
   Pro Asn Pro Leu Glu Trp Phe Ala Ser Glu Glu Pro Thr Glu Glu
   2105 2110 2115
   Ser Glu Lys Pro Leu Thr Gln Ser Gly Gly Ala Pro Pro Pro Pro
   2120 2125 2130
   Gly Gly Lys Gln Gln Gly Arg Gln Cys Asp Glu Thr Pro Pro Thr
   2135 2140 2145
   Ser Val Ser Glu Ser Ala Pro Ser Gln Thr Asp Ser Asp Val Pro
   2150 2155 2160
   Pro Glu Thr Glu Glu Cys Pro Ser Ile Thr Ala Asp Ala Asn Ile
   2165 2170 2175
   Asp Ser Glu Asp Glu Ser Glu Thr Ile Pro Thr Asp Lys Thr Val
   2180 2185 2190
   Thr Tyr Lys His Met Asp Pro Pro Pro Ala Pro Val Gln Asp Arg
   2195 2200 2205
   Ser Pro Ser Pro Arg His Pro Asp Val Ser Met Val Asp Pro Glu
   2210 2215 2220
   Ala Leu Ala Ile Glu Gln Asn Leu Gly Lys Ala Leu Lys Lys Asp
   2225 2230 2235
   Leu Lys Glu Lys Thr Lys Thr Lys Lys Pro Gly Thr Lys Thr Lys
   2240 2245 2250
   Ser Ser Ser Pro Val Lys Lys Ser Asp Gly Lys Ser Lys Pro Leu
   2255 2260 2265
   Ala Ala Ser Pro Lys Pro Ala Gly Leu Lys Glu Ser Ser Asp Lys
   2270 2275 2280
   Val Ser Arg Val Ala Ser Pro Lys Lys Lys Glu Ser Val Glu Lys
   2285 2290 2295
   Ala Ala Lys Pro Thr Thr Thr Pro Glu Val Lys Ala Ala Arg Gly
   2300 2305 2310
   Glu Glu Lys Asp Lys Glu Thr Lys Asn Ala Ala Asn Ala Ser Ala
   2315 2320 2325
   Ser Lys Ser Ala Lys Thr Ala Thr Ala Gly Pro Gly Thr Thr Lys
   2330 2335 2340
   Thr Thr Lys Ser Ser Ala Val Pro Pro Gly Leu Pro Val Tyr Leu
   2345 2350 2355
   Asp Leu Cys Tyr Ile Pro Asn His Ser Asn Ser Lys Asn Val Asp
   2360 2365 2370
   Val Glu Phe Phe Lys Arg Val Arg Ser Ser Tyr Tyr Val Val Ser
   2375 2380 2385
   Gly Asn Asp Pro Ala Ala Glu Glu Pro Ser Arg Ala Val Leu Asp
   2390 2395 2400
   Ala Leu Leu Glu Gly Lys Ala Gln Trp Gly Ser Asn Met Gln Val
   2405 2410 2415
   Thr Leu Ile Pro Thr His Asp Ser Glu Val Met Arg Glu Trp Tyr
   2420 2425 2430
   Gln Glu Thr His Glu Lys Gln Gln Asp Leu Asn Ile Met Val Leu
   2435 2440 2445
   Ala Ser Ser Ser Thr Val Val Met Gln Asp Glu Ser Phe Pro Ala
   2450 2455 2460
   Cys Lys Ile Glu Leu
   2465
<210> 27
   <211> 2511
   <212> PRT
   <213> Homo sapiens
<400> 27
   Met Glu Glu Val Val Ile Ala Gly Met Ser Gly Lys Leu Pro Glu Ser
   1 5 10 15
   Glu Asn Leu Gln Glu Phe Trp Asp Asn Leu Ile Gly Gly Val Asp Met
   20 25 30
   Val Thr Asp Asp Asp Arg Arg Trp Lys Ala Gly Leu Tyr Gly Leu Pro
   35 40 45
   Arg Arg Ser Gly Lys Leu Lys Asp Leu Ser Arg Phe Asp Ala Ser Phe
   50 55 60
   Phe Gly Val His Pro Lys Gln Ala His Thr Met Asp Pro Gln Leu Arg
   65 70 75 80
   Leu Leu Leu Glu Val Thr Tyr Glu Ala Ile Val Asp Gly Gly Ile Asn
   85 90 95
   Pro Asp Ser Leu Arg Gly Thr His Thr Gly Val Trp Val Gly Val Ser
   100 105 110
   Gly Ser Glu Thr Ser Glu Ala Leu Ser Arg Asp Pro Glu Thr Leu Val
   115 120 125
   Gly Tyr Ser Met Val Gly Cys Gln Arg Ala Met Met Ala Asn Arg Leu
   130 135 140
   Ser Phe Phe Phe Asp Phe Arg Gly Pro Ser Ile Ala Leu Asp Thr Ala
   145 150 155 160
   Cys Ser Ser Ser Leu Met Ala Leu Gln Asn Ala Tyr Gln Ala Ile His
   165 170 175
   Ser Gly Gln Cys Pro Ala Ala Ile Val Gly Gly Ile Asn Val Leu Leu
   180 185 190
   Lys Pro Asn Thr Ser Val Gln Phe Leu Arg Leu Gly Met Leu Ser Pro
   195 200 205
   Glu Gly Thr Cys Lys Ala Phe Asp Thr Ala Gly Asn Gly Tyr Cys Arg
   210 215 220
   Ser Glu Gly Val Val Ala Val Leu Leu Thr Lys Lys Ser Leu Ala Arg
   225 230 235 240
   Arg Val Tyr Ala Thr Ile Leu Asn Ala Gly Thr Asn Thr Asp Gly Phe
   245 250 255
   Lys Glu Gln Gly Val Thr Phe Pro Ser Gly Asp Ile Gln Glu Gln Leu
   260 265 270
   Ile Arg Ser Leu Tyr Gln Ser Ala Gly Val Ala Pro Glu Ser Phe Glu
   275 280 285
   Tyr Ile Glu Ala His Gly Thr Gly Thr Lys Val Gly Asp Pro Gln Glu
   290 295 300
   Leu Asn Gly Ile Thr Arg Ala Leu Cys Ala Thr Arg Gln Glu Pro Leu
   305 310 315 320
   Leu Ile Gly Ser Thr Lys Ser Asn Met Gly His Pro Glu Pro Ala Ser
   325 330 335
   Gly Leu Ala Ala Leu Ala Lys Val Leu Leu Ser Leu Glu His Gly Leu
   340 345 350
   Trp Ala Pro Asn Leu His Phe His Ser Pro Asn Pro Glu Ile Pro Ala
   355 360 365
   Leu Leu Asp Gly Arg Leu Gln Val Val Asp Gln Pro Leu Pro Val Arg
   370 375 380
   Gly Gly Asn Val Gly Ile Asn Ser Phe Gly Phe Gly Gly Ser Asn Val
   385 390 395 400
   His Ile Ile Leu Arg Pro Asn Thr Gln Pro Pro Pro Ala Pro Ala Pro
   405 410 415
   His Ala Thr Leu Pro Arg Leu Leu Arg Ala Ser Gly Arg Thr Pro Glu
   420 425 430
   Ala Val Gln Lys Leu Leu Glu Gln Gly Leu Arg His Ser Gln Asp Leu
   435 440 445
   Ala Phe Leu Ser Met Leu Asn Asp Ile Ala Ala Val Pro Ala Thr Ala
   450 455 460
   Met Pro Phe Arg Gly Tyr Ala Val Leu Gly Gly Glu Arg Gly Gly Pro
   465 470 475 480
   Glu Val Gln Gln Val Pro Ala Gly Glu Arg Pro Leu Trp Phe Ile Cys
   485 490 495
   Ser Gly Met Gly Thr Gln Trp Arg Gly Met Gly Leu Ser Leu Met Arg
   500 505 510
   Leu Asp Arg Phe Arg Asp Ser Ile Leu Arg Ser Asp Glu Ala Val Lys
   515 520 525
   Pro Phe Gly Leu Lys Val Ser Gln Leu Leu Leu Ser Thr Asp Glu Ser
   530 535 540
   Thr Phe Asp Asp Ile Val His Ser Phe Val Ser Leu Thr Ala Ile Gln
   545 550 555 560
   Ile Gly Leu Ile Asp Leu Leu Ser Cys Met Gly Leu Arg Pro Asp Gly
   565 570 575
   Ile Val Gly His Ser Leu Gly Glu Val Ala Cys Gly Tyr Ala Asp Gly
   580 585 590
   Cys Leu Ser Gln Glu Glu Ala Val Leu Ala Ala Tyr Trp Arg Gly Gln
   595 600 605
   Cys Ile Lys Glu Ala His Leu Pro Pro Gly Ala Met Ala Ala Val Gly
   610 615 620
   Leu Ser Trp Glu Glu Cys Lys Gln Arg Cys Pro Pro Gly Val Val Pro
   625 630 635 640
   Ala Cys His Asn Ser Lys Asp Thr Val Thr Ile Ser Gly Pro Gln Ala
   645 650 655
   Pro Val Phe Glu Phe Val Glu Gln Leu Arg Lys Glu Gly Val Phe Ala
   660 665 670
   Lys Glu Val Arg Thr Gly Gly Met Ala Phe His Ser Tyr Phe Met Glu
           675                 680                 685
   Ala Ile Ala Pro Pro Leu Leu Gln Glu Leu Lys Lys Val Ile Arg Glu
       690                 695                 700
   Pro Lys Pro Arg Ser Ala Arg Trp Leu Ser Thr Ser Ile Pro Glu Ala
   705                 710                 715                 720
   Gln Trp His Ser Ser Leu Ala Arg Thr Ser Ser Ala Glu Tyr Asn Val
                   725                 730                 735
   Asn Asn Leu Val Ser Pro Val Leu Phe Gln Glu Ala Leu Trp His Val
               740                 745                 750
   Pro Glu His Ala Val Val Leu Glu Ile Ala Pro His Ala Leu Leu Gln
           755                 760                 765
   Ala Val Leu Lys Arg Gly Leu Lys Pro Ser Cys Thr Ile Ile Pro Leu
       770                 775                 780
   Met Lys Lys Asp His Arg Asp Asn Leu Glu Phe Phe Leu Ala Gly Ile
   785                 790                 795                 800
   Gly Arg Leu His Leu Ser Gly Ile Asp Ala Asn Pro Asn Ala Leu Phe
                   805                 810                 815
   Pro Pro Val Glu Phe Pro Ala Pro Arg Gly Thr Pro Leu Ile Ser Pro
               820                 825                 830
   Leu Ile Lys Trp Asp His Ser Leu Ala Trp Asp Val Pro Ala Ala Glu
           835                 840                 845
   Asp Phe Pro Asn Gly Ser Gly Ser Pro Ser Ala Ala Ile Tyr Asn Ile
       850                 855                 860
   Asp Thr Ser Ser Glu Ser Pro Asp His Tyr Leu Val Asp His Thr Leu
   865                 870                 875                 880
   Asp Gly Arg Val Leu Phe Pro Ala Thr Gly Tyr Leu Ser Ile Val Trp
                   885                 890                 895
   Lys Thr Leu Ala Arg Ala Leu Gly Leu Gly Val Glu Gln Leu Pro Val
               900                 905                 910
   Val Phe Glu Asp Val Val Leu His Gln Ala Thr Ile Leu Pro Lys Thr
           915                 920                 925
   Gly Thr Val Ser Leu Glu Val Arg Leu Leu Glu Ala Ser Arg Ala Phe
       930                 935                 940
   Glu Val Ser Glu Asn Gly Asn Leu Val Val Ser Gly Lys Val Tyr Gln
   945                 950                 955                 960
   Trp Asp Asp Pro Asp Pro Arg Leu Phe Asp His Pro Glu Ser Pro Thr
                   965                 970                 975
   Pro Asn Pro Thr Glu Pro Leu Phe Leu Ala Gln Ala Glu Val Tyr Lys
               980                 985                 990
   Glu Leu Arg Leu Arg Gly Tyr Asp  Tyr Gly Pro His Phe  Gln Gly Ile
           995                 1000                 1005
   Leu Glu  Ala Ser Leu Glu Gly  Asp Ser Gly Arg Leu  Leu Trp Lys
       1010                 1015                 1020
   Asp Asn  Trp Val Ser Phe Met  Asp Thr Met Leu Gln  Met Ser Ile
       1025                 1030                 1035
   Leu Gly  Ser Ala Lys His Gly  Leu Tyr Leu Pro Thr  Arg Val Thr
       1040                 1045                 1050
   Ala Ile  His Ile Asp Pro Ala  Thr His Arg Gln Lys  Leu Tyr Thr
       1055                 1060                 1065
   Leu Gln  Asp Lys Ala Gln Val  Ala Asp Val Val Val  Ser Arg Trp
       1070                 1075                 1080
   Leu Arg  Val Thr Val Ala Gly  Gly Val His Ile Ser  Gly Leu His
       1085                 1090                 1095
   Thr Glu  Ser Ala Pro Arg Arg  Gln Gln Glu Gln Gln  Val Pro Ile
       1100                 1105                 1110
   Leu Glu  Lys Phe Cys Phe Thr  Pro His Thr Glu Glu  Gly Cys Leu
       1115                 1120                 1125
   Ser Glu  Arg Ala Ala Leu Gln  Glu Glu Leu Gln Leu  Cys Lys Gly
       1130                 1135                 1140
   Leu Val  Gln Ala Leu Gln Thr  Lys Val Thr Gln Gln  Gly Leu Lys
       1145                 1150                 1155
   Met Val  Val Pro Gly Leu Asp  Gly Ala Gln Ile Pro  Arg Asp Pro
       1160                 1165                 1170
   Ser Gln  Gln Glu Leu Pro Arg  Leu Leu Ser Ala Ala  Cys Arg Leu
       1175                 1180                 1185
   Gln Leu  Asn Gly Asn Leu Gln  Leu Glu Leu Ala Gln  Val Leu Ala
       1190                 1195                 1200
   Gln Glu Arg Pro Lys Leu Pro Glu Asp Pro Leu Leu Ser Gly Leu
   1205 1210 1215
   Leu Asp Ser Pro Ala Leu Lys Ala Cys Leu Asp Thr Ala Val Glu
   1220 1225 1230
   Asn Met Pro Ser Leu Lys Met Lys Val Val Glu Val Leu Ala Gly
   1235 1240 1245
   His Gly His Leu Tyr Ser Arg Ile Pro Gly Leu Leu Ser Pro His
   1250 1255 1260
   Pro Leu Leu Gln Leu Ser Tyr Thr Ala Thr Asp Arg His Pro Gln
   1265 1270 1275
   Ala Leu Glu Ala Ala Gln Ala Glu Leu Gln Gln His Asp Val Ala
   1280 1285 1290
   Gln Gly Gln Trp Asp Pro Ala Asp Pro Ala Pro Ser Ala Leu Gly
   1295 1300 1305
   Ser Ala Asp Leu Leu Val Cys Asn Cys Ala Val Ala Ala Leu Gly
   1310 1315 1320
   Asp Pro Ala Ser Ala Leu Ser Asn Met Val Ala Ala Leu Arg Glu
   1325 1330 1335
   Gly Gly Phe Leu Leu Leu His Thr Leu Leu Arg Gly His Pro Leu
   1340 1345 1350
   Gly Asp Ile Val Ala Phe Leu Thr Ser Thr Glu Pro Gln Tyr Gly
   1355 1360 1365
   Gln Gly Ile Leu Ser Gln Asp Ala Trp Glu Ser Leu Phe Ser Arg
   1370 1375 1380
   Val Ser Leu Arg Leu Val Gly Leu Lys Lys Ser Phe Tyr Gly Ser
   1385 1390 1395
   Thr Leu Phe Leu Cys Arg Arg Pro Thr Pro Gln Asp Ser Pro Ile
   1400 1405 1410
   Phe Leu Pro Val Asp Asp Thr Ser Phe Arg Trp Val Glu Ser Leu
   1415 1420 1425
   Lys Gly Ile Leu Ala Asp Glu Asp Ser Ser Arg Pro Val Trp Leu
   1430 1435 1440
   Lys Ala Ile Asn Cys Ala Thr Ser Gly Val Val Gly Leu Val Asn
   1445 1450 1455
   Cys Leu Arg Arg Glu Pro Gly Gly Asn Arg Leu Arg Cys Val Leu
   1460 1465 1470
   Leu Ser Asn Leu Ser Ser Thr Ser His Val Pro Glu Val Asp Pro
   1475 1480 1485
   Gly Ser Ala Glu Leu Gln Lys Val Leu Gln Gly Asp Leu Val Met
   1490 1495 1500
   Asn Val Tyr Arg Asp Gly Ala Trp Gly Ala Phe Arg His Phe Leu
   1505 1510 1515
   Leu Glu Glu Asp Lys Pro Glu Glu Pro Thr Ala His Ala Phe Val
   1520 1525 1530
   Ser Thr Leu Thr Arg Gly Asp Leu Ser Ser Ile Arg Trp Val Cys
   1535 1540 1545
   Ser Ser Leu Arg His Ala Gln Pro Thr Cys Pro Gly Ala Gln Leu
   1550 1555 1560
   Cys Thr Val Tyr Tyr Ala Ser Leu Asn Phe Arg Asp Ile Met Leu
   1565 1570 1575
   Ala Thr Gly Lys Leu Ser Pro Asp Ala Ile Pro Gly Lys Trp Thr
   1580 1585 1590
   Ser Gln Asp Ser Leu Leu Gly Met Glu Phe Ser Gly Arg Asp Ala
   1595 1600 1605
   Ser Gly Lys Arg Val Met Gly Leu Val Pro Ala Lys Gly Leu Ala
   1610 1615 1620
   Thr Ser Val Leu Leu Ser Pro Asp Phe Leu Trp Asp Val Pro Ser
   1625 1630 1635
   Asn Trp Thr Leu Glu Glu Ala Ala Ser Val Pro Val Val Tyr Ser
   1640 1645 1650
   Thr Ala Tyr Tyr Ala Leu Val Val Arg Gly Arg Val Arg Pro Gly
   1655 1660 1665
   Glu Thr Leu Leu Ile His Ser Gly Ser Gly Gly Val Gly Gln Ala
   1670 1675 1680
   Ala Ile Ala Ile Ala Leu Ser Leu Gly Cys Arg Val Phe Thr Thr
   1685 1690 1695
   Val Gly Ser Ala Glu Lys Arg Ala Tyr Leu Gln Ala Arg Phe Pro
   1700 1705 1710
   Gln Leu Asp Ser Thr Ser Phe Ala Asn Ser Arg Asp Thr Ser Phe
   1715 1720 1725
   Glu Gln His Val Leu Trp His Thr Gly Gly Lys Gly Val Asp Leu
   1730 1735 1740
   Val Leu Asn Ser Leu Ala Glu Glu Lys Leu Gln Ala Ser Val Arg
   1745 1750 1755
   Cys Leu Ala Thr His Gly Arg Phe Leu Glu Ile Gly Lys Phe Asp
   1760 1765 1770
   Leu Ser Gln Asn His Pro Leu Gly Met Ala Ile Phe Leu Lys Asn
   1775 1780 1785
   Val Thr Phe His Gly Val Leu Leu Asp Ala Phe Phe Asn Glu Ser
   1790 1795 1800
   Ser Ala Asp Trp Arg Glu Val Trp Ala Leu Val Gln Ala Gly Ile
   1805 1810 1815
   Arg Asp Gly Val Val Arg Pro Leu Lys Cys Thr Val Phe His Gly
   1820 1825 1830
   Ala Gln Val Glu Asp Ala Phe Arg Tyr Met Ala Gln Gly Lys His
   1835 1840 1845
   Ile Gly Lys Val Val Val Gln Val Leu Ala Glu Glu Pro Glu Ala
   1850 1855 1860
   Val Leu Lys Gly Ala Lys Pro Lys Leu Met Ser Ala Ile Ser Lys
   1865 1870 1875
   Thr Phe Cys Pro Ala His Lys Ser Tyr Ile Ile Ala Gly Gly Leu
   1880 1885 1890
   Gly Gly Phe Gly Leu Glu Leu Ala Gln Trp Leu Ile Gln Arg Gly
   1895 1900 1905
   Val Gln Lys Leu Val Leu Thr Ser Arg Ser Gly Ile Arg Thr Gly
   1910 1915 1920
   Tyr Gln Ala Lys Gln Val Arg Arg Trp Arg Arg Gln Gly Val Gln
   1925 1930 1935
   Val Gln Val Ser Thr Ser Asn Ile Ser Ser Leu Glu Gly Ala Arg
   1940 1945 1950
   Gly Leu Ile Ala Glu Ala Ala Gln Leu Gly Pro Val Gly Gly Val
   1955 1960 1965
   Phe Asn  Leu Ala Val Val Leu  Arg Asp Gly Leu Leu  Glu Asn Gln
       1970                 1975                 1980
   Thr Pro  Glu Phe Phe Gln Asp  Val Cys Lys Pro Lys  Tyr Ser Gly
       1985                 1990                 1995
   Thr Leu  Asn Leu Asp Arg Val  Thr Arg Glu Ala Cys  Pro Glu Leu
       2000                 2005                 2010
   Asp Tyr  Phe Val Val Phe Ser  Ser Val Ser Cys Gly  Arg Gly Asn
       2015                 2020                 2025
   Ala Gly  Gln Ser Asn Tyr Gly  Phe Ala Asn Ser Ala  Met Glu Arg
       2030                 2035                 2040
   Ile Cys  Glu Lys Arg Arg His  Glu Gly Leu Pro Gly  Leu Ala Val
       2045                 2050                 2055
   Gln Trp  Gly Ala Ile Gly Asp  Val Gly Ile Leu Val  Glu Thr Met
       2060                 2065                 2070
   Ser Thr  Asn Asp Thr Ile Val  Ser Gly Thr Leu Pro  Gln Arg Met
       2075                 2080                 2085
   Ala Ser  Cys Leu Glu Val Leu  Asp Leu Phe Leu Asn  Gln Pro His
       2090                 2095                 2100
   Met Val  Leu Ser Ser Phe Val  Leu Ala Glu Lys Ala  Ala Ala Tyr
       2105                 2110                 2115
   Arg Asp  Arg Asp Ser Gln Arg  Asp Leu Val Glu Ala  Val Ala His
       2120                 2125                 2130
   Ile Leu  Gly Ile Arg Asp Leu  Ala Ala Val Asn Leu  Asp Ser Ser
       2135                 2140                 2145
   Leu Ala  Asp Leu Gly Leu Asp  Ser Leu Met Ser Val  Glu Val Arg
       2150                 2155                 2160
   Gln Thr  Leu Glu Arg Glu Leu  Asn Leu Val Leu Ser  Val Arg Glu
       2165                 2170                 2175
   Val Arg  Gln Leu Thr Leu Arg  Lys Leu Gln Glu Leu  Ser Ser Lys
       2180                 2185                 2190
   Ala Asp  Glu Ala Ser Glu Leu  Ala Cys Pro Thr Pro  Lys Glu Asp
       2195                 2200                 2205
   Gly Leu  Ala Gln Gln Gln Thr  Gln Leu Asn Leu Arg  Ser Leu Leu
       2210                 2215                 2220
   Val Asn Pro Glu Gly Pro Thr Leu Met Arg Leu Asn Ser Val Gln
   2225 2230 2235
   Ser Ser Glu Arg Pro Leu Phe Leu Val His Pro Ile Glu Gly Ser
   2240 2245 2250
   Thr Thr Val Phe His Ser Leu Ala Ser Arg Leu Ser Ile Pro Thr
   2255 2260 2265
   Tyr Gly Leu Gln Cys Thr Arg Ala Ala Pro Leu Asp Ser Ile His
   2270 2275 2280
   Ser Leu Ala Ala Tyr Tyr Ile Asp Cys Ile Arg Gln Val Gln Pro
   2285 2290 2295
   Glu Gly Pro Tyr Arg Val Ala Gly Tyr Ser Tyr Gly Ala Cys Val
   2300 2305 2310
   Ala Phe Glu Met Cys Ser Gln Leu Gln Ala Gln Gln Ser Pro Ala
   2315 2320 2325
   Pro Thr His Asn Ser Leu Phe Leu Phe Asp Gly Ser Pro Thr Tyr
   2330 2335 2340
   Val Leu Ala Tyr Thr Gln Ser Tyr Arg Ala Lys Leu Thr Pro Gly
   2345 2350 2355
   Cys Glu Ala Glu Ala Glu Thr Glu Ala Ile Cys Phe Phe Val Gln
   2360 2365 2370
   Gln Phe Thr Asp Met Glu His Asn Arg Val Leu Glu Ala Leu Leu
   2375 2380 2385
   Pro Leu Lys Gly Leu Glu Glu Arg Val Ala Ala Ala Val Asp Leu
   2390 2395 2400
   Ile Ile Lys Ser His Gln Gly Leu Asp Arg Gln Glu Leu Ser Phe
   2405 2410 2415
   Ala Ala Arg Ser Phe Tyr Tyr Lys Leu Arg Ala Ala Glu Gln Tyr
   2420 2425 2430
   Thr Pro Lys Ala Lys Tyr His Gly Asn Val Met Leu Leu Arg Ala
   2435 2440 2445
   Lys Thr Gly Gly Ala Tyr Gly Glu Asp Leu Gly Ala Asp Tyr Asn
   2450 2455 2460
   Leu Ser Gln Val Cys Asp Gly Lys Val Ser Val His Val Ile Glu
   2465 2470 2475
   Gly Asp His Arg Thr Leu Leu Glu Gly Ser Gly Leu Glu Ser Ile
       2480                2485                2490
   Ile Ser Ile Ile His Ser Ser Leu Ala Glu Pro Arg Val Ser Val
       2495                2500                2505
   Arg Glu Gly
       2510
<210> 28
   <211> 286
   <212> PRT
   <213> Homo sapiens
<400> 28
   Met Ala Glu Asp Gly Glu Glu Ala Glu Phe His Phe Ala Ala Leu Tyr
   1               5                   10                  15
   Ile Ser Gly Gln Trp Pro Arg Leu Arg Ala Asp Thr Asp Leu Gln Arg
               20                  25                  30
   Leu Gly Ser Ser Ala Met Ala Pro Ser Arg Lys Phe Phe Val Gly Gly
           35                  40                  45
   Asn Trp Lys Met Asn Gly Arg Lys Gln Ser Leu Gly Glu Leu Ile Gly
       50                  55                  60
   Thr Leu Asn Ala Ala Lys Val Pro Ala Asp Thr Glu Val Val Cys Ala
   65                  70                  75                  80
   Pro Pro Thr Ala Tyr Ile Asp Phe Ala Arg Gln Lys Leu Asp Pro Lys
                   85                  90                  95
   Ile Ala Val Ala Ala Gln Asn Cys Tyr Lys Val Thr Asn Gly Ala Phe
               100                 105                 110
   Thr Gly Glu Ile Ser Pro Gly Met Ile Lys Asp Cys Gly Ala Thr Trp
           115                 120                 125
   Val Val Leu Gly His Ser Glu Arg Arg His Val Phe Gly Glu Ser Asp
       130                 135                 140
   Glu Leu Ile Gly Gln Lys Val Ala His Ala Leu Ala Glu Gly Leu Gly
   145                 150                 155                 160
   Val Ile Ala Cys Ile Gly Glu Lys Leu Asp Glu Arg Glu Ala Gly Ile
                   165                 170                 175
   Thr Glu Lys Val Val Phe Glu Gln Thr Lys Val Ile Ala Asp Asn Val
               180                 185                 190
   Lys Asp Trp Ser Lys Val Val Leu Ala Tyr Glu Pro Val Trp Ala Ile
   195 200 205
   Gly Thr Gly Lys Thr Ala Thr Pro Gln Gln Ala Gln Glu Val His Glu
   210 215 220
   Lys Leu Arg Gly Trp Leu Lys Ser Asn Val Ser Asp Ala Val Ala Gln
   225 230 235 240
   Ser Thr Arg Ile Ile Tyr Gly Gly Ser Val Thr Gly Ala Thr Cys Lys
   245 250 255
   Glu Leu Ala Ser Gln Pro Asp Val Asp Gly Phe Leu Val Gly Gly Ala
   260 265 270
   Ser Leu Lys Pro Glu Phe Val Asp Ile Ile Asn Ala Lys Gln
   275 280 285
<210> 29
   <211> 1101
   <212> PRT
   <213> Homo sapiens
<400> 29
   Met Ser Ala Lys Ala Ile Ser Glu Gln Thr Gly Lys Glu Leu Leu Tyr
   1               5                   10                  15
   Lys Phe Ile Cys Thr Thr Ser Ala Ile Gln Asn Arg Phe Lys Tyr Ala
               20                  25                  30
   Arg Val Thr Pro Asp Thr Asp Trp Ala Arg Leu Leu Gln Asp His Pro
           35                  40                  45
   Trp Leu Leu Ser Gln Asn Leu Val Val Lys Pro Asp Gln Leu Ile Lys
       50                  55                  60
   Arg Arg Gly Lys Leu Gly Leu Val Gly Val Asn Leu Thr Leu Asp Gly
   65                  70                  75                  80
   Val Lys Ser Trp Leu Lys Pro Arg Leu Gly Gln Glu Ala Thr Val Gly
                   85                  90                  95
   Lys Ala Thr Gly Phe Leu Lys Asn Phe Leu Ile Glu Pro Phe Val Pro
               100                 105                 110
   His Ser Gln Ala Glu Glu Phe Tyr Val Cys Ile Tyr Ala Thr Arg Glu
           115                 120                 125
   Gly Asp Tyr Val Leu Phe His His Glu Gly Gly Val Asp Val Gly Asp
       130                 135                 140
   Val Asp Ala Lys Ala Gln Lys Leu Leu Val Gly Val Asp Glu Lys Leu
   145                 150                 155                 160
   Asn Pro Glu Asp Ile Lys Lys His Leu Leu Val His Ala Pro Glu Asp
   165 170 175
   Lys Lys Glu Ile Leu Ala Ser Phe Ile Ser Gly Leu Phe Asn Phe Tyr
   180 185 190
   Glu Asp Leu Tyr Phe Thr Tyr Leu Glu Ile Asn Pro Leu Val Val Thr
   195 200 205
   Lys Asp Gly Val Tyr Val Leu Asp Leu Ala Ala Lys Val Asp Ala Thr
   210 215 220
   Ala Asp Tyr Ile Cys Lys Val Lys Trp Gly Asp Ile Glu Phe Pro Pro
   225 230 235 240
   Pro Phe Gly Arg Glu Ala Tyr Pro Glu Glu Ala Tyr Ile Ala Asp Leu
   245 250 255
   Asp Ala Lys Ser Gly Ala Ser Leu Lys Leu Thr Leu Leu Asn Pro Lys
   260 265 270
   Gly Arg Ile Trp Thr Met Val Ala Gly Gly Gly Ala Ser Val Val Tyr
   275 280 285
   Ser Asp Thr Ile Cys Asp Leu Gly Gly Val Asn Glu Leu Ala Asn Tyr
   290 295 300
   Gly Glu Tyr Ser Gly Ala Pro Ser Glu Gln Gln Thr Tyr Asp Tyr Ala
   305 310 315 320
   Lys Thr Ile Leu Ser Leu Met Thr Arg Glu Lys His Pro Asp Gly Lys
   325 330 335
   Ile Leu Ile Ile Gly Gly Ser Ile Ala Asn Phe Thr Asn Val Ala Ala
   340 345 350
   Thr Phe Lys Gly Ile Val Arg Ala Ile Arg Asp Tyr Gln Gly Pro Leu
   355 360 365
   Lys Glu His Glu Val Thr Ile Phe Val Arg Arg Gly Gly Pro Asn Tyr
   370 375 380
   Gln Glu Gly Leu Arg Val Met Gly Glu Val Gly Lys Thr Thr Gly Ile
   385 390 395 400
   Pro Ile His Val Phe Gly Thr Glu Thr His Met Thr Ala Ile Val Gly
   405 410 415
   Met Ala Leu Gly His Arg Pro Ile Pro Asn Gln Pro Pro Thr Ala Ala
   420 425 430
   His Thr Ala Asn Phe Leu Leu Asn Ala Ser Gly Ser Thr Ser Thr Pro
   435 440 445
   Ala Pro Ser Arg Thr Ala Ser Phe Ser Glu Ser Arg Ala Asp Glu Val
   450 455 460
   Ala Pro Ala Lys Lys Ala Lys Pro Ala Met Pro Gln Asp Ser Val Pro
   465 470 475 480
   Ser Pro Arg Ser Leu Gln Gly Lys Ser Thr Thr Leu Phe Ser Arg His
   485 490 495
   Thr Lys Ala Ile Val Trp Gly Met Gln Thr Arg Ala Val Gln Gly Met
   500 505 510
   Leu Asp Phe Asp Tyr Val Cys Ser Arg Asp Glu Pro Ser Val Ala Ala
   515 520 525
   Met Val Tyr Pro Phe Thr Gly Asp His Lys Gln Lys Phe Tyr Trp Gly
   530 535 540
   His Lys Glu Ile Leu Ile Pro Val Phe Lys Asn Met Ala Asp Ala Met
   545 550 555 560
   Arg Lys His Pro Glu Val Asp Val Leu Ile Asn Phe Ala Ser Leu Arg
   565 570 575
   Ser Ala Tyr Asp Ser Thr Met Glu Thr Met Asn Tyr Ala Gln Ile Arg
   580 585 590
   Thr Ile Ala Ile Ile Ala Glu Gly Ile Pro Glu Ala Leu Thr Arg Lys
   595 600 605
   Leu Ile Lys Lys Ala Asp Gln Lys Gly Val Thr Ile Ile Gly Pro Ala
   610 615 620
   Thr Val Gly Gly Ile Lys Pro Gly Cys Phe Lys Ile Gly Asn Thr Gly
   625 630 635 640
   Gly Met Leu Asp Asn Ile Leu Ala Ser Lys Leu Tyr Arg Pro Gly Ser
   645 650 655
   Val Ala Tyr Val Ser Arg Ser Gly Gly Met Ser Asn Glu Leu Asn Asn
   660 665 670
   Ile Ile Ser Arg Thr Thr Asp Gly Val Tyr Glu Gly Val Ala Ile Gly
   675 680 685
   Gly Asp Arg Tyr Pro Gly Ser Thr Phe Met Asp His Val Leu Arg Tyr
   690 695 700
   Gln Asp Thr Pro Gly Val Lys Met Ile Val Val Leu Gly Glu Ile Gly
   705 710 715 720
   Gly Thr Glu Glu Tyr Lys Ile Cys Arg Gly Ile Lys Glu Gly Arg Leu
   725 730 735
   Thr Lys Pro Ile Val Cys Trp Cys Ile Gly Thr Cys Ala Thr Met Phe
   740 745 750
   Ser Ser Glu Val Gln Phe Gly His Ala Gly Ala Cys Ala Asn Gln Ala
   755 760 765
   Ser Glu Thr Ala Val Ala Lys Asn Gln Ala Leu Lys Glu Ala Gly Val
   770 775 780
   Phe Val Pro Arg Ser Phe Asp Glu Leu Gly Glu Ile Ile Gln Ser Val
   785 790 795 800
   Tyr Glu Asp Leu Val Ala Asn Gly Val Ile Val Pro Ala Gln Glu Val
   805 810 815
   Pro Pro Pro Thr Val Pro Met Asp Tyr Ser Trp Ala Arg Glu Leu Gly
   820 825 830
   Leu Ile Arg Lys Pro Ala Ser Phe Met Thr Ser Ile Cys Asp Glu Arg
   835 840 845
   Gly Gln Glu Leu Ile Tyr Ala Gly Met Pro Ile Thr Glu Val Phe Lys
   850 855 860
   Glu Glu Met Gly Ile Gly Gly Val Leu Gly Leu Leu Trp Phe Gln Lys
   865 870 875 880
   Arg Leu Pro Lys Tyr Ser Cys Gln Phe Ile Glu Met Cys Leu Met Val
   885 890 895
   Thr Ala Asp His Gly Pro Ala Val Ser Gly Ala His Asn Thr Ile Ile
   900 905 910
   Cys Ala Arg Ala Gly Lys Asp Leu Val Ser Ser Leu Thr Ser Gly Leu
   915 920 925
   Leu Thr Ile Gly Asp Arg Phe Gly Gly Ala Leu Asp Ala Ala Ala Lys
   930 935 940
   Met Phe Ser Lys Ala Phe Asp Ser Gly Ile Ile Pro Met Glu Phe Val
   945 950 955 960
   Asn Lys Met Lys Lys Glu Gly Lys Leu Ile Met Gly Ile Gly His Arg
   965 970 975
   Val Lys Ser Ile Asn Asn Pro Asp Met Arg Val Gln Ile Leu Lys Asp
   980 985 990
   Tyr Val Arg Gln His Phe Pro Ala Thr Pro Leu Leu Asp Tyr Ala Leu
   995 1000 1005
   Glu Val Glu Lys Ile Thr Thr Ser Lys Lys Pro Asn Leu Ile Leu
   1010 1015 1020
   Asn Val Asp Gly Leu Ile Gly Val Ala Phe Val Asp Met Leu Arg
   1025 1030 1035
   Asn Cys Gly Ser Phe Thr Arg Glu Glu Ala Asp Glu Tyr Ile Asp
   1040 1045 1050
   Ile Gly Ala Leu Asn Gly Ile Phe Val Leu Gly Arg Ser Met Gly
   1055 1060 1065
   Phe Ile Gly His Tyr Leu Asp Gln Lys Arg Leu Lys Gln Gly Leu
   1070 1075 1080
   Tyr Arg His Pro Trp Asp Asp Ile Ser Tyr Val Leu Pro Glu His
   1085 1090 1095
   Met Ser Met
   1100
<210> 30
   <211> 417
   <212> PRT
   <213> Homo sapiens
<400> 30
   Met Leu Leu Ser Val Pro Leu Leu Leu Gly Leu Leu Gly Leu Ala Val
   1 5 10 15
   Ala Glu Pro Ala Val Tyr Phe Lys Glu Gln Phe Leu Asp Gly Asp Gly
   20 25 30
   Trp Thr Ser Arg Trp Ile Glu Ser Lys His Lys Ser Asp Phe Gly Lys
   35 40 45
   Phe Val Leu Ser Ser Gly Lys Phe Tyr Gly Asp Glu Glu Lys Asp Lys
   50 55 60
   Gly Leu Gln Thr Ser Gln Asp Ala Arg Phe Tyr Ala Leu Ser Ala Ser
   65 70 75 80
   Phe Glu Pro Phe Ser Asn Lys Gly Gln Thr Leu Val Val Gln Phe Thr
   85 90 95
   Val Lys His Glu Gln Asn Ile Asp Cys Gly Gly Gly Tyr Val Lys Leu
   100 105 110
   Phe Pro Asn Ser Leu Asp Gln Thr Asp Met His Gly Asp Ser Glu Tyr
   115 120 125
   Asn Ile Met Phe Gly Pro Asp Ile Cys Gly Pro Gly Thr Lys Lys Val
   130 135 140
   His Val Ile Phe Asn Tyr Lys Gly Lys Asn Val Leu Ile Asn Lys Asp
   145 150 155 160
   Ile Arg Cys Lys Asp Asp Glu Phe Thr His Leu Tyr Thr Leu Ile Val
   165 170 175
   Arg Pro Asp Asn Thr Tyr Glu Val Lys Ile Asp Asn Ser Gln Val Glu
   180 185 190
   Ser Gly Ser Leu Glu Asp Asp Trp Asp Phe Leu Pro Pro Lys Lys Ile
   195 200 205
   Lys Asp Pro Asp Ala Ser Lys Pro Glu Asp Trp Asp Glu Arg Ala Lys
   210 215 220
   Ile Asp Asp Pro Thr Asp Ser Lys Pro Glu Asp Trp Asp Lys Pro Glu
   225 230 235 240
   His Ile Pro Asp Pro Asp Ala Lys Lys Pro Glu Asp Trp Asp Glu Glu
   245 250 255
   Met Asp Gly Glu Trp Glu Pro Pro Val Ile Gln Asn Pro Glu Tyr Lys
   260 265 270
   Gly Glu Trp Lys Pro Arg Gln Ile Asp Asn Pro Asp Tyr Lys Gly Thr
   275 280 285
   Trp Ile His Pro Glu Ile Asp Asn Pro Glu Tyr Ser Pro Asp Pro Ser
   290 295 300
   Ile Tyr Ala Tyr Asp Asn Phe Gly Val Leu Gly Leu Asp Leu Trp Gln
   305 310 315 320
   Val Lys Ser Gly Thr Ile Phe Asp Asn Phe Leu Ile Thr Asn Asp Glu
   325 330 335
   Ala Tyr Ala Glu Glu Phe Gly Asn Glu Thr Trp Gly Val Thr Lys Ala
   340 345 350
   Ala Glu Lys Gln Met Lys Asp Lys Gln Asp Glu Glu Gln Arg Leu Lys
   355 360 365
   Glu Glu Glu Glu Asp Lys Lys Arg Lys Glu Glu Glu Glu Ala Glu Asp
   370 375 380
   Lys Glu Asp Asp Glu Asp Lys Asp Glu Asp Glu Glu Asp Glu Glu Asp
   385 390 395 400
   Lys Glu Glu Asp Glu Glu Glu Asp Val Pro Gly Gln Ala Lys Asp Glu
   405 410 415
   Leu
<210> 31
   <211> 1268
   <212> PRT
   <213> Homo sapiens
<400> 31
<210> 32
   <211> 4128
   <212> PRT
   <213> Homo sapiens
<400> 32
<210> 33
   <211> 445
   <212> PRT
   <213> Homo sapiens
<400> 33
   Met Asn Glu Glu Tyr Asp Val Ile Val Leu Gly Thr Gly Leu Thr Glu
   1               5                   10                  15
   Cys Ile Leu Ser Gly Ile Met Ser Val Asn Gly Lys Lys Val Leu His
               20                  25                  30
   Met Asp Arg Asn Pro Tyr Tyr Gly Gly Glu Ser Ala Ser Ile Thr Pro
           35                  40                  45
   Leu Glu Asp Leu Tyr Lys Arg Phe Lys Ile Pro Gly Ser Pro Pro Glu
       50                  55                  60
   Ser Met Gly Arg Gly Arg Asp Trp Asn Val Asp Leu Ile Pro Lys Phe
   65                  70                  75                  80
   Leu Met Ala Asn Gly Gln Leu Val Lys Met Leu Leu Tyr Thr Glu Val
                   85                  90                  95
   Thr Arg Tyr Leu Asp Phe Lys Val Thr Glu Gly Ser Phe Val Tyr Lys
               100                 105                 110
   Gly Gly Lys Ile Tyr Lys Val Pro Ser Thr Glu Ala Glu Ala Leu Ala
           115                 120                 125
   Ser Ser Leu Met Gly Leu Phe Glu Lys Arg Arg Phe Arg Lys Phe Leu
       130                 135                 140
   Val Tyr Val Ala Asn Phe Asp Glu Lys Asp Pro Arg Thr Phe Glu Gly
   145                 150                 155                 160
   Ile Asp Pro Lys Lys Thr Thr Met Arg Asp Val Tyr Lys Lys Phe Asp
                   165                 170                 175
   Leu Gly Gln Asp Val Ile Asp Phe Thr Gly His Ala Leu Ala Leu Tyr
               180                 185                 190
   Arg Thr Asp Asp Tyr Leu Asp Gln Pro Cys Tyr Glu Thr Ile Asn Arg
           195                 200                 205
   Ile Lys Leu Tyr Ser Glu Ser Leu Ala Arg Tyr Gly Lys Ser Pro Tyr
       210                 215                 220
   Leu Tyr Pro Leu Tyr Gly Leu Gly Glu Leu Pro Gln Gly Phe Ala Arg
   225                 230                 235                 240
   Leu Ser Ala Ile Tyr Gly Gly Thr Tyr Met Leu Asn Lys Pro Ile Glu
                   245                 250                 255
   Glu Ile Ile Val Gln Asn Gly Lys Val Ile Gly Val Lys Ser Glu Gly
               260                 265                 270
   Glu Ile Ala Arg Cys Lys Gln Leu Ile Cys Asp Pro Ser Tyr Val Lys
           275                 280                 285
   Asp Arg Val Glu Lys Val Gly Gln Val Ile Arg Val Ile Cys Ile Leu
       290                 295                 300
   Ser His Pro Ile Lys Asn Thr Asn Asp Ala Asn Ser Cys Gln Ile Ile
   305                 310                 315                 320
   Ile Pro Gln Asn Gln Val Asn Arg Lys Ser Asp Ile Tyr Val Cys Met
                   325                 330                 335
   Ile Ser Phe Ala His Asn Val Ala Ala Gln Gly Lys Tyr Ile Ala Ile
               340                 345                 350
   Val Ser Thr Thr Val Glu Thr Lys Glu Pro Glu Lys Glu Ile Arg Pro
           355                 360                 365
   Ala Leu Glu Leu Leu Glu Pro Ile Glu Gln Lys Phe Val Ser Ile Ser
       370                 375                 380
   Asp Leu Leu Val Pro Lys Asp Leu Gly Thr Glu Ser Gln Ile Phe Ile
   385                 390                 395                 400
   Ser Arg Thr Tyr Asp Ala Thr Thr His Phe Glu Thr Thr Cys Asp Asp
                   405                 410                 415
   Ile Lys Asn Ile Tyr Lys Arg Met Thr Gly Ser Glu Phe Asp Phe Glu
               420                 425                 430
   Glu Met Lys Arg Lys Lys Asn Asp Ile Tyr Gly Glu Asp
           435                 440                 445
<210> 34
   <211> 529
   <212> PRT
   <213> Homo sapiens
<400> 34
   Met Leu Gly Phe Val Gly Arg Val Ala Ala Ala Pro Ala Ser Gly Ala
   1               5                   10                  15
   Leu Arg Arg Leu Thr Pro Ser Ala Ser Leu Pro Pro Ala Gln Leu Leu
               20                  25                  30
   Leu Arg Ala Ala Pro Thr Ala Val His Pro Val Arg Asp Tyr Ala Ala
           35                  40                  45
   Gln Thr Ser Pro Ser Pro Lys Ala Gly Ala Ala Thr Gly Arg Ile Val
       50                  55                  60
   Ala Val Ile Gly Ala Val Val Asp Val Gln Phe Asp Glu Gly Leu Pro
   65                  70                  75                  80
   Pro Ile Leu Asn Ala Leu Glu Val Gln Gly Arg Glu Thr Arg Leu Val
                   85                  90                  95
   Leu Glu Val Ala Gln His Leu Gly Glu Ser Thr Val Arg Thr Ile Ala
               100                 105                 110
   Met Asp Gly Thr Glu Gly Leu Val Arg Gly Gln Lys Val Leu Asp Ser
           115                 120                 125
   Gly Ala Pro Ile Lys Ile Pro Val Gly Pro Glu Thr Leu Gly Arg Ile
       130                 135                 140
   Met Asn Val Ile Gly Glu Pro Ile Asp Glu Arg Gly Pro Ile Lys Thr
   145                 150                 155                 160
   Lys Gln Phe Ala Pro Ile His Ala Glu Ala Pro Glu Phe Met Glu Met
                   165                 170                 175
   Ser Val Glu Gln Glu Ile Leu Val Thr Gly Ile Lys Val Val Asp Leu
               180                 185                 190
   Leu Ala Pro Tyr Ala Lys Gly Gly Lys Ile Gly Leu Phe Gly Gly Ala
           195                 200                 205
   Gly Val Gly Lys Thr Val Leu Ile Met Glu Leu Ile Asn Asn Val Ala
       210                 215                 220
   Lys Ala His Gly Gly Tyr Ser Val Phe Ala Gly Val Gly Glu Arg Thr
   225                 230                 235                 240
   Arg Glu Gly Asn Asp Leu Tyr His Glu Met Ile Glu Ser Gly Val Ile
                   245                 250                 255
   Asn Leu Lys Asp Ala Thr Ser Lys Val Ala Leu Val Tyr Gly Gln Met
               260                 265                 270
   Asn Glu Pro Pro Gly Ala Arg Ala Arg Val Ala Leu Thr Gly Leu Thr
           275                 280                 285
   Val Ala Glu Tyr Phe Arg Asp Gln Glu Gly Gln Asp Val Leu Leu Phe
       290                 295                 300
   Ile Asp Asn Ile Phe Arg Phe Thr Gln Ala Gly Ser Glu Val Ser Ala
   305                 310                 315                 320
   Leu Leu Gly Arg Ile Pro Ser Ala Val Gly Tyr Gln Pro Thr Leu Ala
                   325                 330                 335
   Thr Asp Met Gly Thr Met Gln Glu Arg Ile Thr Thr Thr Lys Lys Gly
               340                 345                 350
   Ser Ile Thr Ser Val Gln Ala Ile Tyr Val Pro Ala Asp Asp Leu Thr
           355                 360                 365
   Asp Pro Ala Pro Ala Thr Thr Phe Ala His Leu Asp Ala Thr Thr Val
       370                 375                 380
   Leu Ser Arg Ala Ile Ala Glu Leu Gly Ile Tyr Pro Ala Val Asp Pro
   385                 390                 395                 400
   Leu Asp Ser Thr Ser Arg Ile Met Asp Pro Asn Ile Val Gly Ser Glu
                   405                 410                 415
   His Tyr Asp Val Ala Arg Gly Val Gln Lys Ile Leu Gln Asp Tyr Lys
               420                 425                 430
   Ser Leu Gln Asp Ile Ile Ala Ile Leu Gly Met Asp Glu Leu Ser Glu
           435                 440                 445
   Glu Asp Lys Leu Thr Val Ser Arg Ala Arg Lys Ile Gln Arg Phe Leu
       450                 455                 460
   Ser Gln Pro Phe Gln Val Ala Glu Val Phe Thr Gly His Met Gly Lys
   465                 470                 475                 480
   Leu Val Pro Leu Lys Glu Thr Ile Lys Gly Phe Gln Gln Ile Leu Ala
                   485                 490                 495
   Gly Glu Tyr Asp His Leu Pro Glu Gln Ala Phe Tyr Met Val Gly Pro
               500                 505                 510
   Ile Glu Glu Ala Val Ala Lys Ala Asp Lys Leu Ala Glu Glu His Ser
           515                 520                 525
   Ser
<210> 35
   <211> 1045
   <212> PRT
   <213> Homo sapiens
<400> 35
   Met Glu Gly Leu Glu Glu Ala Glu Ala Asn Cys Ser Val Ala Phe Ala
   1               5                   10                  15
   Glu Ala Gln Arg Trp Val Glu Ala Val Thr Glu Lys Asn Phe Glu Thr
               20                  25                  30
   Lys Asp Phe Arg Ala Ser Leu Glu Asn Gly Val Leu Leu Cys Asp Leu
           35                  40                  45
   Ile Asn Lys Leu Lys Pro Gly Val Ile Lys Lys Ile Asn Arg Leu Ser
       50                  55                  60
   Thr Pro Ile Ala Gly Leu Asp Asn Ile Asn Val Phe Leu Lys Ala Cys
   65                  70                  75                  80
   Glu Gln Ile Gly Leu Lys Glu Ala Gln Leu Phe His Pro Gly Asp Leu
                   85                  90                  95
   Gln Asp Leu Ser Asn Arg Val Thr Val Lys Gln Glu Glu Thr Asp Arg
               100                 105                 110
   Arg Val Lys Asn Val Leu Ile Thr Leu Tyr Trp Leu Gly Arg Lys Ala
           115                 120                 125
   Gln Ser Asn Pro Tyr Tyr Asn Gly Pro His Leu Asn Leu Lys Ala Phe
       130                 135                 140
   Glu Asn Leu Leu Gly Gln Ala Leu Thr Lys Ala Leu Glu Asp Ser Ser
   145                 150                 155                 160
   Phe Leu Lys Arg Ser Gly Arg Asp Ser Gly Tyr Gly Asp Ile Trp Cys
                   165                 170                 175
   Pro Glu Arg Gly Glu Phe Leu Ala Pro Pro Arg His His Lys Arg Glu
               180                 185                 190
   Asp Ser Phe Glu Ser Leu Asp Ser Leu Gly Ser Arg Ser Leu Thr Ser
           195                 200                 205
   Cys Ser Ser Asp Ile Thr Leu Arg Gly Gly Arg Glu Gly Phe Glu Ser
       210                 215                 220
   Asp Thr Asp Ser Glu Phe Thr Phe Lys Met Gln Asp Tyr Asn Lys Asp
   225                 230                 235                 240
   Asp Met Ser Tyr Arg Arg Ile Ser Ala Val Glu Pro Lys Thr Ala Leu
   245 250 255
   Pro Phe Asn Arg Phe Leu Pro Asn Lys Ser Arg Gln Pro Ser Tyr Val
   260 265 270
   Pro Ala Pro Leu Arg Lys Lys Lys Pro Asp Lys His Glu Asp Asn Arg
   275 280 285
   Arg Ser Trp Ala Ser Pro Val Tyr Thr Glu Ala Asp Gly Thr Phe Ser
   290 295 300
   Arg Leu Phe Gln Lys Ile Tyr Gly Glu Asn Gly Ser Lys Ser Met Ser
   305 310 315 320
   Asp Val Ser Ala Glu Asp Val Gln Asn Leu Arg Gln Leu Arg Tyr Glu
   325 330 335
   Glu Met Gln Lys Ile Lys Ser Gln Leu Lys Glu Gln Asp Gln Lys Trp
   340 345 350
   Gln Asp Asp Leu Ala Lys Trp Lys Asp Arg Arg Lys Ser Tyr Thr Ser
   355 360 365
   Asp Leu Gln Lys Lys Lys Glu Glu Arg Glu Glu Ile Glu Lys Gln Ala
   370 375 380
   Leu Glu Lys Ser Lys Arg Ser Ser Lys Thr Phe Lys Glu Met Leu Gln
   385 390 395 400
   Asp Arg Glu Ser Gln Asn Gln Lys Ser Thr Val Pro Ser Arg Arg Arg
   405 410 415
   Met Tyr Ser Phe Asp Asp Val Leu Glu Glu Gly Lys Arg Pro Pro Thr
   420 425 430
   Met Thr Val Ser Glu Ala Ser Tyr Gln Ser Glu Arg Val Glu Glu Lys
   435 440 445
   Gly Ala Thr Tyr Pro Ser Glu Ile Pro Lys Glu Asp Ser Thr Thr Phe
   450 455 460
   Ala Lys Arg Glu Asp Arg Val Thr Thr Glu Ile Gln Leu Pro Ser Gln
   465 470 475 480
   Ser Pro Val Glu Glu Gln Ser Pro Ala Ser Leu Ser Ser Leu Arg Ser
   485 490 495
   Arg Ser Thr Gln Met Glu Ser Thr Arg Val Ser Ala Ser Leu Pro Arg
   500 505 510
   Ser Tyr Arg Lys Thr Asp Thr Val Arg Leu Thr Ser Val Val Thr Pro
           515                 520                 525
   Arg Pro Phe Gly Ser Gln Thr Arg Gly Ile Ser Ser Leu Pro Arg Ser
       530                 535                 540
   Tyr Thr Met Asp Asp Ala Trp Lys Tyr Asn Gly Asp Val Glu Asp Ile
   545                 550                 555                 560
   Lys Arg Thr Pro Asn Asn Val Val Ser Thr Pro Ala Pro Ser Pro Asp
                   565                 570                 575
   Ala Ser Gln Leu Ala Ser Ser Leu Ser Ser Gln Lys Glu Val Ala Ala
               580                 585                 590
   Thr Glu Glu Asp Val Thr Arg Leu Pro Ser Pro Thr Ser Pro Phe Ser
           595                 600                 605
   Ser Leu Ser Gln Asp Gln Ala Ala Thr Ser Lys Ala Thr Leu Ser Ser
       610                 615                 620
   Thr Ser Gly Leu Asp Leu Met Ser Glu Ser Gly Glu Gly Glu Ile Ser
   625                 630                 635                 640
   Pro Gln Arg Glu Val Ser Arg Ser Gln Asp Gln Phe Ser Asp Met Arg
                   645                 650                 655
   Ile Ser Ile Asn Gln Thr Pro Gly Lys Ser Leu Asp Phe Gly Phe Thr
               660                 665                 670
   Ile Lys Trp Asp Ile Pro Gly Ile Phe Val Ala Ser Val Glu Ala Gly
           675                 680                 685
   Ser Pro Ala Glu Phe Ser Gln Leu Gln Val Asp Asp Glu Ile Ile Ala
       690                 695                 700
   Ile Asn Asn Thr Lys Phe Ser Tyr Asn Asp Ser Lys Glu Trp Glu Glu
   705                 710                 715                 720
   Ala Met Ala Lys Ala Gln Glu Thr Gly His Leu Val Met Asp Val Arg
                   725                 730                 735
   Arg Tyr Gly Lys Ala Gly Ser Pro Glu Thr Lys Trp Ile Asp Ala Thr
               740                 745                 750
   Ser Gly Ile Tyr Asn Ser Glu Lys Ser Ser Asn Leu Ser Val Thr Thr
           755                 760                 765
   Asp Phe Ser Glu Ser Leu Gln Ser Ser Asn Ile Glu Ser Lys Glu Ile
       770                 775                 780
   Asn Gly Ile His Asp Glu Ser Asn Ala Phe Glu Ser Lys Ala Ser Glu
   785 790 795 800
   Ser Ile Ser Leu Lys Asn Leu Lys Arg Arg Ser Gln Phe Phe Glu Gln
   805 810 815
   Gly Ser Ser Asp Ser Val Val Pro Asp Leu Pro Val Pro Thr Ile Ser
   820 825 830
   Ala Pro Ser Arg Trp Val Trp Asp Gln Glu Glu Glu Arg Lys Arg Gln
   835 840 845
   Glu Arg Trp Gln Lys Glu Gln Asp Arg Leu Leu Gln Glu Lys Tyr Gln
   850 855 860
   Arg Glu Gln Glu Lys Leu Arg Glu Glu Trp Gln Arg Ala Lys Gln Glu
   865 870 875 880
   Ala Glu Arg Glu Asn Ser Lys Tyr Leu Asp Glu Glu Leu Met Val Leu
   885 890 895
   Ser Ser Asn Ser Met Ser Leu Thr Thr Arg Glu Pro Ser Leu Ala Thr
   900 905 910
   Trp Glu Ala Thr Trp Ser Glu Gly Ser Lys Ser Ser Asp Arg Glu Gly
   915 920 925
   Thr Arg Ala Gly Glu Glu Glu Arg Arg Gln Pro Gln Glu Glu Val Val
   930 935 940
   His Glu Asp Gln Gly Lys Lys Pro Gln Asp Gln Leu Val Ile Glu Arg
   945 950 955 960
   Glu Arg Lys Trp Glu Gln Gln Leu Gln Glu Glu Gln Glu Gln Lys Arg
   965 970 975
   Leu Gln Ala Glu Ala Glu Glu Gln Lys Arg Pro Ala Glu Glu Gln Lys
   980 985 990
   Arg Gln Ala Glu Ile Glu Arg Glu Thr Ser Val Arg Ile Tyr Gln Tyr
   995 1000 1005
   Arg Arg Pro Val Asp Ser Tyr Asp Ile Pro Lys Thr Glu Glu Ala
   1010 1015 1020
   Ser Ser Gly Phe Leu Pro Gly Asp Arg Asn Lys Ser Arg Ser Thr
   1025 1030 1035
   Thr Glu Leu Asp Asp Tyr Ser
   1040 1045
<210> 36
   <211> 759
   <212> PRT
   <213> Homo sapiens
<400> 36
   Met Glu Ser Ser Pro Phe Asn Arg Arg Gln Trp Thr Ser Leu Ser Leu
   1               5                   10                  15
   Arg Val Thr Ala Lys Glu Leu Ser Leu Val Asn Lys Asn Lys Ser Ser
               20                  25                  30
   Ala Ile Val Glu Ile Phe Ser Lys Tyr Gln Lys Ala Ala Glu Glu Thr
           35                  40                  45
   Asn Met Glu Lys Lys Arg Ser Asn Thr Glu Asn Leu Ser Gln His Phe
       50                  55                  60
   Arg Lys Gly Thr Leu Thr Val Leu Lys Lys Lys Trp Glu Asn Pro Gly
   65                  70                  75                  80
   Leu Gly Ala Glu Ser His Thr Asp Ser Leu Arg Asn Ser Ser Thr Glu
                   85                  90                  95
   Ile Arg His Arg Ala Asp His Pro Pro Ala Glu Val Thr Ser His Ala
               100                 105                 110
   Ala Ser Gly Ala Lys Ala Asp Gln Glu Glu Gln Ile His Pro Arg Ser
           115                 120                 125
   Arg Leu Arg Ser Pro Pro Glu Ala Leu Val Gln Gly Arg Tyr Pro His
       130                 135                 140
   Ile Lys Asp Gly Glu Asp Leu Lys Asp His Ser Thr Glu Ser Lys Lys
   145                 150                 155                 160
   Met Glu Asn Cys Leu Gly Glu Ser Arg His Glu Val Glu Lys Ser Glu
                   165                 170                 175
   Ile Ser Glu Asn Thr Asp Ala Ser Gly Lys Ile Glu Lys Tyr Asn Val
               180                 185                 190
   Pro Leu Asn Arg Leu Lys Met Met Phe Glu Lys Gly Glu Pro Thr Gln
           195                 200                 205
   Thr Lys Ile Leu Arg Ala Gln Ser Arg Ser Ala Ser Gly Arg Lys Ile
       210                 215                 220
   Ser Glu Asn Ser Tyr Ser Leu Asp Asp Leu Glu Ile Gly Pro Gly Gln
   225                 230                 235                 240
   Leu Ser Ser Ser Thr Phe Asp Ser Glu Lys Asn Glu Ser Arg Arg Asn
   245 250 255
   Leu Glu Leu Pro Arg Leu Ser Glu Thr Ser Ile Lys Asp Arg Met Ala
   260 265 270
   Lys Tyr Gln Ala Ala Val Ser Lys Gln Ser Ser Ser Thr Asn Tyr Thr
   275 280 285
   Asn Glu Leu Lys Ala Ser Gly Gly Glu Ile Lys Ile His Lys Met Glu
   290 295 300
   Gln Lys Glu Asn Val Pro Pro Gly Pro Glu Val Cys Ile Thr His Gln
   305 310 315 320
   Glu Gly Glu Lys Ile Ser Ala Asn Glu Asn Ser Leu Ala Val Arg Ser
   325 330 335
   Thr Pro Ala Glu Asp Asp Ser Arg Asp Ser Gln Val Lys Ser Glu Val
   340 345 350
   Gln Gln Pro Val His Pro Lys Pro Leu Ser Pro Asp Ser Arg Ala Ser
   355 360 365
   Ser Leu Ser Glu Ser Ser Pro Pro Lys Ala Met Lys Lys Phe Gln Ala
   370 375 380
   Pro Ala Arg Glu Thr Cys Val Glu Cys Gln Lys Thr Val Tyr Pro Met
   385 390 395 400
   Glu Arg Leu Leu Ala Asn Gln Gln Val Phe His Ile Ser Cys Phe Arg
   405 410 415
   Cys Ser Tyr Cys Asn Asn Lys Leu Ser Leu Gly Thr Tyr Ala Ser Leu
   420 425 430
   His Gly Arg Ile Tyr Cys Lys Pro His Phe Asn Gln Leu Phe Lys Ser
   435 440 445
   Lys Gly Asn Tyr Asp Glu Gly Phe Gly His Arg Pro His Lys Asp Leu
   450 455 460
   Trp Ala Ser Lys Asn Glu Asn Glu Glu Ile Leu Glu Arg Pro Ala Gln
   465 470 475 480
   Leu Ala Asn Ala Arg Glu Thr Pro His Ser Pro Gly Val Glu Asp Ala
   485 490 495
   Pro Ile Ala Lys Val Gly Val Leu Ala Ala Ser Met Glu Ala Lys Ala
   500 505 510
   Ser Ser Gln Gln Glu Lys Glu Asp Lys Pro Ala Glu Thr Lys Lys Leu
           515                 520                 525
   Arg Ile Ala Trp Pro Pro Pro Thr Glu Leu Gly Ser Ser Gly Ser Ala
       530                 535                 540
   Leu Glu Glu Gly Ile Lys Met Ser Lys Pro Lys Trp Pro Pro Glu Asp
   545                 550                 555                 560
   Glu Ile Ser Lys Pro Glu Val Pro Glu Asp Val Asp Leu Asp Leu Lys
                   565                 570                 575
   Lys Leu Arg Arg Ser Ser Ser Leu Lys Glu Arg Ser Arg Pro Phe Thr
               580                 585                 590
   Val Ala Ala Ser Phe Gln Ser Thr Ser Val Lys Ser Pro Lys Thr Val
           595                 600                 605
   Ser Pro Pro Ile Arg Lys Gly Trp Ser Met Ser Glu Gln Ser Glu Glu
       610                 615                 620
   Ser Val Gly Gly Arg Val Ala Glu Arg Lys Gln Val Glu Asn Ala Lys
   625                 630                 635                 640
   Ala Ser Lys Lys Asn Gly Asn Val Gly Lys Thr Thr Trp Gln Asn Lys
                   645                 650                 655
   Glu Ser Lys Gly Glu Thr Gly Lys Arg Ser Lys Glu Gly His Ser Leu
               660                 665                 670
   Glu Met Glu Asn Glu Asn Leu Val Glu Asn Gly Ala Asp Ser Asp Glu
           675                 680                 685
   Asp Asp Asn Ser Phe Leu Lys Gln Gln Ser Pro Gln Glu Pro Lys Ser
       690                 695                 700
   Leu Asn Trp Ser Ser Phe Val Asp Asn Thr Phe Ala Glu Glu Phe Thr
   705                 710                 715                 720
   Thr Gln Asn Gln Lys Ser Gln Asp Val Glu Leu Trp Glu Gly Glu Val
                   725                 730                 735
   Val Lys Glu Leu Ser Val Glu Glu Gln Ile Lys Arg Asn Arg Tyr Tyr
               740                 745                 750
   Asp Glu Asp Glu Asp Glu Glu
           755
<210> 37
   <211> 877
   <212> PRT
   <213> Homo sapiens
<400> 37
   Met Leu Ala Ser Leu Tyr Asn Gly Ser Val Cys Val Trp Asn His Glu
   1               5                   10                  15
   Thr Gln Thr Leu Val Lys Thr Phe Glu Val Cys Asp Leu Pro Val Arg
               20                  25                  30
   Ala Ala Lys Phe Val Ala Arg Lys Asn Trp Val Val Thr Gly Ala Asp
           35                  40                  45
   Asp Met Gln Ile Arg Val Phe Asn Tyr Asn Thr Leu Glu Arg Val His
       50                  55                  60
   Met Phe Glu Ala His Ser Asp Tyr Ile Arg Cys Ile Ala Val His Pro
   65                  70                  75                  80
   Thr Gln Pro Phe Ile Leu Thr Ser Ser Asp Asp Met Leu Ile Lys Leu
                   85                  90                  95
   Trp Asp Trp Asp Lys Lys Trp Ser Cys Ser Gln Val Phe Glu Gly His
               100                 105                 110
   Thr His Tyr Val Met Gln Ile Val Ile Asn Pro Lys Asp Asn Asn Gln
           115                 120                 125
   Phe Ala Ser Ala Ser Leu Asp Arg Thr Ile Lys Val Trp Gln Leu Gly
       130                 135                 140
   Ser Ser Ser Pro Asn Phe Thr Leu Glu Gly His Glu Lys Gly Val Asn
   145                 150                 155                 160
   Cys Ile Asp Tyr Tyr Ser Gly Gly Asp Lys Pro Tyr Leu Ile Ser Gly
                   165                 170                 175
   Ala Asp Asp Arg Leu Val Lys Ile Trp Asp Tyr Gln Asn Lys Thr Cys
               180                 185                 190
   Val Gln Thr Leu Glu Gly His Ala Gln Asn Val Ser Cys Ala Ser Phe
           195                 200                 205
   His Pro Glu Leu Pro Ile Ile Ile Thr Gly Ser Glu Asp Gly Thr Val
       210                 215                 220
   Arg Ile Trp His Ser Ser Thr Tyr Arg Leu Glu Ser Thr Leu Asn Tyr
   225                 230                 235                 240
   Gly Met Glu Arg Val Trp Cys Val Ala Ser Leu Arg Gly Ser Asn Asn
                   245                 250                 255
   Val Ala Leu Gly Tyr Asp Glu Gly Ser Ile Ile Val Lys Leu Gly Arg
               260                 265                 270
   Glu Glu Pro Ala Met Ser Met Asp Ala Asn Gly Lys Ile Ile Trp Ala
           275                 280                 285
   Lys His Ser Glu Val Gln Gln Ala Asn Leu Lys Ala Met Gly Asp Ala
       290                 295                 300
   Glu Ile Lys Asp Gly Glu Arg Leu Pro Leu Ala Val Lys Asp Met Gly
   305                 310                 315                 320
   Ser Cys Glu Ile Tyr Pro Gln Thr Ile Gln His Asn Pro Asn Gly Arg
                   325                 330                 335
   Phe Val Val Val Cys Gly Asp Gly Glu Tyr Ile Ile Tyr Thr Ala Met
               340                 345                 350
   Ala Leu Arg Asn Lys Ser Phe Gly Ser Ala Gln Glu Phe Ala Trp Ala
           355                 360                 365
   His Asp Ser Ser Glu Tyr Ala Ile Arg Glu Ser Asn Ser Ile Val Lys
       370                 375                 380
   Ile Phe Lys Asn Phe Lys Glu Lys Lys Ser Phe Lys Pro Asp Phe Gly
   385                 390                 395                 400
   Ala Glu Ser Ile Tyr Gly Gly Phe Leu Leu Gly Val Arg Ser Val Asn
                   405                 410                 415
   Gly Leu Ala Phe Tyr Asp Trp Asp Asn Thr Glu Leu Ile Arg Arg Ile
               420                 425                 430
   Glu Ile Gln Pro Lys His Ile Phe Trp Ser Asp Ser Gly Glu Leu Val
           435                 440                 445
   Cys Ile Ala Thr Glu Glu Ser Phe Phe Ile Leu Lys Tyr Leu Ser Glu
       450                 455                 460
   Lys Val Leu Ala Ala Gln Glu Thr His Glu Gly Val Thr Glu Asp Gly
   465                 470                 475                 480
   Ile Glu Asp Ala Phe Glu Val Leu Gly Glu Ile Gln Glu Ile Val Lys
                   485                 490                 495
   Thr Gly Leu Trp Val Gly Asp Cys Phe Ile Tyr Thr Ser Ser Val Asn
               500                 505                 510
   Arg Leu Asn Tyr Tyr Val Gly Gly Glu Ile Val Thr Ile Ala His Leu
           515                 520                 525
   Asp Arg Thr Met Tyr Leu Leu Gly Tyr Ile Pro Lys Asp Asn Arg Leu
       530                 535                 540
   Tyr Leu Gly Asp Lys Glu Leu Asn Ile Ile Ser Tyr Ser Leu Leu Val
   545                 550                 555                 560
   Ser Val Leu Glu Tyr Gln Thr Ala Val Met Arg Arg Asp Phe Ser Met
                   565                 570                 575
   Ala Asp Lys Val Leu Pro Thr Ile Pro Lys Glu Gln Arg Thr Arg Val
               580                 585                 590
   Ala His Phe Leu Glu Lys Gln Gly Phe Lys Gln Gln Ala Leu Thr Val
           595                 600                 605
   Ser Thr Asp Pro Glu His Arg Phe Glu Leu Ala Leu Gln Leu Gly Glu
       610                 615                 620
   Leu Lys Ile Ala Tyr Gln Leu Ala Val Glu Ala Glu Ser Glu Gln Lys
   625                 630                 635                 640
   Trp Lys Gln Leu Ala Glu Leu Ala Ile Ser Lys Cys Gln Phe Gly Leu
                   645                 650                 655
   Ala Gln Glu Cys Leu His His Ala Gln Asp Tyr Gly Gly Leu Leu Leu
               660                 665                 670
   Leu Ala Thr Ala Ser Gly Asn Ala Asn Met Val Asn Lys Leu Ala Glu
           675                 680                 685
   Gly Ala Glu Arg Asp Gly Lys Asn Asn Val Ala Phe Met Ser Tyr Phe
       690                 695                 700
   Leu Gln Gly Lys Val Asp Ala Cys Leu Glu Leu Leu Ile Arg Thr Gly
   705                 710                 715                 720
   Arg Leu Pro Glu Ala Ala Phe Leu Ala Arg Thr Tyr Leu Pro Ser Gln
                   725                 730                 735
   Val Ser Arg Val Val Lys Leu Trp Arg Glu Asn Leu Ser Lys Val Asn
               740                 745                 750
   Gln Lys Ala Ala Glu Ser Leu Ala Asp Pro Thr Glu Tyr Glu Asn Leu
           755                 760                 765
   Phe Pro Gly Leu Lys Glu Ala Phe Val Val Glu Glu Trp Val Lys Glu
       770                 775                 780
   Thr His Ala Asp Leu Trp Pro Ala Lys Gln Tyr Pro Leu Val Thr Pro
   785                 790                 795                 800
   Asn Glu Glu Arg Asn Val Met Glu Glu Gly Lys Asp Phe Gln Pro Ser
                   805                 810                 815
   Arg Ser Thr Ala Gln Gln Glu Leu Asp Gly Lys Pro Ala Ser Pro Thr
   820 825 830
   Pro Val Ile Val Ala Ser His Thr Ala Asn Lys Glu Glu Lys Ser Leu
   835 840 845
   Leu Glu Leu Glu Val Asp Leu Asp Asn Leu Glu Leu Glu Asp Ile Asp
   850 855 860
   Thr Thr Asp Ile Asn Leu Asp Glu Asp Ile Leu Asp Asp
   865 870 875
<210> 38
   <211> 461
   <212> PRT
   <213> Homo sapiens
<400> 38
<210> 39
   <211> 596
   <212> PRT
   <213> Homo sapiens
<400> 39
   Met Ser Asn Tyr Ser Val Ser Leu Val Gly Pro Ala Pro Trp Gly Phe
   1 5 10 15
   Arg Leu Gln Gly Gly Lys Asp Phe Asn Met Pro Leu Thr Ile Ser Ser
   20 25 30
   Leu Lys Asp Gly Gly Lys Ala Ala Gln Ala Asn Val Arg Ile Gly Asp
   35 40 45
   Val Val Leu Ser Ile Asp Gly Ile Asn Ala Gln Gly Met Thr His Leu
   50 55 60
   Glu Ala Gln Asn Lys Ile Lys Gly Cys Thr Gly Ser Leu Asn Met Thr
   65 70 75 80
   Leu Gln Arg Ala Ser Ala Ala Pro Lys Pro Glu Pro Val Pro Val Gln
   85 90 95
   Lys Gly Glu Pro Lys Glu Val Val Lys Pro Val Pro Ile Thr Ser Pro
   100 105 110
   Ala Val Ser Lys Val Thr Ser Thr Asn Asn Met Ala Tyr Asn Lys Ala
   115 120 125
   Pro Arg Pro Phe Gly Ser Val Ser Ser Pro Lys Val Thr Ser Ile Pro
   130 135 140
   Ser Pro Ser Ser Ala Phe Thr Pro Ala His Ala Thr Thr Ser Ser His
   145 150 155 160
   Ala Ser Pro Ser Pro Val Ala Ala Val Thr Pro Pro Leu Phe Ala Ala
   165 170 175
   Ser Gly Leu His Ala Asn Ala Asn Leu Ser Ala Asp Gln Ser Pro Ser
   180 185 190
   Ala Leu Ser Ala Gly Lys Thr Ala Val Asn Val Pro Arg Gln Pro Thr
   195 200 205
   Val Thr Ser Val Cys Ser Glu Thr Ser Gln Glu Leu Ala Glu Gly Gln
   210 215 220
   Arg Arg Gly Ser Gln Gly Asp Ser Lys Gln Gln Asn Gly Pro Pro Arg
   225                 230                 235                 240
   Lys His Ile Val Glu Arg Tyr Thr Glu Phe Tyr His Val Pro Thr His
                   245                 250                 255
   Ser Asp Ala Ser Lys Lys Arg Leu Ile Glu Asp Thr Glu Asp Trp Arg
               260                 265                 270
   Pro Arg Thr Gly Thr Thr Gln Ser Arg Ser Phe Arg Ile Leu Ala Gln
           275                 280                 285
   Ile Thr Gly Thr Glu His Leu Lys Glu Ser Glu Ala Asp Asn Thr Lys
       290                 295                 300
   Lys Ala Asn Asn Ser Gln Glu Pro Ser Pro Gln Leu Ala Ser Ser Val
   305                 310                 315                 320
   Ala Ser Thr Arg Ser Met Pro Glu Ser Leu Asp Ser Pro Thr Ser Gly
                   325                 330                 335
   Arg Pro Gly Val Thr Ser Leu Thr Ala Ala Ala Ala Phe Lys Pro Val
               340                 345                 350
   Gly Ser Thr Gly Val Ile Lys Ser Pro Ser Trp Gln Arg Pro Asn Gln
           355                 360                 365
   Gly Val Pro Ser Thr Gly Arg Ile Ser Asn Ser Ala Thr Tyr Ser Gly
       370                 375                 380
   Ser Val Ala Pro Ala Asn Ser Ala Leu Gly Gln Thr Gln Pro Ser Asp
   385                 390                 395                 400
   Gln Asp Thr Leu Val Gln Arg Ala Glu His Ile Pro Ala Gly Lys Arg
                   405                 410                 415
   Thr Pro Met Cys Ala His Cys Asn Gln Val Ile Arg Gly Pro Phe Leu
               420                 425                 430
   Val Ala Leu Gly Lys Ser Trp His Pro Glu Glu Phe Asn Cys Ala His
           435                 440                 445
   Cys Lys Asn Thr Met Ala Tyr Ile Gly Phe Val Glu Glu Lys Gly Ala
       450                 455                 460
   Leu Tyr Cys Glu Leu Cys Tyr Glu Lys Phe Phe Ala Pro Glu Cys Gly
   465                 470                 475                 480
   Arg Cys Gln Arg Lys Ile Leu Gly Glu Val Ile Ser Ala Leu Lys Gln
                   485                 490                 495
   Thr Trp His Val Ser Cys Phe Val Cys Val Ala Cys Gly Lys Pro Ile
   500 505 510
   Arg Asn Asn Val Phe His Leu Glu Asp Gly Glu Pro Tyr Cys Glu Thr
   515 520 525
   Asp Tyr Tyr Ala Leu Phe Gly Thr Ile Cys His Gly Cys Glu Phe Pro
   530 535 540
   Ile Glu Ala Gly Asp Met Phe Leu Glu Ala Leu Gly Tyr Thr Trp His
   545 550 555 560
   Asp Thr Cys Phe Val Cys Ser Val Cys Cys Glu Ser Leu Glu Gly Gln
   565 570 575
   Thr Phe Phe Ser Lys Lys Asp Lys Pro Leu Cys Lys Lys His Ala His
   580 585 590
   Ser Val Asn Phe
   595
<210> 40
   <211> 331
   <212> PRT
   <213> Homo sapiens
<400> 40
   Met Ala Arg Gly Gly Arg Gly Arg Arg Leu Gly Leu Ala Leu Gly Leu
   1               5                   10                  15
   Leu Leu Ala Leu Val Leu Ala Pro Arg Val Leu Arg Ala Lys Pro Thr
               20                  25                  30
   Val Arg Lys Glu Arg Val Val Arg Pro Asp Ser Glu Leu Gly Glu Arg
           35                  40                  45
   Pro Pro Glu Asp Asn Gln Ser Phe Gln Tyr Asp His Glu Ala Phe Leu
       50                  55                  60
   Gly Lys Glu Asp Ser Lys Thr Phe Asp Gln Leu Thr Pro Asp Glu Ser
   65                  70                  75                  80
   Lys Glu Arg Leu Gly Lys Ile Val Asp Arg Ile Asp Asn Asp Gly Asp
                   85                  90                  95
   Gly Phe Val Thr Thr Glu Glu Leu Lys Thr Trp Ile Lys Arg Val Gln
               100                 105                 110
   Lys Arg Tyr Ile Phe Asp Asn Val Ala Lys Val Trp Lys Asp Tyr Asp
           115                 120                 125
   Arg Asp Lys Asp Asp Lys Ile Ser Trp Glu Glu Tyr Lys Gln Ala Thr
   130 135 140
   Tyr Gly Tyr Tyr Leu Gly Asn Pro Ala Glu Phe His Asp Ser Ser Asp
   145 150 155 160
   His His Thr Phe Lys Lys Met Leu Pro Arg Asp Glu Arg Arg Phe Lys
   165 170 175
   Ala Ala Asp Leu Asn Gly Asp Leu Thr Ala Thr Arg Glu Glu Phe Thr
   180 185 190
   Ala Phe Leu His Pro Glu Glu Phe Glu His Met Lys Glu Ile Val Val
   195 200 205
   Leu Glu Thr Leu Glu Asp Ile Asp Lys Asn Gly Asp Gly Phe Val Asp
   210 215 220
   Gln Asp Glu Tyr Ile Ala Asp Met Phe Ser His Glu Glu Asn Gly Pro
   225 230 235 240
   Glu Pro Asp Trp Val Leu Ser Glu Arg Glu Gln Phe Asn Glu Phe Arg
   245 250 255
   Asp Leu Asn Lys Asp Gly Lys Leu Asp Lys Asp Glu Ile Arg His Trp
   260 265 270
   Ile Leu Pro Gln Asp Tyr Asp His Ala Gln Ala Glu Ala Arg His Leu
   275 280 285
   Val Tyr Glu Ser Asp Lys Asn Lys Asp Glu Lys Leu Thr Lys Glu Glu
   290 295 300
   Ile Leu Glu Asn Trp Asn Met Phe Val Gly Ser Gln Ala Thr Asn Tyr
   305 310 315 320
   Gly Glu Asp Leu Thr Lys Asn His Asp Glu Leu
   325 330
<210> 41
   <211> 1411
   <212> PRT
   <213> Homo sapiens
<400> 41
   Met Leu Arg Arg Ile Leu Gln Arg Thr Pro Gly Arg Val Gly Ser Gln
   1               5                   10                  15
   Gly Ser Asp Leu Asp Ser Ser Ala Thr Pro Ile Asn Thr Val Asp Val
               20                  25                  30
   Asn Asn Glu Ser Ser Ser Glu Gly Phe Ile Cys Pro Gln Cys Met Lys
           35                  40                  45
   Ser Leu Gly Ser Ala Asp Glu Leu Phe Lys His Tyr Glu Ala Val His
   50 55 60
   Asp Ala Gly Asn Asp Ser Gly His Gly Gly Glu Ser Asn Leu Ala Leu
   65 70 75 80
   Lys Arg Asp Asp Val Thr Leu Leu Arg Gln Glu Val Gln Asp Leu Gln
   85 90 95
   Ala Ser Leu Lys Glu Glu Lys Trp Tyr Ser Glu Glu Leu Lys Lys Glu
   100 105 110
   Leu Glu Lys Tyr Gln Gly Leu Gln Gln Gln Glu Ala Lys Pro Asp Gly
   115 120 125
   Leu Val Thr Asp Ser Ser Ala Glu Leu Gln Ser Leu Glu Gln Gln Leu
   130 135 140
   Glu Glu Ala Gln Thr Glu Asn Phe Asn Ile Lys Gln Met Lys Asp Leu
   145 150 155 160
   Phe Glu Gln Lys Ala Ala Gln Leu Ala Thr Glu Ile Ala Asp Ile Lys
   165 170 175
   Ser Lys Tyr Asp Glu Glu Arg Ser Leu Arg Glu Ala Ala Glu Gln Lys
   180 185 190
   Val Thr Arg Leu Thr Glu Glu Leu Asn Lys Glu Ala Thr Val Ile Gln
   195 200 205
   Asp Leu Lys Thr Glu Leu Leu Gln Arg Pro Gly Ile Glu Asp Val Ala
   210 215 220
   Val Leu Lys Lys Glu Leu Val Gln Val Gln Thr Leu Met Asp Asn Met
   225 230 235 240
   Thr Leu Glu Arg Glu Arg Glu Ser Glu Lys Leu Lys Asp Glu Cys Lys
   245 250 255
   Lys Leu Gln Ser Gln Tyr Ala Ser Ser Glu Ala Thr Ile Ser Gln Leu
   260 265 270
   Arg Ser Glu Leu Ala Lys Gly Pro Gln Glu Val Ala Val Tyr Val Gln
   275 280 285
   Glu Leu Gln Lys Leu Lys Ser Ser Val Asn Glu Leu Thr Gln Lys Asn
   290 295 300
   Gln Thr Leu Thr Glu Asn Leu Leu Lys Lys Glu Gln Asp Tyr Thr Lys
   305 310 315 320
   Leu Glu Glu Lys His Asn Glu Glu Ser Val Ser Lys Lys Asn Ile Gln
                   325                 330                 335
   Ala Thr Leu His Gln Lys Asp Leu Asp Cys Gln Gln Leu Gln Ser Arg
               340                 345                 350
   Leu Ser Ala Ser Glu Thr Ser Leu His Arg Ile His Val Glu Leu Ser
           355                 360                 365
   Glu Lys Gly Glu Ala Thr Gln Lys Leu Lys Glu Glu Leu Ser Glu Val
       370                 375                 380
   Glu Thr Lys Tyr Gln His Leu Lys Ala Glu Phe Lys Gln Leu Gln Gln
   385                 390                 395                 400
   Gln Arg Glu Glu Lys Glu Gln His Gly Leu Gln Leu Gln Ser Glu Ile
                   405                 410                 415
   Asn Gln Leu His Ser Lys Leu Leu Glu Thr Glu Arg Gln Leu Gly Glu
               420                 425                 430
   Ala His Gly Arg Leu Lys Glu Gln Arg Gln Leu Ser Ser Glu Lys Leu
           435                 440                 445
   Met Asp Lys Glu Gln Gln Val Ala Asp Leu Gln Leu Lys Leu Ser Arg
       450                 455                 460
   Leu Glu Glu Gln Leu Lys Glu Lys Val Thr Asn Ser Thr Glu Leu Gln
   465                 470                 475                 480
   His Gln Leu Asp Lys Thr Lys Gln Gln His Gln Glu Gln Gln Ala Leu
                   485                 490                 495
   Gln Gln Ser Thr Thr Ala Lys Leu Arg Glu Ala Gln Asn Asp Leu Glu
               500                 505                 510
   Gln Val Leu Arg Gln Ile Gly Asp Lys Asp Gln Lys Ile Gln Asn Leu
           515                 520                 525
   Glu Ala Leu Leu Gln Lys Ser Lys Glu Asn Ile Ser Leu Leu Glu Lys
       530                 535                 540
   Glu Arg Glu Asp Leu Tyr Ala Lys Ile Gln Ala Gly Glu Gly Glu Thr
   545                 550                 555                 560
   Ala Val Leu Asn Gln Leu Gln Glu Lys Asn His Thr Leu Gln Glu Gln
                   565                 570                 575
   Val Thr Gln Leu Thr Glu Lys Leu Lys Asn Gln Ser Glu Ser His Lys
               580                 585                 590
   Gln Ala Gln Glu Asn Leu His Asp Gln Val Gln Glu Gln Lys Ala His
   595 600 605
   Leu Arg Ala Ala Gln Asp Arg Val Leu Ser Leu Glu Thr Ser Val Asn
   610 615 620
   Glu Leu Asn Ser Gln Leu Asn Glu Ser Lys Glu Lys Val Ser Gln Leu
   625 630 635 640
   Asp Ile Gln Ile Lys Ala Lys Thr Glu Leu Leu Leu Ser Ala Glu Ala
   645 650 655
   Ala Lys Thr Ala Gln Arg Ala Asp Leu Gln Asn His Leu Asp Thr Ala
   660 665 670
   Gln Asn Ala Leu Gln Asp Lys Gln Gln Glu Leu Asn Lys Ile Thr Thr
   675 680 685
   Gln Leu Asp Gln Val Thr Ala Lys Leu Gln Asp Lys Gln Glu His Cys
   690 695 700
   Ser Gln Leu Glu Ser His Leu Lys Glu Tyr Lys Glu Lys Tyr Leu Ser
   705 710 715 720
   Leu Glu Gln Lys Thr Glu Glu Leu Glu Gly Gln Ile Lys Lys Leu Glu
   725 730 735
   Ala Asp Ser Leu Glu Val Lys Ala Ser Lys Glu Gln Ala Leu Gln Asp
   740 745 750
   Leu Gln Gln Gln Arg Gln Leu Asn Thr Asp Leu Glu Leu Arg Ala Thr
   755 760 765
   Glu Leu Ser Lys Gln Leu Glu Met Glu Lys Glu Ile Val Ser Ser Thr
   770 775 780
   Arg Leu Asp Leu Gln Lys Lys Ser Glu Ala Leu Glu Ser Ile Lys Gln
   785 790 795 800
   Lys Leu Thr Lys Gln Glu Glu Glu Lys Lys Ile Leu Lys Gln Asp Phe
   805 810 815
   Glu Thr Leu Ser Gln Glu Thr Lys Ile Gln His Glu Glu Leu Asn Asn
   820 825 830
   Arg Ile Gln Thr Thr Val Thr Glu Leu Gln Lys Val Lys Met Glu Lys
   835 840 845
   Glu Ala Leu Met Thr Glu Leu Ser Thr Val Lys Asp Lys Leu Ser Lys
   850 855 860
   Val Ser Asp Ser Leu Lys Asn Ser Lys Ser Glu Phe Glu Lys Glu Asn
   865                 870                 875                 880
   Gln Lys Gly Lys Ala Ala Ile Leu Asp Leu Glu Lys Thr Cys Lys Glu
                   885                 890                 895
   Leu Lys His Gln Leu Gln Val Gln Met Glu Asn Thr Leu Lys Glu Gln
               900                 905                 910
   Lys Glu Leu Lys Lys Ser Leu Glu Lys Glu Lys Glu Ala Ser His Gln
           915                 920                 925
   Leu Lys Leu Glu Leu Asn Ser Met Gln Glu Gln Leu Ile Gln Ala Gln
       930                 935                 940
   Asn Thr Leu Lys Gln Asn Glu Lys Glu Glu Gln Gln Leu Gln Gly Asn
   945                 950                 955                 960
   Ile Asn Glu Leu Lys Gln Ser Ser Glu Gln Lys Lys Lys Gln Ile Glu
                   965                 970                 975
   Ala Leu Gln Gly Glu Leu Lys Ile Ala Val Leu Gln Lys Thr Glu Leu
               980                 985                 990
   Glu Asn Lys Leu Gln Gln Gln Leu  Thr Gln Ala Ala Gln  Glu Leu Ala
           995                 1000                1005
   Ala Glu  Lys Glu Lys Ile Ser  Val Leu Gln Asn Asn  Tyr Glu Lys
       1010                1015                1020
   Ser Gln  Glu Thr Phe Lys Gln  Leu Gln Ser Asp Phe  Tyr Gly Arg
       1025                1030                1035
   Glu Ser  Glu Leu Leu Ala Thr  Arg Gln Asp Leu Lys  Ser Val Glu
       1040                1045                1050
   Glu Lys  Leu Ser Leu Ala Gln  Glu Asp Leu Ile Ser  Asn Arg Asn
       1055                1060                1065
   Gln Ile  Gly Asn Gln Asn Lys  Leu Ile Gln Glu Leu  Lys Thr Ala
       1070                1075                1080
   Lys Ala  Thr Leu Glu Gln Asp  Ser Ala Lys Lys Glu  Gln Gln Leu
       1085                1090                1095
   Gln Glu  Arg Cys Lys Ala Leu  Gln Asp Ile Gln Lys  Glu Lys Ser
       1100                1105                1110
   Leu Lys  Glu Lys Glu Leu Val  Asn Glu Lys Ser Lys  Leu Ala Glu
       1115                1120                1125
   Ile Glu Glu Ile Lys Cys Arg Gln Glu Lys Glu Ile Thr Lys Leu
   1130 1135 1140
   Asn Glu Glu Leu Lys Ser His Lys Leu Glu Ser Ile Lys Glu Ile
   1145 1150 1155
   Thr Asn Leu Lys Asp Ala Lys Gln Leu Leu Ile Gln Gln Lys Leu
   1160 1165 1170
   Glu Leu Gln Gly Lys Ala Asp Ser Leu Lys Ala Ala Val Glu Gln
   1175 1180 1185
   Glu Lys Arg Asn Gln Gln Ile Leu Lys Asp Gln Val Lys Lys Glu
   1190 1195 1200
   Glu Glu Glu Leu Lys Lys Glu Phe Ile Glu Lys Glu Ala Lys Leu
   1205 1210 1215
   His Ser Glu Ile Lys Glu Lys Glu Val Gly Met Lys Lys His Glu
   1220 1225 1230
   Glu Asn Glu Ala Lys Leu Thr Met Gln Ile Thr Ala Leu Asn Glu
   1235 1240 1245
   Asn Leu Gly Thr Val Lys Lys Glu Trp Gln Ser Ser Gln Arg Arg
   1250 1255 1260
   Val Ser Glu Leu Glu Lys Gln Thr Asp Asp Leu Arg Gly Glu Ile
   1265 1270 1275
   Ala Val Leu Glu Ala Thr Val Gln Asn Asn Gln Asp Glu Arg Arg
   1280 1285 1290
   Ala Leu Leu Glu Arg Cys Leu Lys Gly Glu Gly Glu Ile Glu Lys
   1295 1300 1305
   Leu Gln Thr Lys Val Leu Glu Leu Gln Arg Lys Leu Asp Asn Thr
   1310 1315 1320
   Thr Ala Ala Val Gln Glu Leu Gly Arg Glu Asn Gln Ser Leu Gln
   1325 1330 1335
   Ile Lys His Thr Gln Ala Leu Asn Arg Lys Trp Ala Glu Asp Asn
   1340 1345 1350
   Glu Val Gln Asn Cys Met Ala Cys Gly Lys Gly Phe Ser Val Thr
   1355 1360 1365
   Val Arg Arg His His Cys Arg Gln Cys Gly Asn Ile Phe Cys Ala
   1370 1375 1380
   Glu Cys  Ser Ala Lys Asn Ala  Leu Thr Pro Ser Ser  Lys Lys Pro
       1385                1390                1395
   Val Arg  Val Cys Asp Ala Cys  Phe Asn Asp Leu Gln  Gly
       1400                1405                1410
<210> 42
   <211> 360
   <212> PRT
   <213> Homo sapiens
<400> 42
   Met Ser Lys Gln Gln Pro Thr Gln Phe Ile Asn Pro Glu Thr Pro Gly
   1               5                   10                  15
   Tyr Val Gly Phe Ala Asn Leu Pro Asn Gln Val His Arg Lys Ser Val
               20                  25                  30
   Lys Lys Gly Phe Glu Phe Thr Leu Met Val Val Gly Glu Ser Gly Leu
           35                  40                  45
   Gly Lys Ser Thr Leu Ile Asn Ser Leu Phe Leu Thr Asp Leu Tyr Pro
       50                  55                  60
   Glu Arg Val Ile Pro Gly Ala Ala Glu Lys Ile Glu Arg Thr Val Gln
   65                  70                  75                  80
   Ile Glu Ala Ser Thr Val Glu Ile Glu Glu Arg Gly Val Lys Leu Arg
                   85                  90                  95
   Leu Thr Val Val Asp Thr Pro Gly Tyr Gly Asp Ala Ile Asn Cys Arg
               100                 105                 110
   Asp Cys Phe Lys Thr Ile Ile Ser Tyr Ile Asp Glu Gln Phe Glu Arg
           115                 120                 125
   Tyr Leu His Asp Glu Ser Gly Leu Asn Arg Arg His Ile Ile Asp Asn
       130                 135                 140
   Arg Val His Cys Cys Phe Tyr Phe Ile Ser Pro Phe Gly His Gly Leu
   145                 150                 155                 160
   Lys Pro Leu Asp Val Ala Phe Met Lys Ala Ile His Asn Lys Val Asn
                   165                 170                 175
   Ile Val Pro Val Ile Ala Lys Ala Asp Thr Leu Thr Leu Lys Glu Arg
               180                 185                 190
   Glu Arg Leu Lys Lys Arg Ile Leu Asp Glu Ile Glu Glu His Asn Ile
           195                 200                 205
   Lys Ile Tyr His Leu Pro Asp Ala Glu Ser Asp Glu Asp Glu Asp Phe
   210 215 220
   Lys Glu Gln Thr Arg Leu Leu Lys Ala Ser Ile Pro Phe Ser Val Val
   225 230 235 240
   Gly Ser Asn Gln Leu Ile Glu Ala Lys Gly Lys Lys Val Arg Gly Arg
   245 250 255
   Leu Tyr Pro Trp Gly Val Val Glu Val Glu Asn Pro Glu His Asn Asp
   260 265 270
   Phe Leu Lys Leu Arg Thr Met Leu Ile Thr His Met Gln Asp Leu Gln
   275 280 285
   Glu Val Thr Gln Asp Leu His Tyr Glu Asn Phe Arg Ser Glu Arg Leu
   290 295 300
   Lys Arg Gly Gly Arg Lys Val Glu Asn Glu Asp Met Asn Lys Asp Gln
   305 310 315 320
   Ile Leu Leu Glu Lys Glu Ala Glu Leu Arg Arg Met Gln Glu Met Ile
   325 330 335
   Ala Arg Met Gln Ala Gln Met Gln Met Gln Met Gln Gly Gly Asp Gly
   340 345 350
   Asp Gly Gly Ala Leu Gly His His
   355 360
<210> 43
   <211> 300
   <212> PRT
   <213> Homo sapiens
<400> 43
   Met Ile Leu Leu Glu Val Asn Asn Arg Ile Ile Glu Glu Thr Leu Ala
   1 5 10 15
   Leu Lys Phe Glu Asn Ala Ala Ala Gly Asn Lys Pro Glu Ala Val Glu
   20 25 30
   Val Thr Phe Ala Asp Phe Asp Gly Val Leu Tyr His Ile Ser Asn Pro
   35 40 45
   Asn Gly Asp Lys Thr Lys Val Met Val Ser Ile Ser Leu Lys Phe Tyr
   50 55 60
   Lys Glu Leu Gln Ala His Gly Ala Asp Glu Leu Leu Lys Arg Val Tyr
   65 70 75 80
   Gly Ser Phe Leu Val Asn Pro Glu Ser Gly Tyr Asn Val Ser Leu Leu
   85 90 95
   Tyr Asp Leu Glu Asn Leu Pro Ala Ser Lys Asp Ser Ile Val His Gln
   100 105 110
   Ala Gly Met Leu Lys Arg Asn Cys Phe Ala Ser Val Phe Glu Lys Tyr
   115 120 125
   Phe Gln Phe Gln Glu Glu Gly Lys Glu Gly Glu Asn Arg Ala Val Ile
   130 135 140
   His Tyr Arg Asp Asp Glu Thr Met Tyr Val Glu Ser Lys Lys Asp Arg
   145 150 155 160
   Val Thr Val Val Phe Ser Thr Val Phe Lys Asp Asp Asp Asp Val Val
   165 170 175
   Ile Gly Lys Val Phe Met Gln Glu Phe Lys Glu Gly Arg Arg Ala Ser
   180 185 190
   His Thr Ala Pro Gln Val Leu Phe Ser His Arg Glu Pro Pro Leu Glu
   195 200 205
   Leu Lys Asp Thr Asp Ala Ala Val Gly Asp Asn Ile Gly Tyr Ile Thr
   210 215 220
   Phe Val Leu Phe Pro Arg His Thr Asn Ala Ser Ala Arg Asp Asn Thr
   225 230 235 240
   Ile Asn Leu Ile His Thr Phe Arg Asp Tyr Leu His Tyr His Ile Lys
   245 250 255
   Cys Ser Lys Ala Tyr Ile His Thr Arg Met Arg Ala Lys Thr Ser Asp
   260 265 270
   Phe Leu Lys Val Leu Asn Arg Ala Arg Pro Asp Ala Glu Lys Lys Glu
   275 280 285
   Met Lys Thr Ile Thr Gly Lys Thr Phe Ser Ser Arg
   290 295 300
<210> 44
   <211> 425
   <212> PRT
   <213> Homo sapiens
<400> 44
   Met Ala Val Ala Val Gly Arg Pro Ser Asn Glu Glu Leu Arg Asn Leu
   1               5                   10                  15
   Ser Leu Ser Gly His Val Gly Phe Asp Ser Leu Pro Asp Gln Leu Val
   20 25 30
   Asn Lys Ser Thr Ser Gln Gly Phe Cys Phe Asn Ile Leu Cys Val Gly
   35 40 45
   Glu Thr Gly Ile Gly Lys Ser Thr Leu Met Asp Thr Leu Phe Asn Thr
   50 55 60
   Lys Phe Glu Ser Asp Pro Ala Thr His Asn Glu Pro Gly Val Arg Leu
   65 70 75 80
   Lys Ala Arg Ser Tyr Glu Leu Gln Glu Ser Asn Val Arg Leu Lys Leu
   85 90 95
   Thr Ile Val Asp Thr Val Gly Phe Gly Asp Gln Ile Asn Lys Asp Asp
   100 105 110
   Ser Tyr Lys Pro Ile Val Glu Tyr Ile Asp Ala Gln Phe Glu Ala Tyr
   115 120 125
   Leu Gln Glu Glu Leu Lys Ile Lys Arg Ser Leu Phe Asn Tyr His Asp
   130 135 140
   Thr Arg Ile His Ala Cys Leu Tyr Phe Ile Ala Pro Thr Gly His Ser
   145 150 155 160
   Leu Lys Ser Leu Asp Leu Val Thr Met Lys Lys Leu Asp Ser Lys Val
   165 170 175
   Asn Ile Ile Pro Ile Ile Ala Lys Ala Asp Thr Ile Ala Lys Asn Glu
   180 185 190
   Leu His Lys Phe Lys Ser Lys Ile Met Ser Glu Leu Val Ser Asn Gly
   195 200 205
   Val Gln Ile Tyr Gln Phe Pro Thr Asp Glu Glu Thr Val Ala Glu Ile
   210 215 220
   Asn Ala Thr Met Ser Val His Leu Pro Phe Ala Val Val Gly Ser Thr
   225 230 235 240
   Glu Glu Val Lys Ile Gly Asn Lys Met Ala Lys Ala Arg Gln Tyr Pro
   245 250 255
   Trp Gly Val Val Gln Val Glu Asn Glu Asn His Cys Asp Phe Val Lys
   260 265 270
   Leu Arg Glu Met Leu Ile Arg Val Asn Met Glu Asp Leu Arg Glu Gln
   275 280 285
   Thr His Thr Arg His Tyr Glu Leu Tyr Arg Arg Cys Lys Leu Glu Glu
   290 295 300
   Met Gly Phe Lys Asp Thr Asp Pro Asp Ser Lys Pro Phe Ser Leu Gln
   305 310 315 320
   Glu Thr Tyr Glu Ala Lys Arg Asn Glu Phe Leu Gly Glu Leu Gln Lys
   325 330 335
   Lys Glu Glu Glu Met Arg Gln Met Phe Val Met Arg Val Lys Glu Lys
   340 345 350
   Glu Ala Glu Leu Lys Glu Ala Glu Lys Glu Leu His Glu Lys Phe Asp
   355 360 365
   Leu Leu Lys Arg Thr His Gln Glu Glu Lys Lys Lys Val Glu Asp Lys
   370 375 380
   Lys Lys Glu Leu Glu Glu Glu Val Asn Asn Phe Gln Lys Lys Lys Ala
   385 390 395 400
   Ala Ala Gln Leu Leu Gln Ser Gln Ala Gln Gln Ser Gly Ala Gln Gln
   405 410 415
   Thr Lys Lys Asp Lys Asp Lys Lys Lys
   420 425